# EUROPÄISCHE PATENTSCHRIFT

(11) **EP 0 833 827 B1**
(45) Veröffentlichungstag und Bekanntmachung des Hinweises auf die Patenterteilung: **21.11.2001**
(21) Anmeldenummer: 96921943.5
(22) Anmeldetag: 07.06.1996
(51) Int. Cl.: C07D 405/04, A01N 43/68, C07D 251/18

(54) **2-AMINO-1,3,5-TRIAZINE, VERFAHREN ZU DEREN HERSTELLUNG UND DEREN VERWENDUNG ALS HERBIZIDE UND PFLANZENWACHSTUMSREGULATOREN**
2-AMINO-1,3,5-TRIAZINES, PROCESS FOR THEIR PREPARATION AND THEIR USE AS HERBICIDES AND PLANT-GROWTH REGULATORS
2-AMINO-1,3,5-TRIAZINES, LEURS PROCEDES DE PREPARATION ET D'UTILISATION COMME HERBICIDES ET REGULATEURS DE CROISSANCE VEGETALE

(30) Priorität: 19.06.1995 DE 19522137
(43) Veröffentlichungstag der Anmeldung: 08.04.1998
(73) Patentinhaber: Aventis CropScience GmbH, 65929 Frankfurt am Main (DE)
(72) Erfinder: LORENZ, Klaus, D-64331 Weiterstadt (DE); MINN, Klemens, D-65795 Hattersheim (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9602479
(87) Internationale Veröffentlichungsnummer: WO9700254

(56) Entgegenhaltungen:
- EP-A- 0 411 153
- EP-A- 0 509 544

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß 2-Amino-4-alkylamino-6-*a*-halogenalkyl-1,3,5-triazine herbizide und pflanzenwachstumsregulierende Eigenschaften besitzen (WO 90/09378 (EP-A-411153), WO 88/02368 (EP-A-283522), WO 94/24086 EP-A-509544, EP-A-492,615). Die Anwendung vieler der bekannten Derivate dieses Typs als selektive Herbizide zur Schadpflanzenbekämpfung oder als Pflanzenwachstumsregulatoren in verschiedenen Nutzpflanzenkulturen führt jedoch häufig zu unerwünschten Schädigungen der Nutzpflanzen. Überraschenderweise wurden nun neue 2-Amino-1,3,5-triazine gefunden, die vorteilhaft als Herbizide und Pflanzenwachstumsregulatoren eingesetzt werden können. Beispielsweise werden beim Einsatz der erfindungsgemäßen Verbindungen die Kulturpflanzen nicht oder in geringerem Umfang geschädigt als bei den bekannten Wirkstoffen ähnlichen Typs.
Gegenstand der vorliegenden Erfindung sind Verbindungen der Formel (I) und deren Salze, 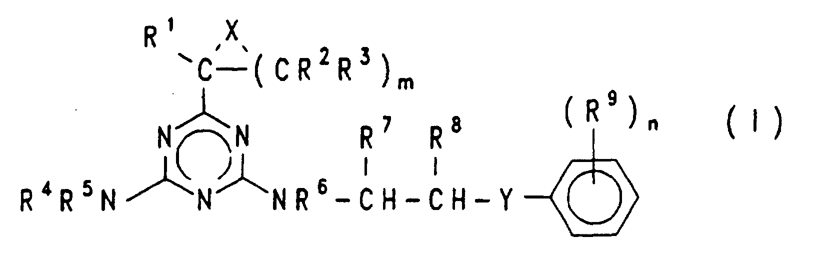 worin
- R¹: Wasserstoff, Halogen, Nitro, Cyano, Thiocyanato oder einen Rest der Formel -Z¹-R¹⁰,
- R² und R³: jeweils unabhängig voneinander Wasserstoff, Halogen, Nitro, Cyano, Thiocyanato oder einen Rest der Formel -Z²-R¹¹ oder
R² und R³ gemeinsam mit dem C-Atom der Gruppe CR²R³ einen carbocyclischen Rest mit 4 bis 10 Ringgliedern oder einen heterocyclischen Rest mit 4 bis 10 Ringgliedern und mit Heteroringatomen aus der Gruppe N, O und S, wobei jeder der letztgenannten zwei Reste unsubstituiert oder durch einen oder mehrere Reste aus der Gruppe Halogen, (C₁-C₄)Alkyl und Oxo substituiert ist,
- R⁴ und R⁵: jeweils unabhängig voneinander Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest oder
R⁴ und R⁵ gemeinsam mit dem Stickstoffatom der Gruppe NR⁴R⁵ einen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder substituiert ist,
- R⁶: Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest,
- R⁷ und R⁸: jeweils unabhängig voneinander Wasserstoff, Halogen, Nitro, Cyano, Thiocyanato oder einen Rest der Formel -Z³-R¹² oder R⁷ und R⁸ gemeinsam eine Alkylenkette mit 2 bis 6 C-Atomen, die unsubstituiert oder durch einen oder mehrere Reste aus der Gruppe Halogen, (C₁-C₄)Alkyl und Oxo substituiert ist,
- R⁹: jeweils unabhängig voneinander Halogen, Nitro, Formyl, Carboxy, Cyano, Thiocyanato, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, (C₁-C₄)Alkylcarbonyl, (C₁-C₄)Alkyloxycarbonyl, (C₁-C₄)Alkoxy-thiocarbonyl, (C₁-C₄)Alkylthiocarbonyl oder (C₁-C₄)Alkylthio-thiocarbonyl, wobei die Alkylreste in den letztgenannten 11 Resten unsubstituiert oder substituiert sind, oder (C₁-C₄)Alkylsulfonyl, (C₁-C₄)Halogenalkylsulfonyl, (C₁-C₄)Alkoxysulfonyl, Amino, Mono- oder Di-[(C₁-C₆)alkyl]amino, Aminocarbonyl, Mono- oder Di-[(C₁-C₆)alkyl]aminocarbonyl, (C₁-C₆)Alkanoylamino, N-(C₁-C₆)Alkanoyl-N-(C₁-C₄)alkyl-amino oder einen Rest der Formel Z⁴-R^{o}, wobei Z⁴ wie unten definiert und R^{o} einen aromatischen, gesättigten oder teilgesättigten carbocyclischen oder heterocyclischen Rest bedeutet, wobei der cyclische Rest substituiert oder unsubstituiert ist, oder zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
- R¹⁰, R¹¹, R¹²: jeweils unabhängig voneinander H oder einen acyclischen Kohlenwasserstoffrest beispielsweise jeweils mit 1 bis 20 C-Atomen, vorzugsweise mit 1 bis 10, oder einen cyclischen Kohlenwasserstoffrest, vorzugsweise mit 3 bis 8 C-Atomen, insbesondere 3 bis 6 C-Atomen, oder einen heterocyclischen Rest, vorzugsweise mit 3 bis 9 Ringatomen und 1 bis 3 Heteroringatomen aus der Gruppe N, O und S, wobei jeder der drei letztgenannten Reste unsubstituiert oder substituiert ist,
- X: eine Gruppe der Formel -O-, -S(O)_{q}-, -NR*- oder -N(O)-, wobei q = 0, 1 oder 2 ist und R* Wasserstoff oder Alkyl mit 1 bis 4 C-Atomen ist, oder eine Gruppe der Formel CR¹³R¹⁴, wobei die Definitionen für R¹³ und R¹⁴ aus den für R² und R³ möglichen Resten ausgewählt sind,
- Y: eine direkte Bindung oder eine Gruppe der Formel -O-, -S(O)ᵣ-, -NR**- oder -N(O)-, wobei r = 0, 1 oder 2 ist und R** Wasserstoff oder Alkyl mit 1 bis 4 C-Atomen ist, oder eine Gruppe der Formel -CH₂-, -C(CH₃)H- oder -C(CH₃)₂-,
- Z¹, Z², Z³, Z⁴: jeweils unabhängig voneinander eine direkte Bindung oder eine divalente Gruppe der Formel
-O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'- bedeutet und dabei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
- m: 1, 2, 3 oder 4, insbesondere 1 oder 2, und
- n: 0, 1, 2, 3, 4 oder 5, vorzugsweise 0, 1, 2, 3 oder 4, insbesondere 2 oder 3,
bedeuten.

Die Verbindungen der Formel (I) können durch Anlagerung einer geeigneten anorganischen oder organischen Säure, wie beispielsweise HCI, HBr, H₂SO₄ oder HNO₃, aber auch Oxalsäure oder Sulfonsäuren an eine basiche Gruppe, wie z.B. Amino oder Alkylamino, Salze bilden. Geeignete Substituenten, die in deprotonierter Form, wie z.B. Sulfonsäuren oder Carbonsäuren, vorliegen können, können innere Salze mit ihrerseits protonierbaren Gruppen, wie Aminogruppen bilden. Salze können ebenfalls dadurch gebildet werden, daß bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, z.B. Cyclopropyl, Cyclopentyl oder Cyclohexyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl
(= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.
Ein heterocyclischer Rest oder Ring (Heterocyclyl) kann gesättigt, ungesättigt oder heteroaromatisch sein; er enthält vorzugsweise eine oder mehrere Heteroeinheiten im Ring, d.h. Heteroatome oder Ringglieder, welche auch substituierte Heteroatome einschließen, vorzugsweise aus der Gruppe N, O, S, SO, SO₂; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, mund p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse, worin
- R¹: H. Halogen, NO₂, CN, SCN oder -Z¹-R¹⁰,
- R² und R³: jeweils unabhängig voneinander H, Halogen, NO₂, CN, SCN oder einen Rest der Formel -Z²-R¹¹ oder
R² und R³ gemeinsam mit dem C-Atom der Gruppe CR²R³ (C₃-C₈)Cycloalkyl, (C₃-C₈)Cycloalkenyl oder einen heterocyclischen Rest mit 3 bis 6 Ringgliedern und mit Heteroringatomen aus der Gruppe N, O und S, wobei jeder der letztgenannten 3 Reste unsubstituiert oder durch einen oder mehrere Reste aus der Gruppe Halogen, (C₁-C₄)Alkyl und Oxo substituiert ist,
- R⁴ und R⁵: jeweils unabhängig voneinander Wasserstoff, Amino, Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido,
[(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl,
(C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
- R⁶: Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy,
(C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest,
- R⁷ und R⁸: jeweils unabhängig voneinander H, Halogen, NO₂, CN, SCN oder einen Rest der Formel -Z³-R¹² oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
- R⁹: jeweils unabhängig voneinander Halogen, Nitro, Formyl, Carboxy, Cyano, Thiocyanato, (C₁-C₆)Alkyl, (C₁-C₆)Alkoxy, (C₁-C₄)Alkylcarbonyl, (C₁-C₄)Alkyloxycarbonyl oder (C₁-C₄)Alkylthiocarbonyl, wobei die letztgenannten fünf Reste unsubstituiert oder durch Halogen oder (C₁-C₄)Alkoxy substituiert sind, oder (C₁-C₄)Alkylsulfonyl, (C₁-C₄)Haloalkylsulfonyl, Mono- oder Di-[(C₁-C₄)alkyl]amino, Aminocarbonyl, Mono- oder Di-[(C₁-C₄)alkyl]aminocarbonyl, (C₁-C₄)Alkanoylamino oder einen Rest der Formel Z⁴-R^{o}, worin Z⁴ wie unten definiert ist und R^{o} (C₃-C₆)Cycloalkyl, (C₃-C₆)Cycloalkenyl oder Phenyl bedeutet, wobei jeder der letztgenannten drei Reste unsubstituiert oder durch Reste aus der Gruppe Halogen, (C₁-C₄)Alkyl, (C₁-C₄)Haloalkyl und (C₁-C₄)Alkoxy substituiert ist, oder
zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
- R¹⁰, R¹¹, R¹²: jeweils unabhängig voneinander H oder einen acyclischen Kohlenwasserstoffrest mit 1 bis 6 C-Atomen, einen cyclischen Kohlenwasserstoffrest mit 3 bis 6 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxyl, (C₁-C₄)Haloalkoxy, (C₁-C₄)Alkylthio,
(C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl,
(C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
vorzugsweise R¹⁰, R¹¹, R¹² Wasserstoff, (C₁-C₈)Alkyl, (C₂-C₆)Alkenyl, (C₂-C₆)Alkinyl, (C₃-C₉)Cycloalkyl, (C₃-C₉)Cycloalkenyl, Phenyl,
Heterocyclyl mit 3 bis 6 Ringatomen und 1 bis 3 Heteroringatomen aus der Gruppe N, O und S, wobei jeder der letztgenannten 7 Reste unsubstituiert oder durch einen oder mehrere Reste aus der Gruppe Halogen, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl, (C₁-C₄)Alkylsulfonyl und, im Fall cyclischer Reste, auch (C₁-C₄)Alkyl und (C₁-C₄)Haloalkyl substituiert ist,
- x: eine Gruppe der Formel -O-, -S(O)_{q}-, -NR*- oder -N(O)-, wobei q = 0, 1 oder 2 ist und R* Wasserstoff oder Alkyl mit 1 bis 4 C-Atomen ist, oder eine Gruppe der Formel CR¹³R¹⁴, wobei die Definitionen für R¹³ und R¹⁴ aus den für R² und R³ möglichen Resten ausgewählt sind,
- Y: eine direkte Bindung oder eine Gruppe der Formel -O-, -S(O)ᵣ-, -NR**- oder - N(O)-, wobei r = 0, 1 oder 2 ist und R** Wasserstoff oder Alkyl mit 1 bis 4 C-Atomen ist, oder eine Gruppe der Formel -CH₂-, -C(CH₃)H- oder -C(CH₃)₂-,
- Z¹, Z², Z³, Z⁴: jeweils unabhängig voneinander eine direkte Bindung oder eine divalente Gruppe der Formel
-O-, -S-, -CO-, -O-CO-, -CO-O-, -NR'-, -NR'-CO- oder -CO-NR'-, wobei R' H oder (C₁-C₄)Alkyl ist, vorzugsweise Z¹, Z², Z³, Z⁴ eine direkte Bindung oder Sauerstoff
bedeuten.

Von besonderem Interesse sind weiterhin erfindungsgemäße Verbindungen der Formel (I) und deren Salze, worin
- R¹, R², R³: unabhängig voneinander Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
- R² und R³: gemeinsam mit dem C-Atom der Gruppe CR²R³ einen gesättigten oder teilweise ungesättigten carbocyclischen Rest mit 3 bis 6 Ringgliedern oder Heterocyclyl mit 3 bis 6 Ringgliedern und 1 bis 3 Heteroringatomen aus der Gruppe O, N und S, wobei jeder der letztgenannten 2 cyclischen Reste unsubstituiert oder mit einem oder mehreren Resten aus der Gruppe (C₁-C₄)Alkyl, Halogen und Oxo substituiert ist,
- R⁴ und R⁵: unabhängig voneinander Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl mit 3 bis 9 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
- R⁶: Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl mit 3 bis 9 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
- R⁷ und R⁸: unabhängig voneinander Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
- R⁹,: wenn n = 1 ist, und die Reste R⁹, jeweils unabhängig voneinander, wenn n größer als 1 ist, Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
- x: eine Gruppe der Formel -O-, -S- oder -NR*-, wobei R* Wasserstoff oder Alkyl mit 1 bis 4 C-Atomen ist, oder
eine Gruppe der Formel CR¹³R¹⁴, wobei die Definitionen für R¹³ und R¹⁴ aus den für R² und R³ möglichen Resten ausgewählt sind,
- Y: eine direkte Bindung oder eine Gruppe der Formel -O-, -S- oder -NR**-, wobei R** Wasserstoff oder Alkyl mit 1 bis 4 C-Atomen ist, oder eine Gruppe der Formel -CH₂-, -C(CH₃)H- oder -C(CH₃)₂-
bedeuten.

Von besonderem Interesse sind auch erfindungsgemäße Verbindungen der Formel (I) und deren Salze, worin
- R¹: Wasserstoff, Halogen, Hydroxy, Amino, (C₁-C₄)Alkyl, (C₁-C₄)Alkoxy, (C₁-C₄)Alkylamino, Di-[(C₁-C₄)alkyl]-amino, Halo-(C₁-C₄)alkyl, (C₁-C₄)Alkoxy-(C₁-C₄)alkyl, (C₂-C₆)Alkenyl, (C₂-C₆)Alkinyl, Phenyl, Phenoxy oder einen Phenyl- oder Phenoxyrest, der im Phenylteil durch einen oder mehrere, vorzugsweise bis zu insgesamt drei, Reste aus der Gruppe Halogen, (C₁-C₄)Alkyl, (C₁-C₄)Alkoxy und (C₁-C₄)Alkoxy-carbonyl substituiert ist,
- R² und R³: unabhängig voneinander Wasserstoff, Halogen, Hydroxy, Amino, (C₁-C₄)Alkyl, (C₁-C₄)Alkoxy, (C₁-C₄)Alkylamino, Di-[(C₁-C₄)alkyl]-amino, Halo-(C₁-C₄)alkyl, (C₁-C₄)Alkoxy-(C₁-C₄)alkyl, (C₂-C₆)Alkenyl, (C₂-C₆)Alkinyl, Phenyl oder Phenoxy oder einen Phenyl- oder
Phenoxyrest, der im Phenylteil durch einen oder mehrere, vorzugsweise bis zu insgesamt drei, Reste aus der Gruppe Halogen, (C₁-C₄)Alkyl, (C₁-C₄)Alkoxy und (C₁-C₄)Alkoxy-carbonyl substituiert ist, oder
R² und R³ gemeinsam mit dem C-Atom der Gruppe CR²R³ einen gesättigten carbocyclischen Rest mit 3 bis 6 Ringgliedern, der unsubstituiert oder mit einem oder mehreren Resten aus der Gruppe (C₁-C₄)Alkyl, Halogen und Oxo substituiert ist,
- R⁴ und R⁵: unabhängig voneinander Wasserstoff, Amino, Formyl, (C₁-C₄)Alkyl, (C₁-C₄)Alkoxy-(C₁-C₄)alkyl, (C₂-C₆)Alkenyl, (C₂-C₆)Alkinyl, (C₁-C₄)Alkylamino-(C₁-C₄)alkyl, Di-[(C₁-C₄)alkyl]-amino-(C₁-C₄)alkyl oder Phenyl, Phenyl-(C₁-C₄)alkyl oder Phenoxy-carbonyl oder einen der letztgenannten drei Reste, der im Phenylteil bis zu dreifach durch Reste aus der Gruppe Halogen, (C₁-C₄)Alkyl, (C₁-C₄)Alkoxy und (C₁-C₄)Alkoxy-carbonyl substituiert ist, oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N und O ausgewählt ist und der Rest unsubstituiert oder durch einen oder mehrere Reste aus der Gruppe Halogen, (C₁-C₄)Alkyl und Oxo substituiert ist,
- R⁶: Wasserstoff, Amino, Formyl, (C₁-C₄)Alkyl, Di-[(C₁-C₄)alkyl]-amino, (C₁-C₄)Alkoxy-(C₁-C₄)alkyl, (C₂-C₆)Alkenyl, (C₂-C₆)Alkinyl, (C₁-C₄)Dialkylamino-(C₁-C₄)alkyl, Phenyl, Phenoxy-(C₁-C₄)alkyl, Phenyl-(C₁-C₄)alkyl, Phenoxy-carbonyl, Phenylamino-carbonyl oder einen der letztgenannten fünf Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist,
- R⁷ und R⁸: unabhängig voneinander Wasserstoff, (C₁-C₄)Alkyl, (C₁-C₄)Alkoxy-(C₁-C₄)alkyl, (C₂-C₆)Alkenyl, (C₂-C₆)Alkinyl, Di-[(C₁-C₄)alkyl]-amino-(C₁-C₄)alkyl, Phenyl, Phenoxy-(C₁-C₄)alkyl, Phenyl-(C₁-C₄)alkyl oder einen der letztgenannten drei Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist, oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
- R⁹: unabhängig voneinander Wasserstoff, Halogen, Hydroxy, Amino, Nitro, Cyano, (C₁-C₄)Alkyl, (C₁-C₄)Alkoxy, (C₁-C₄)Alkylamino, Di-[(C₁-C₄)alkyl]amino, Perhalo-(C₁-C₄)alkyl, Hydroxy-(C₁-C₄)alkyl, (C₁-C₄)Alkoxy-(C₁-C₄)alkyl, (C₁-C₄)Alkylthio, (C₂-C₆)Alkenyl, (C₂-C₆)Alkinyl, Di-[(C₁-C₄)alkyl]-amino-(C₁-C₄)alkyl, Phenyl, Phenoxy, Phenylcarbonyl, Phenylcarbonyl-(C₁-C₄)alkyl, (C₁-C₄)Alkoxycarbonyl-(C₁-C₄)alkyl, (C₁-C₄)Alkylcarbonyl, (C₁-C₄)Alkyloxycarbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 13 Reste, der im acyclischen Teil oder, vorzugsweise, im cyclischen Teil durch einen oder mehrere Reste aus der Gruppe Halogen, (C₁-C₄)Alkyl, (C₁-C₄)Alkoxy, (C₁-C₄)Alkylthio, (C₁-C₄)Haloalkyl, (C₁-C₄)Haloalkoxy und (C₁-C₄)Alkoxy-carbonyl substituiert ist, wobei Heterocyclyl in den Resten 3 bis 6 Ringatome und 1 bis 3 Heteroringatome aus der Gruppe N und O aufweist, oder
zwei benachbarte Reste R⁹ gemeinsam einen ankondensierten Cyclus mit 4 bis 6 Ringatomen, der carbocyclisch ist oder Heteroringatome aus der Gruppe O und N enthält und der unsubstituiert oder durch einen oder mehrere Reste (C₁-C₄)Alkyl substituiert ist,
- x: eine Gruppe der Formel -O- oder -NR*-, wobei R* Wasserstoff oder Methyl ist, oder
eine Gruppe der Formel CR¹³R¹⁴, wobei die Definitionen für R¹³ und R¹⁴ aus den für R² und R³ möglichen Resten ausgewählt sind,
- Y: eine direkte Bindung oder eine Gruppe der Formel -O- oder -NR**-, wobei R** Wasserstoff oder Methyl ist,
bedeuten.

Bevorzugte erfindungsgemäße Verbindungen der Formel (I) und deren Salze sind solche, worin
- R¹: Wasserstoff, Halogen oder (C₁-C₄)Alkyl,
- R² und R³: unabhängig voneinander Wasserstoff, Halogen, (C₁-C₄)Alkyl oder Phenyl oder
R² und R³ gemeinsam mit dem C-Atom der Gruppe CR²R³ gesättigtes (C₄-C₆)Cycloalkyl,
- R⁴ und R⁵: unabhängig voneinander Wasserstoff, Amino, Formyl oder (C₁-C₄)Alkyl oder
R⁴ und R⁵ gemeinsam mit dem Stickstoffatom der Gruppe NR⁴R⁵ einen heterocyclischen Rest mit 4 bis 6 Ringatomen und 1 bis 2 Heteroringatomen, wobei neben dem N-Atom das gegebenenfalls weitere Heteroringatom aus der Gruppe N und O ausgewählt ist,
- R⁶: Wasserstoff oder (C₁-C₄)Alkyl,
- R⁷ und R⁸: unabhängig voneinander Wasserstoff, (C₁-C₄)Alkyl oder (C₁-C₄)Alkoxy-(C₁-C₄)alkyl oder
R⁷ und R⁸ gemeinsam eine Alkylenkette mit 2 bis 4 C-Atomen,
- R⁹: unabhängig voneinander Wasserstoff, Halogen, Hydroxy, (C₁-C₄)Alkyl oder (C₁-C₄)Alkoxy,
- m: 1 oder 2,
- n: 2 oder 3,
- x: eine Gruppe der Formel -O- oder -NH- oder
eine Gruppe der Formel CR¹³R¹⁴, wobei die Definitionen für R¹³ und R¹⁴ aus den für R² und R³ möglichen Resten ausgewählt sind,
- Y: eine direkte Bindung oder eine Gruppe der Formel -O- oder -NH-
bedeuten.

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II), 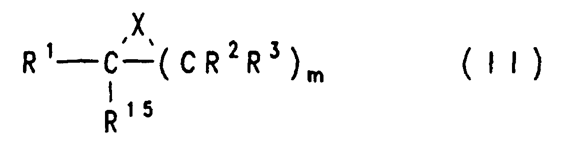 worin R¹⁵ eine funktionelle Gruppe aus der Gruppe Carbonsäureester, Carbonsäureorthoester, Carbonsäurechlorid, Carbonsäureamid, Carbonsäureanhydrid und Trichlormethyl bedeutet,
   mit einem Biguanidid der Formel (III) oder einem Säureadditionssalz hiervon 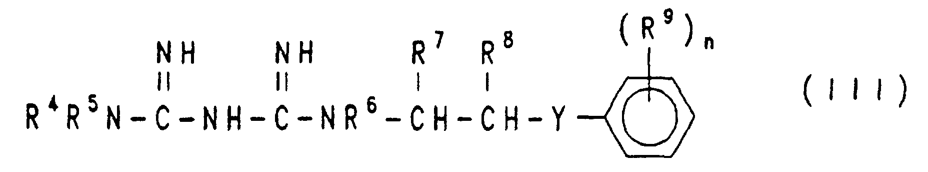 umsetzt oder
b) eine Verbindung der Formel (IV), 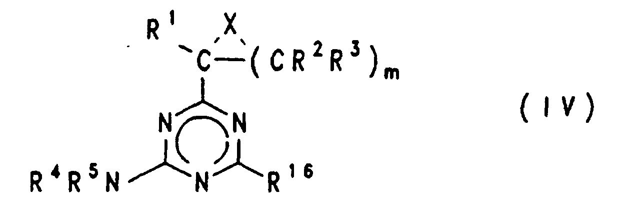 worin R¹⁶ einen austauschfähigen Rest oder eine Abgangsgruppe, z.B.
   Chlor, Trichlormethyl, (C₁-C₄)Alkylsulfonyl und unsubstituiertes oder substituiertes Phenyl-(C₁-C₄)alkylsulfonyl oder (C₁-C₄)Alkylphenylsulfonyl, bedeutet, mit einem geeigneten Amin der Formel (V) oder einem Säureadditionssalz hiervon 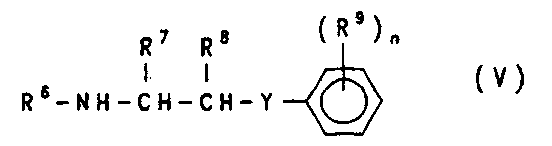 umsetzt, oder
c) unter Reaktion (nucleophiler Substitution) einer austauschfähigen Gruppe am Triazin der Verbindung der genannten Formel (IV) oder der Formel (X), 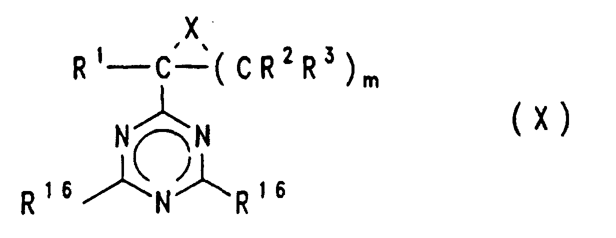 worin jeder der Reste R¹⁶ unabhängig voneinander einen austauschfähigen Rest oder eine Abgangsgruppe, z.B. Chlor, Trichlormethyl, (C₁-C₄)Alkylsulfonyl und unsubstituiertes oder substituiertes Phenyl-(C₁-C₄)alkylsulfonyl, bedeutet, diese Verbindung mit einem geeigneten Aziridin der Formel (XI), 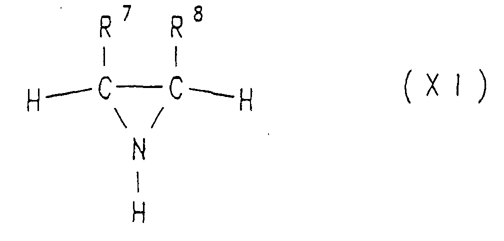 worin R⁷ und R⁸ wie in Formel (I) definiert sind, und nachfolgend mit einem geeigneten Nucleophil der Formel (XII) 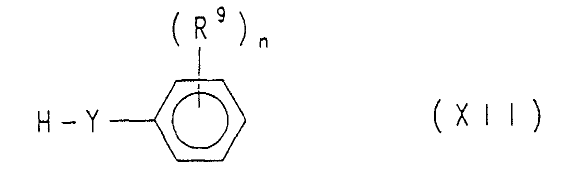 unter Ringöffnung des Azirididinrings und, falls ein Triazin der Formel (X) eingesetzt wurde, durch Reaktion der noch vorhandenen austauschfähigen Gruppe am Triazingrundgerüst mit einer Verbindung der Formel NHR⁴R⁵ (Ammoniak oder Amin) umsetzt oder
d) eine Verbindung der Formel (XIII) 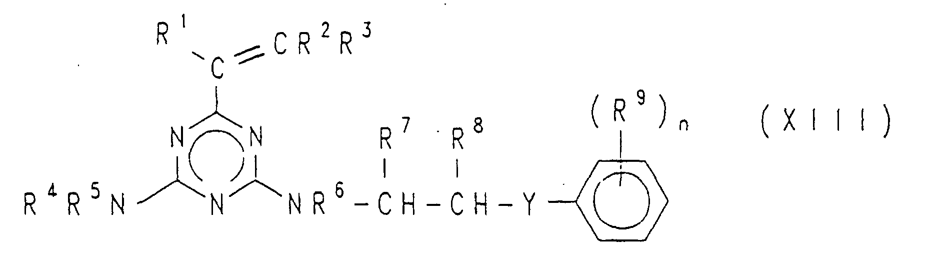 durch Epoxidierung oder Cyclopropanierung in entsprechende Verbindungen der Formel (I) umsetzt,
wobei in den Formeln (II), (III), (IV), (V), (X), (XI), (XII) und (XIII) die Reste R¹, R², R³, R⁴, R⁵, R⁶, R⁷, R⁸, R⁹ sowie X, Y, m und n wie in Formel (I) definiert sind, mit der Maßgabe, daß in Variante d) für Formel (I) m = 1 ist.

Die Umsetzung der Verbindungen der Formel (II) und (III) erfolgt vorzugsweise basenkatalysiert in einem inerten organischen Lösungsmittel, wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei beispielsweise im Bereich von 0,1 bis 3 Moläquivalenten bezogen auf die Verbindung der Formel (III) eingesetzt. Die Verbindung der Formel (II) kann im Verhältnis zur Verbindung der Formel (III) beispielsweise äquimolar oder mit bis zu 2 Moläquvälenten Überschuß eingesetzt werden. Grundsätzlich sind die entsprechenden Verfahren in der Literatur bekannt (vergleiche: Comprehensive Heterocyclic Chemistry, A.R. Katritzky, C.W. Rees, Pergamon Press, Oxford, New York, 1984, Vol.3; Part 2B; ISBN 0-08-030703-5, S.290).

Die Umsetzung der Verbindungen der Formel (IV) und (V) erfolgt vorzugsweise basenkatalysiert in einem inerten organischen Lösungsmittel, wie z.B. THF, Dioxan, Acetonitril, DMF, Methanol und Ethanol, bei Temperaturen zwischen -10 °C und dem Siedepunkt des jeweiligen Lösungsmittels oder Lösungsmittelgemisches, vorzugsweise bei 20 °C bis 60 °C, wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in der Regel im Bereich von 1 bis 3 Moläquivalenten bezogen auf die Verbindung der Formel (IV) eingesetzt, die Verbindung der Formel (IV) kann beispielsweise äquimolar zur Verbindung der Formel (V) oder mit bis zu 2 Moläquvalenten Überschuß eingesetzt werden. Grundsätzlich sind die entsprechenden Verfahren aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030703-5, S. 482).

Die Edukte der Formeln (II), (III), (IV) und (V) sind entweder kommerziell erhältlich oder können nach oder analog literaturbekannten Verfahren hergestellt werden. Die Verbindungen können beispielsweise auch nach einem der nachfolgend beschriebenen Verfahren hergestellt werden.

Die Verbindungen der Formel (III) können beispielsweise durch Reaktion der Amine der Formel (V) oder deren Salze mit Cyanoguanidin in einem inerten Lösungsmittel wie beispielsweise Chlorbenzol oder Diethylenglykol oder Tetrahydrofuran oder gegebenenfalls Wasser analog H.M. Eisa, A.S. Tantawy, M.M. El-Kerdawy, Pharmazie 46 (1991), 182-184 hergestellt werden. Gegebenenfalls kann der Zusatz von anorganischen Salze wie Eisen(III)chlorid die Reaktion beschleunigen oder eine Erniedrigung der Reaktionstemperatur bewirken (Sanwa Chem KK, JP 62215556).

Die Amine der Formel (V) oder Salze hiervon können beispielsweise durch Reduktion der entsprechenden Oxime mit beispielsweise LiAlH₄ (Bristol Labor. Inc. US 2703324) oder Hydrierung mit Wasserstoff und Raney Nickel (Suter, Zuter, Justus Liebigs Ann. Chem. 576 (1952), S. 215 ff, oder auch durch reduktive Aminierung aus den entsprechenden Acetonderivaten (vergl. A. Waefelaer, et. al., Bull. Soc. Chim. Belg. 85 (1976), 421-425) hergestellt werden.

Die Verbindung der Formel (IV), oder eine direkte Vorstufe davon, läßt sich beispielweise wie folgt herstellen:
1. Durch Reaktion einer Verbindung der Formel (II) mit einem Amidino-thioharnstoff-Derivat der Formel (VI), 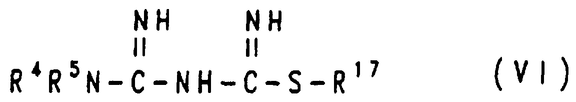 worin R¹⁷ (C₁-C₄)-Alkyl oder Phenyl-(C₁-C₄)-alkyl bedeutet und R⁴ und R⁵ wie in Formel (I) definiert sind, werden Verbindungen der Formel (IV) erhalten, in denen R¹⁶ = -SR¹⁷ bedeutet.
2. Durch Umsetzung eines cyclischen Amidins der Formel (VII) oder eines Säureadditionssalzes davon, 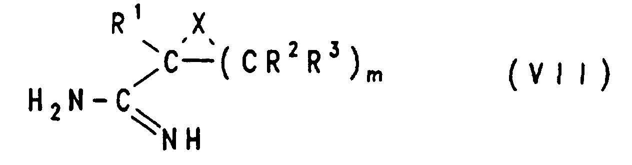 worin R¹, R², R³, X und m wie in Formel (I) definiert sind, mit einem N-Cyanodithioiminocarbonat der Formel (VIII), 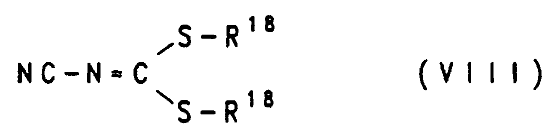 worin R¹⁸ (C₁-C₄)-Alkyl oder Phenyl-(C₁-C₄)-alkyl bedeutet, werden Verbindungen der Formel (IV) erhalten, worin R¹⁶ = -S-R¹⁸ bedeutet.
3. Durch Umsetzung eines Alkali-dicyanamids mit einem cyclischen Carbonsäurederivat der genannten Formel (II) werden Verbindungen der Formel (IV) erhalten, worin R¹⁶ = NH₂ bedeutet,
4. Durch Umsetzung von Trichloracetonitril mit einem cyclischen Carbonsäurenitril der Formel (IX), 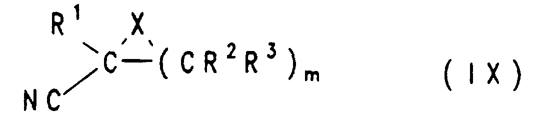 worin R¹, R², R³, X und m wie in Formel (I) definiert sind, werden zunächst Verbindungen der Formel (X), worin R¹⁶ = CCl₃ ist, erhalten, welche durch nachfolgende Umsetzung mit Verbindungen der Formel HNR⁴R⁵ (R⁴ und R⁵ wie in Formel (I)), zu Verbindungen der Formel (IV), worin R¹⁶ = CCl₃ bedeutet, führen.

Gegebenenfalls können analog den vorstehenden Verfahren unter 1.-4. auch Zwischenprodukte der Formel (X), 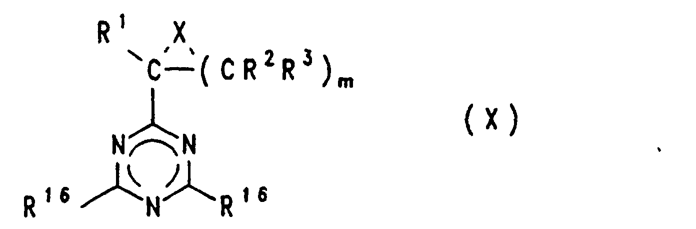 mit 2 austauschfähigen Gruppen R¹⁶ hergestellt werden (vgl. Formel (IV)) und die austauschfähigen Gruppen nacheinander mit geeigneten Aminen oder Ammoniak substituiert werden, um analog allgemein bekannten Verfahrensweisen zu Verbindungen der Formel (I) zu gelangen. Gegebenfalls können analog den vorstehenden Verfahren unter 1.-4. erhaltene Zwischenprodukte der Formel (X), worin R¹⁶ (C₁-C₄)-Alkylthio oder Phenyl-(C₁-C₄)-alkylthio bedeutet, durch eine Chlorierung oder Oxidation in reaktionsfähigere Derivate der Formeln (X) umgewandelt werden.

Ebenso sind nach literaturbekannten Verfahren oder nach den zuvor genannten Verfahren, wobei dann offenkettige, olefinische Derivate analog zu Verbindungen der Formel (II), (VII), (IX), in denen X eine C-C-Bindung und m = 1 darstellten, eingesetzt werden, Verbindungen der Formel (XIII) herstellbar.

Die Umsetzung der Carbonsäurederivate der Formel (II) mit den Amidinothioharnstoff-Derivaten der Formel (VI) erfolgt vorzugsweise basenkatalysiert in einem organischen Lösungsmittel, wie z.B. Aceton, THF, Dioxan, Acetonitril, DMF, Methanol, Ethanol, bei Temperaturen von -10 °C bis zum Siedepunkt des Lösungsmittel, vorzugsweise bei 0 °C bis 20 °C. Die Umsetzung kann aber auch in Wasser oder in wässrigen Lösungsmitttelgemischen mit einem oder mehreren der obengenannten organischen Lösungsmitteln erfolgen. Falls (VI) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei im Bereich von 1 bis 3 Moläquivalenten bezogen auf die Verbindung der Formel (VI) eingesetzt. Verbindungen der Formel (II) und (VI) können beispielsweise äquimolar oder mit bis zu 2 Moläquivalenten Überschuß an Verbindung der Formel (II) eingesetzt werden. Grundsätzlich sind die entsprechenden Verfahren literaturbekannt (vergl.: H. Eilingsfeld, H. Scheuermann, Chem. Ber.; 1967, 100, 1874), die entsprechenden Zwischenprodukte der Formel (IV) sind neu.

Die Umsetzung der cyclischen Amidine der Formel (VII) mit den N-Cyanodithioiminocarbonaten der Formel (VIII) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10 °C bis zum Siedepunkt des Lösungsmittels, vorzugsweise bei 20 °C bis 80 °C. Falls (VII) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in Bereich von 1 bis 3 Moläquivalenten bezogen auf die Verbindung der Formel (VIII) eingesetzt, Verbindungen der Formel (VII) und (VIII) können in der Regel äquimolar oder mit 2 Moläquvalenten Überschuß an Verbindung der Formel (II) eingesetzt werden. Grundsätzlich sind die entsprechenden Verfahren literaturbekannt (vergl.: T.A. Riley, W.J. Henney, N.K. Dalley, B.E. Wilson, R.K. Robins; J. Heterocyclic Chem.; 1986, 23 (6), 1706-1714), die entsprechenden Zwischenprodukte der Formel (IV) sind neu.

Die Herstellung von Zwischenprodukten der Formel (X) mit R¹⁶ = Chlor kann durch Reaktion von Alkali-dicyanamid mit einem cyclischen Carbonsäurederivat der Formel (II), wobei dann R¹⁵ bevorzugt die funktionelle Gruppe Carbonsäurechlorid oder Carbonsäureamid bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10 °C und dem Siedepunkt des Lösungsmittels, vorzugsweise bei 20 °C bis 80 °C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCI, oder auch Lewis-Säuren, wie z.B. AlCl₃ oder BF₃ (vergl. US-A-5095113, DuPont).

Die Herstellung von Zwischenprodukten der Formel (X) mit R¹⁶ = Trihalogenmethyl kann durch Reaktion der entsprechenden Trihalogenessigsäurenitrile mit einem cyclischen Carbonsäurenitril der Formel (IX) erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -40 °C und dem Siedepunkt des Lösungsmittels, vorzugsweise bei -10 °C bis 30 °C. Geeignete Säuren sind z.B. Halogenwasserstoffsäuren wie HCI oder auch Lewis-Säuren wie z.B. AlCl₃ oder BF₃ (vgl. EP-A-130939, Ciba Geigy).

Zwischenprodukte der Formel (IV) oder (X), worin R¹⁶ = (C₁-C₄)Alkylmercapto oder unsubstituiertes Phenyl-(C₁-C₄)-alkylmercapto ist, können in einem inerten organischen Lösungsmittel wie z .B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosporoxychlorid zu reaktionsfähigeren Chlortriazinen der Formel (IV) oder (X), worin R¹⁶ = Cl ist, überführt werden (vgl. J.K. Chakrabarti, D.E. Tupper; Tetrahedron 1975, 31(16), 1879-1882).

Zwischenprodukte der Formel (IV) oder (X), wobei R¹⁶ = (C₁-C₄)Alkylmercapto oder unsubstituiertes oder substituiertes Phenyl-(C₁-C₄)-alkylmercapto oder (C₁-C₄)Alkyl-phenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0 °C und dem Siedepunkt des Lösungsmittels, vorzugsweise von 20 °C bis 80 °C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Verbindungen analog der Formel (IV) erhält man auch durch selektive nucleophile Substitution einer austauschfähigen Gruppe bei Verbindungen analog der Formel (X), wobei R¹⁶ beispielsweise für Halogen, Perhalomethyl, (C₁-C₄)Alkylsulfinyl, (C₁-C₄)Alkylsulfonyl oder andere literaturbekannte Abgangsgruppen steht, in einem geeigneten Lösungsmittel wie z .B. THF, Dioxan, Alkohole, DMF oder Acetonitril oder Mischungen hiervon bei Temperaturen zwischen -10 °C und dem Siedepunkt des Lösungsmittels, vorzugsweise bei 10 °C bis 25 °C, gegebenfalls unter basischen Bedingungen. Als Basen eignen sich dabei Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in Bereich von 1 bis 3 Moläquivalenten bezogen auf die Verbindung der Formel (X) eingesetzt; das Nucleophil wird in der Regel äquimolar bis zu 2 Moläquivalenten im Überschuß eingesetzt und kann gegebenfalls auch selbst als Base gebraucht werden. Grundsätzlich sind die entsprechenden Verfahren literaturbekannt (vergl.: V.I. Kaelarev, Dibi Ammar, A.F. Lunin; Ximinya Geterosikl. Soedin., 1985, N11, 1557 - 1563).

Analog kann man durch Substitution einer Abgangsguppe von Triazinen der Formel (IV) oder (X) mit Aziridinen der Formel (XI) gegebenfalls unter Basenzusatz in einem geeigneten inerten Lösungsmittel wie z.B. THF, Dioxan, Alkohole, DMF oder Acetonitril oder Mischungen hiervon bei Temperaturen zwischen -10 °C und dem Siedepunkt des Lösungsmittels, vorzugsweise bei 20 °C bis 80 °C, Aziridinyltriazine herstellen. Als Basen eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in Bereich von 1 bis 3 Moläquivalenten bezogen auf die Verbindung der Formel (IV) oder (X) eingesetzt; das Aziridin kann mit bis zu 3 Moläquvalenten überschüssig eingesetzt und gegebenfalls auch selbst als Base gebraucht werden. Nachfolged kann der Aziridin-Ring geöffnet werden. Hierzu werden beispielsweise in einem geeigneten Lösungsmittel wie z.B. THF, Dioxan, Alkohole, DMF oder Acetonitril die Zwischenverbindungen mit Nucleophilen der Formel (XII) bei Temperaturen zwischen -10 °C und dem Siedepunkt des Lösungsmittels, vorzugsweise bei 20 °C bis 80 °C, zur Reaktion gebracht. Das Verfahren der Ringöffnung ist im Prinzip bekannt (vergl.: M.D. Nair, J.D. Nagarajan, Indian J. Chem.; 1985, 24B, 940; oder JP 03005466; Chem. Abstracts 115:4914); neu ist die Ringöffnung bei Triazinylderivaten.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat, Kalium-tert.Butylat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria. Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie lnertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoyl-prop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P ( = R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy)-propansäureund -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel 1

### 2-Amino-4-(2-methyl-1-oxaspiro[2.4]heptan-2-yl)-6-(1-(3,5-dimethylphenoxy)propyl-2-amino)-1,3,5-triazin

a) 9,3 g (0,11 mol) Cyclopentanon und 13,5 g (0,11 mol)
   2-Chlorpropionsäure-methylester werden auf 0 bis 2 °C gekühlt, bei dieser Temperatur werden unter Rühren 13,6 g (0,12 mol) Kalium-tert.-butylat, suspendiert in 200 ml Tetrahydrofuran, langsam in einem Zeitraum von ca. 90 min. zugetropft, anschließend wird noch 60 Minuten ohne Kühlung weiter gerührt. Zum Reaktionsgemisch wird etwas Wasser zugeben, diese Mischung wird mit Diethylether extrahiert, die organische Phase mit Natriumsulfat getrocknet, das Trockenmittel wird abfiltriert und nach Abdampfen des Lösungsmittel erhält man 10,6 g 2-Methyl-1-oxaspiro[2.4]heptan-2-carbonsäuremethylester, welcher ohne weitere Reinigung in der Folgestufe eingesetzt werden kann (siehe b).
b) Zu 5,4 g (0,018 mol) 2-Biguanidino-1-(3,5-dimethylphenoxy)-propan-hydrochlorid in 50 ml Methanol und 6 g gemahlenem Molsieb 3A wird eine aus 0,86 g (0,036 mol) Natrium und 50 ml Methanol hergestellte Methanolatlösung gegeben. Nachfolgend fügt man 5,3 g (0,028 mol) 2-Methyl-1-oxaspiro[2.4]heptan-2-carbonsäuremethylester hinzu und rührt 2 Stunden bei 25 °C, dann 4 Stunden bei 65 °C. Die Reaktionsmischung wird filtriert, das Filtrat eingeengt und der Rückstand mit Essigester aufgenommen. Diese Lösung wird mit Wasser gewaschen, die organische Phase abgetrennt und mit Natriumsulfat getrocknet. Das Trockenmittel wird abfiltiriert und die Essigesterphase eingedampft. Man erhält nach säulenchromatographischer Trennung über Kieselgel mit Essigester als Laufmittel 1,8 g (25 % d.Th.) 2-Amino-4-(2-methyl-1-oxaspiro[2.4]heptan-2-yl)-6-(1-(3,5-dimethylphenoxy)propyl-2-amino)-1,3,5-triazin.

### Beispiel 19

### 2-Amino-4-cyclobutyl-6-(1-(3,5-dimethylphenoxy)-propyl-2-amino)-1,3,5-triazin

Zu 7,2 g (0,025 mol) 2-Biguanidino-1-(3,5-dimethylphenoxy)-propan-hydrochlorid in 50 ml Methanol und 7 g gemahlenem Molsieb 3A wird eine aus 1,2 g (0.05 mol) Natrium und 100 ml Methanol hergestellte Methanolatlösung gegeben. Nachfolgend fügt man 5,2 g (0,045 mol) Cyclobutancarbonsäure-ethylester hinzu und rührt 2 Stunden bei 25 °C, dann 4 Stunden bei 65 °C. Die Reaktionsmischung wird filtriert, das Filtrat eingeengt und der Rückstand mit Essigester aufgenommen. Diese Lösung wird mit Wasser gewaschen, die organische Phase abgetrennt und mit Natriumsulfat getrocknet. Das Trockenmittel wird abfiltiriert und die Essigesterphase eingedampft. Man erhält nach säulenchromatographischer Trennung über Kieselgel mit Essigester als Laufmittel 3,5 g (39 % d.Th.) 2-Amino-4-cyclobutyl-6-(1-(3,5-dimethylphenoxy)-propyl-2-amino)-1,3,5-triazin.

### Beispiel 29

### 2-Amino-4-cyclopropyl-6-(1-(4-chlor-3,5-dimethylphenoxy)-propyl-2-amino)-1,3,5-triazin

Zu 10,3 g (0,03 mol) 2-Biguanidino-1-(4-chlor-3,5-dimethylphenoxy)-propan-hydrochlorid in 50 ml Ethanol und 10 g gemahlenem Molsieb 3A wird eine aus 1,5 g (0,06 mol) Natrium und 100 ml Ethanol hergestellte Ethanolatlösung gegeben. Nachfolgend fügt man 5,2 g (0,045 mol) Cyclopropancarbonsäureethylester hinzu und rührt 2 Stunden bei 25 °C, dann 4 Stunden bei 65 °C. Die Reaktionsmischung wird filtriert, das Filtrat eingeengt und der Rückstand mit Essigester aufgenommen. Diese Lösung wird mit Wasser gewaschen, die organische Phase abgetrennt und mit Natriumsulfat getrocknet. Das Trockenmittel wird abfiltiriert und die Essigesterphase eingedampft. Man erhält nach säulenchromatographischer Trennung über Kieselgel mit Essigester als Laufmittel 1,5 g (14 % d.Th.) 2-Amino-4-cyclopropyl-6-(1-(4-chlor-3,5-dimethylphenoxy)-propyl-2-amino)-1,3,5-triazin.

Die in der Tabelle 1 beschriebenen Verbindungen erhält man analog zu den vorstehenden Beispielen 1, 19 und 29. In der Tabelle bedeuten:

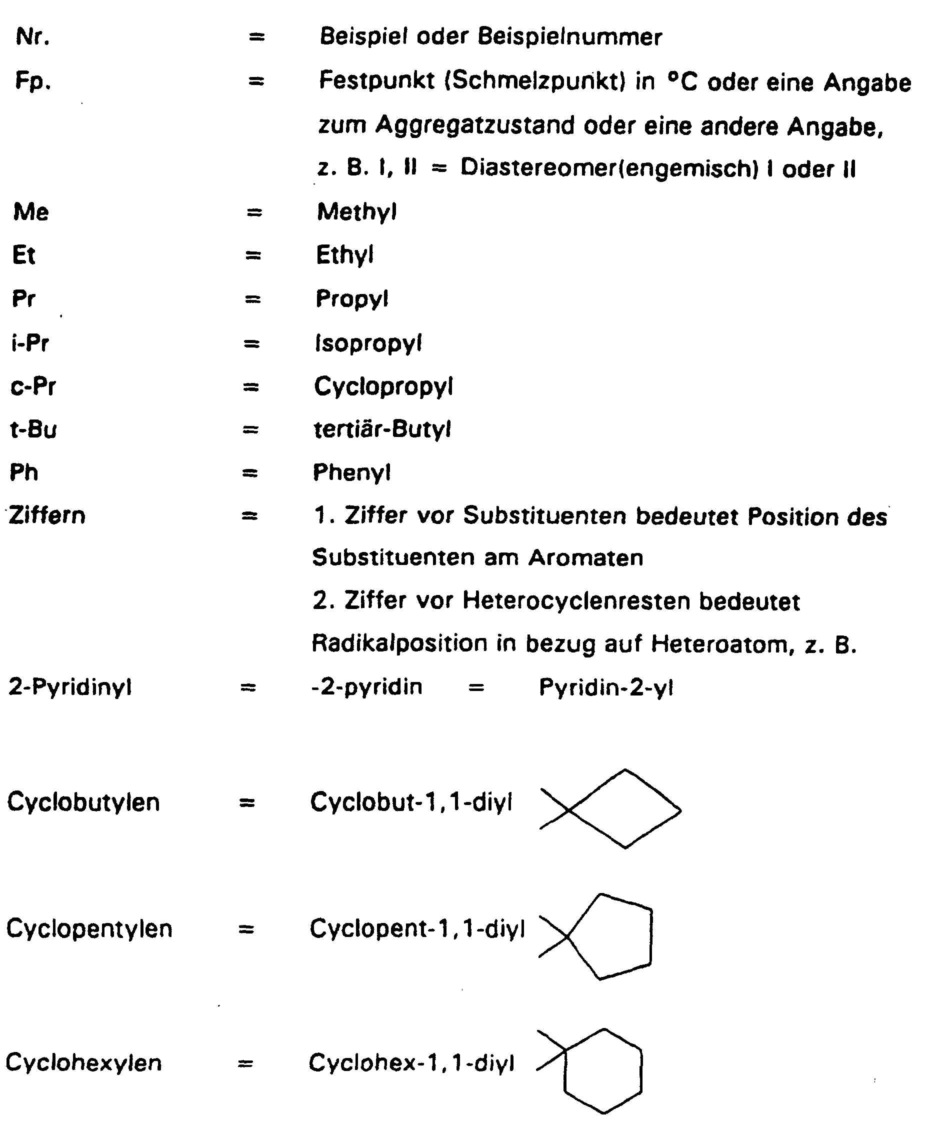

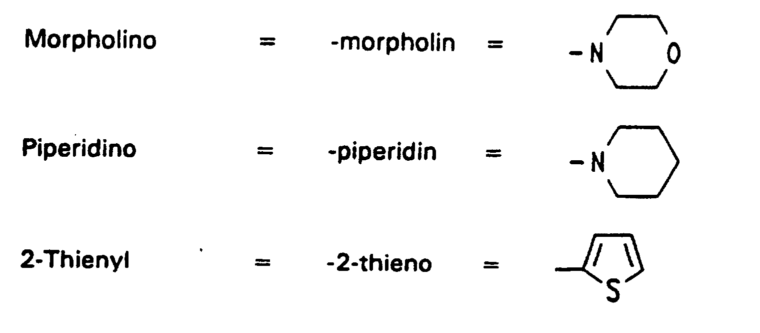

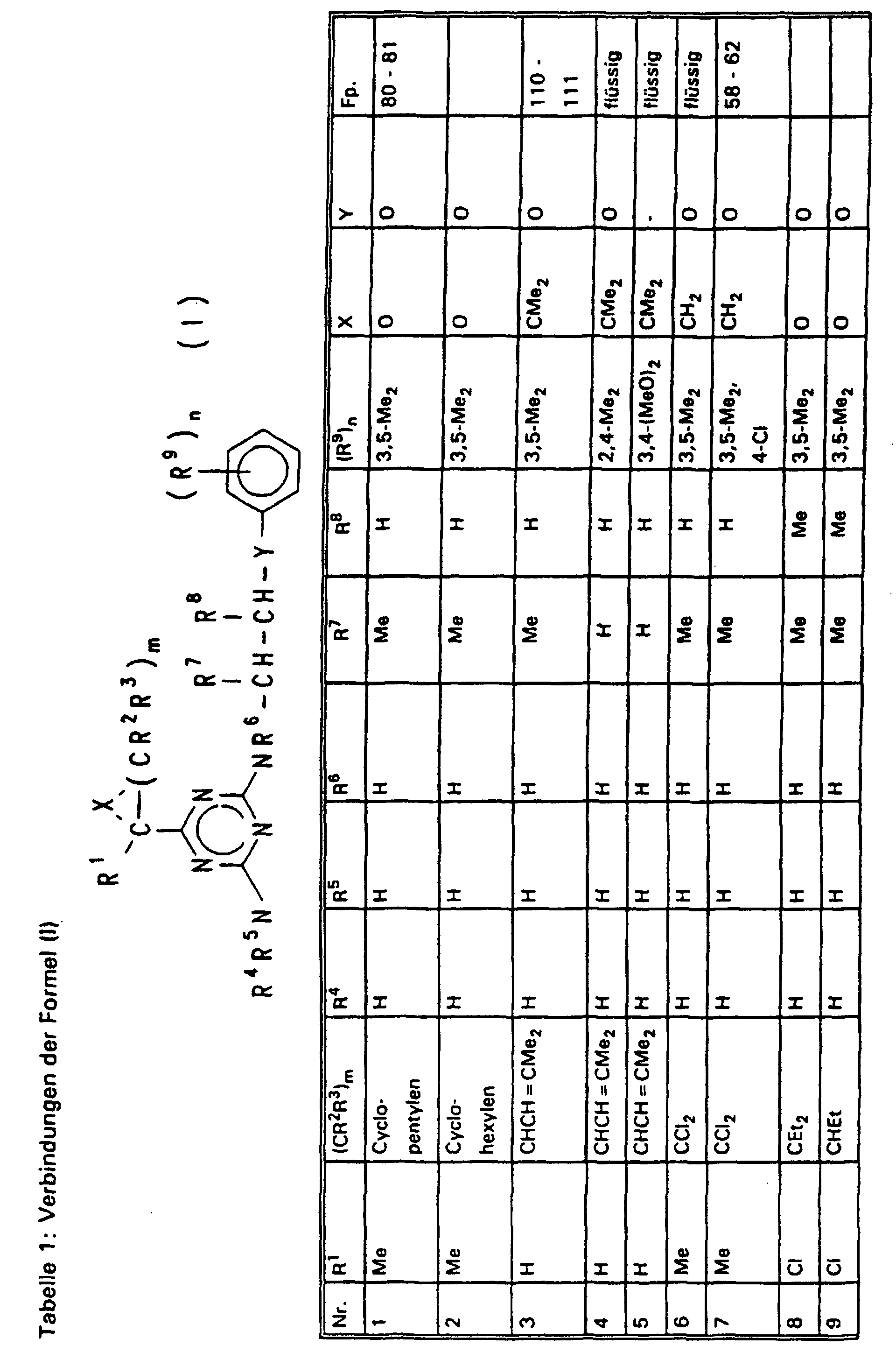

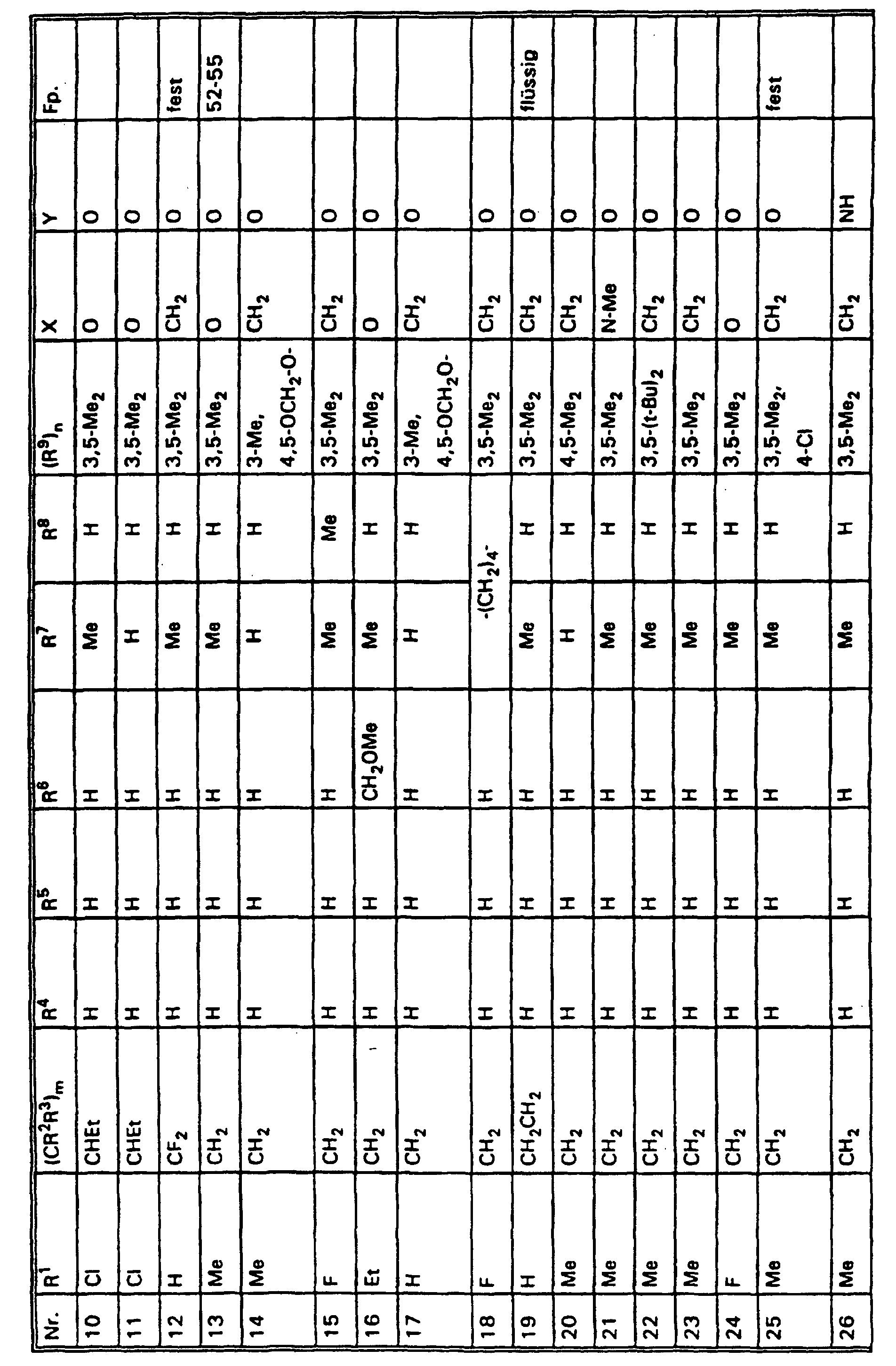

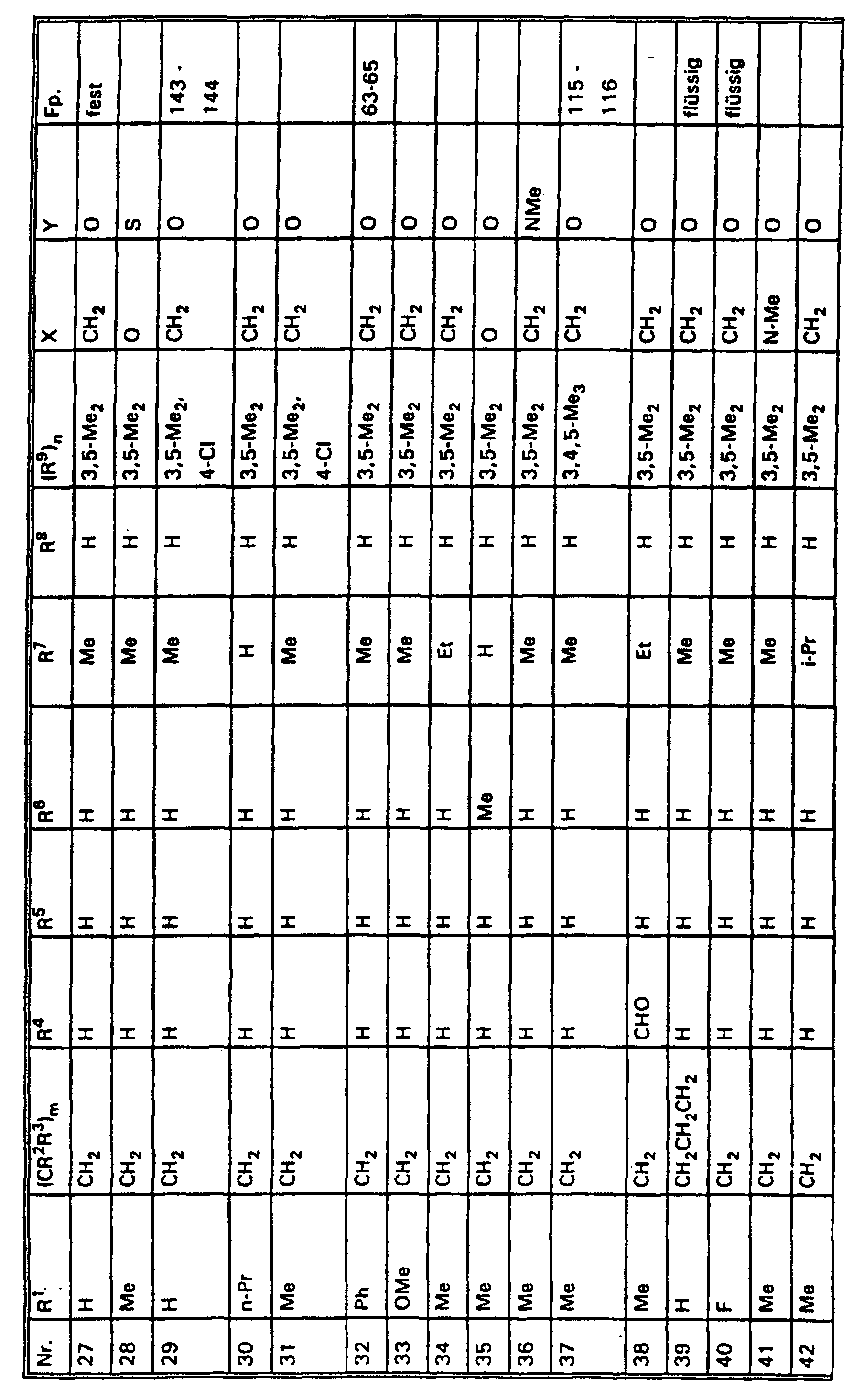

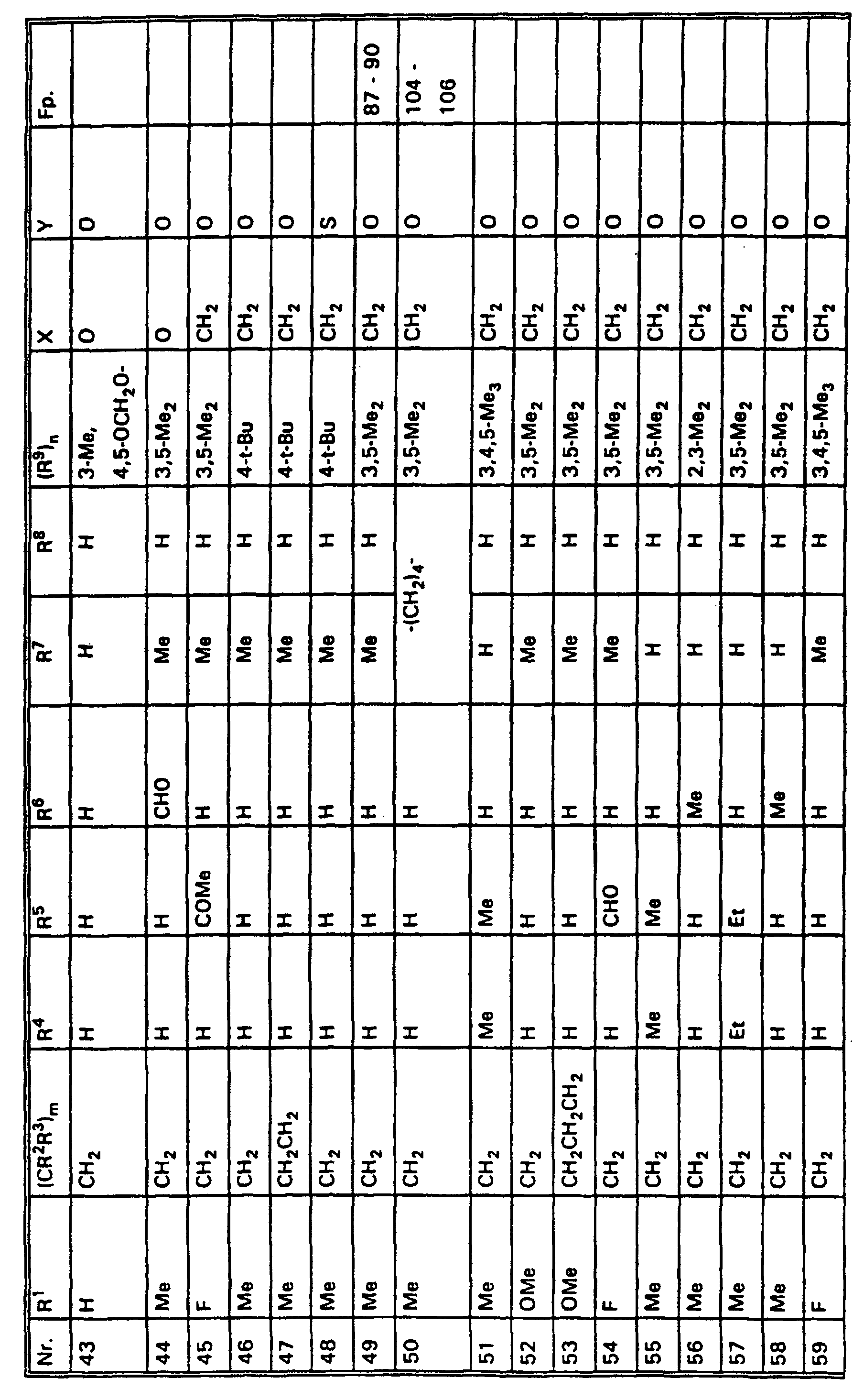

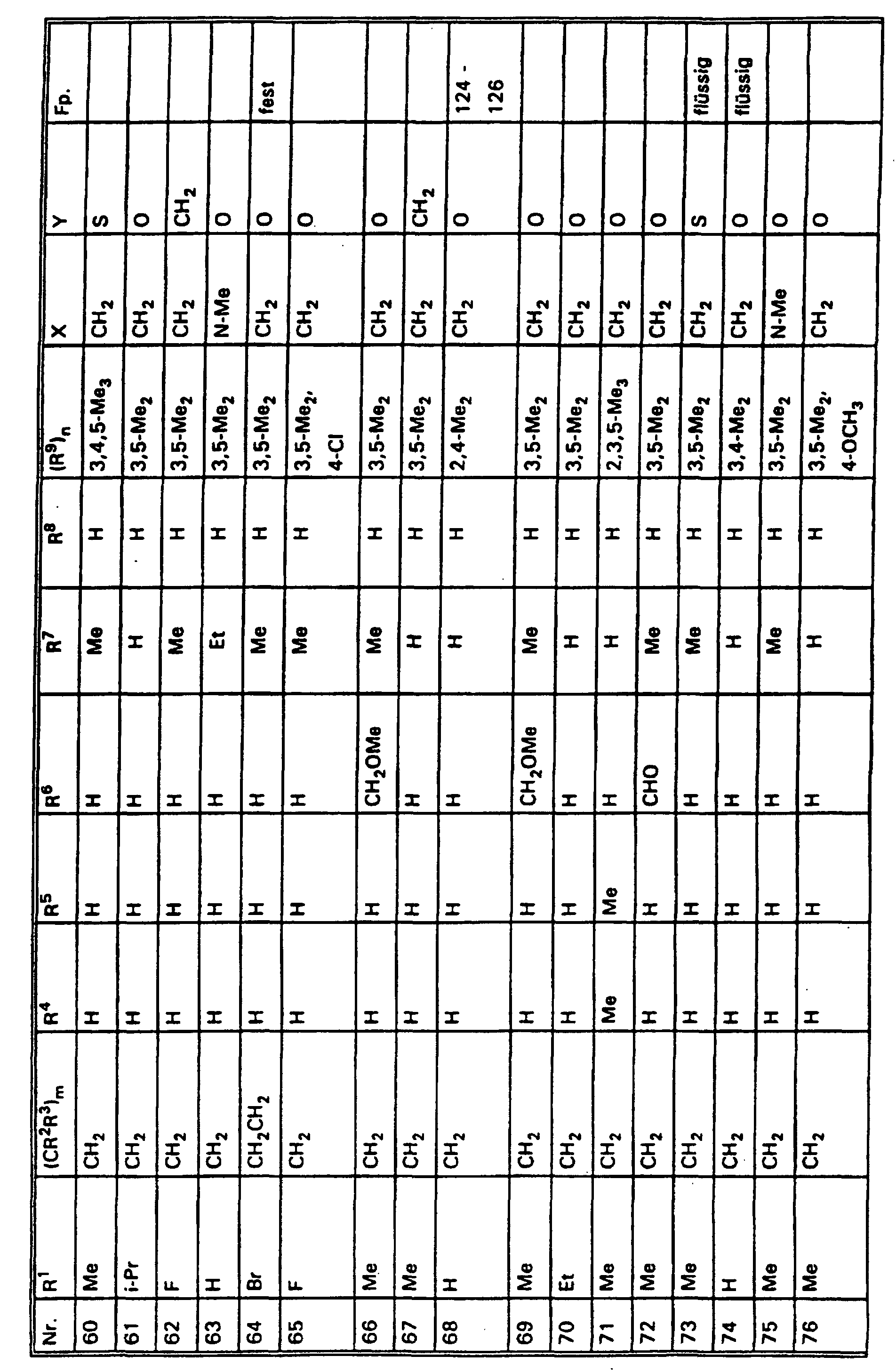

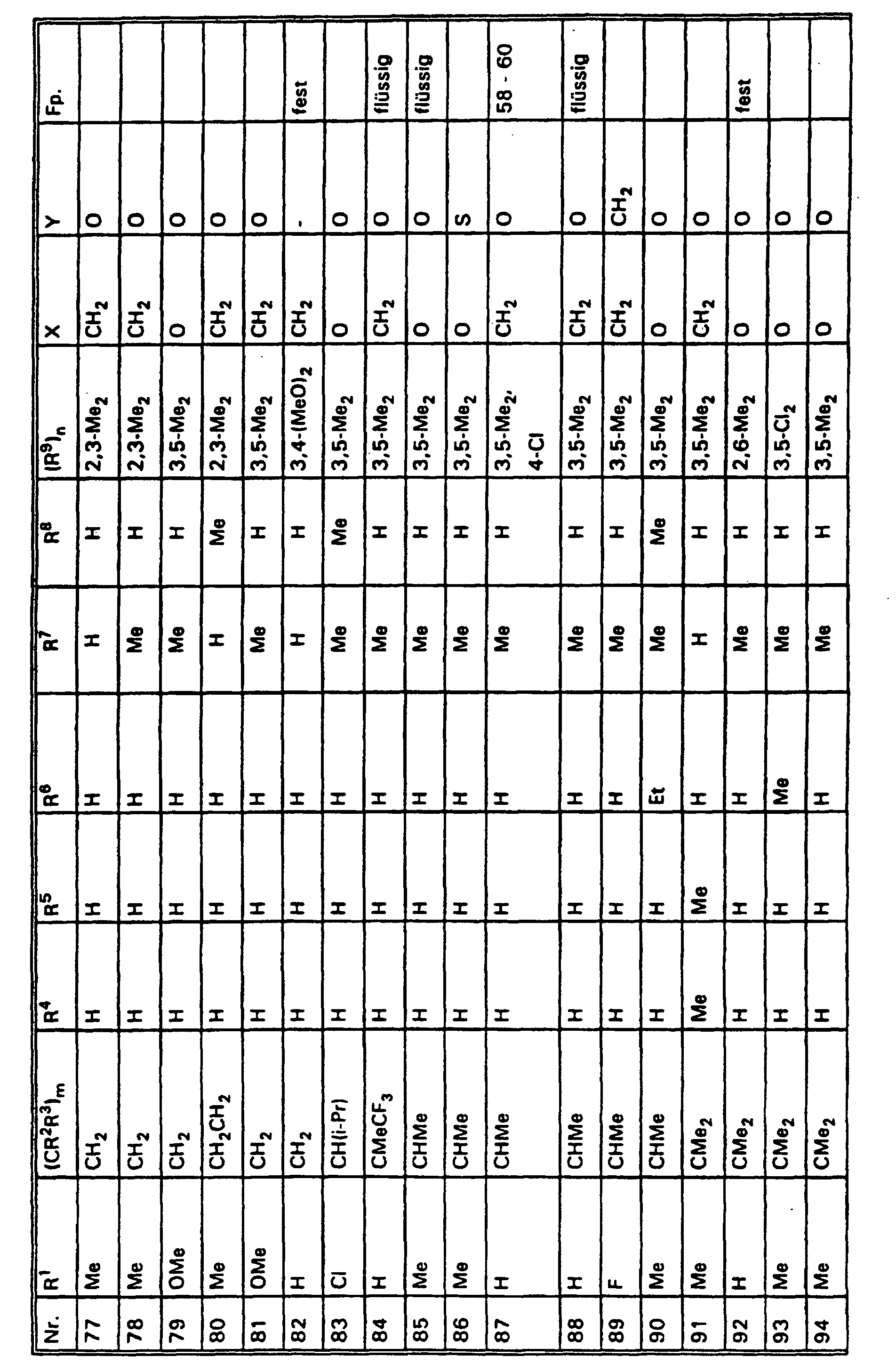

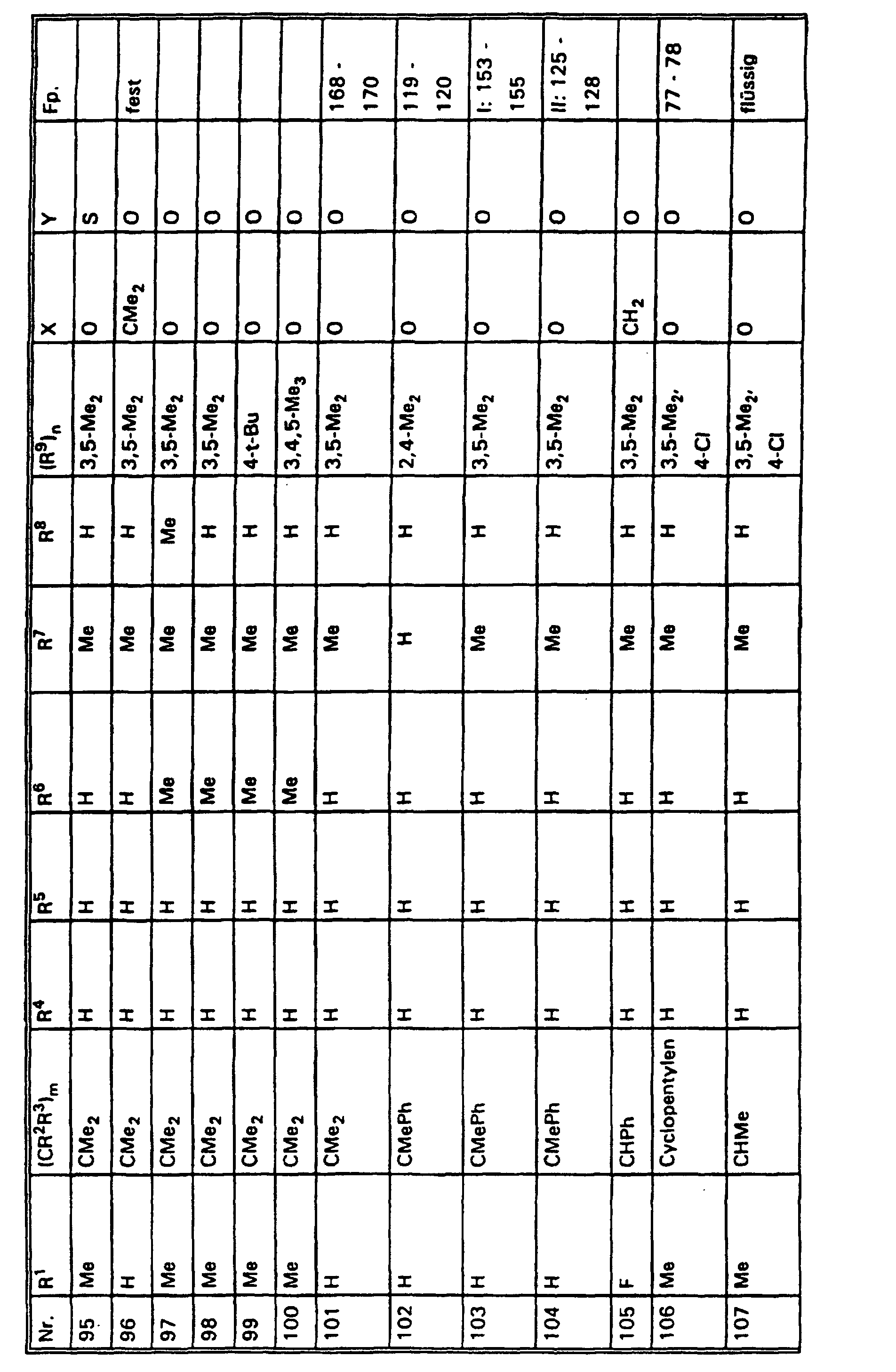

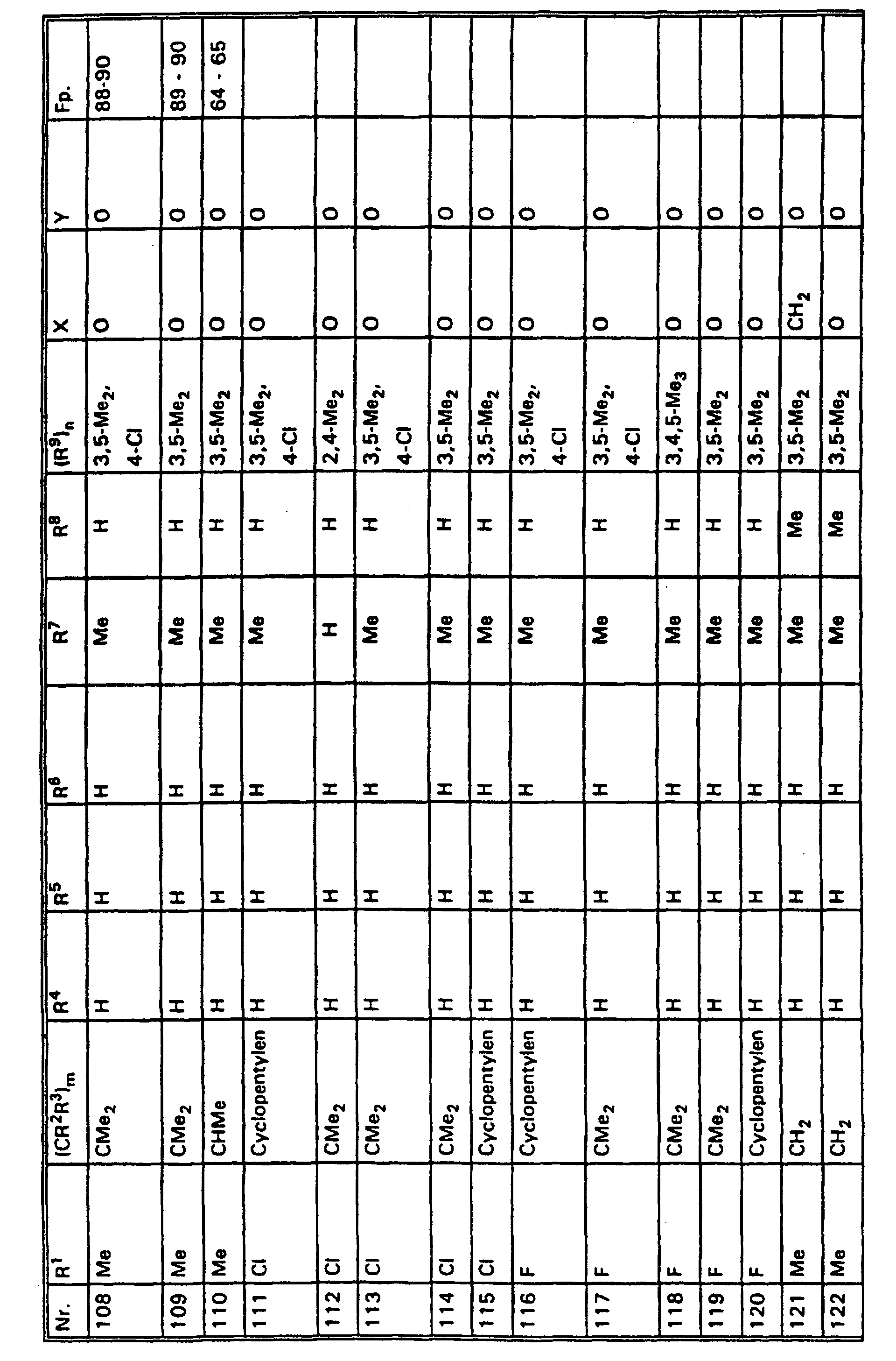

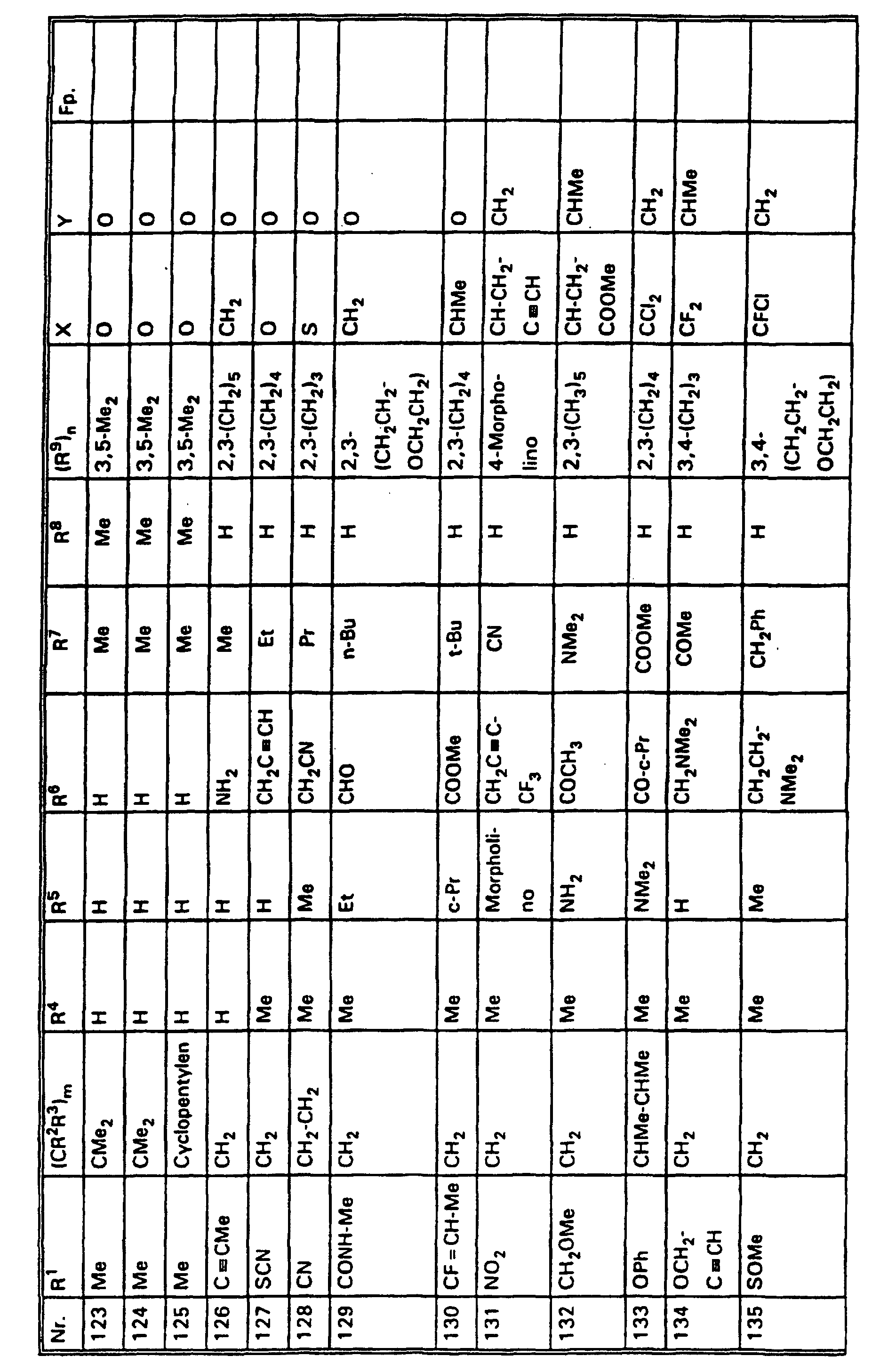

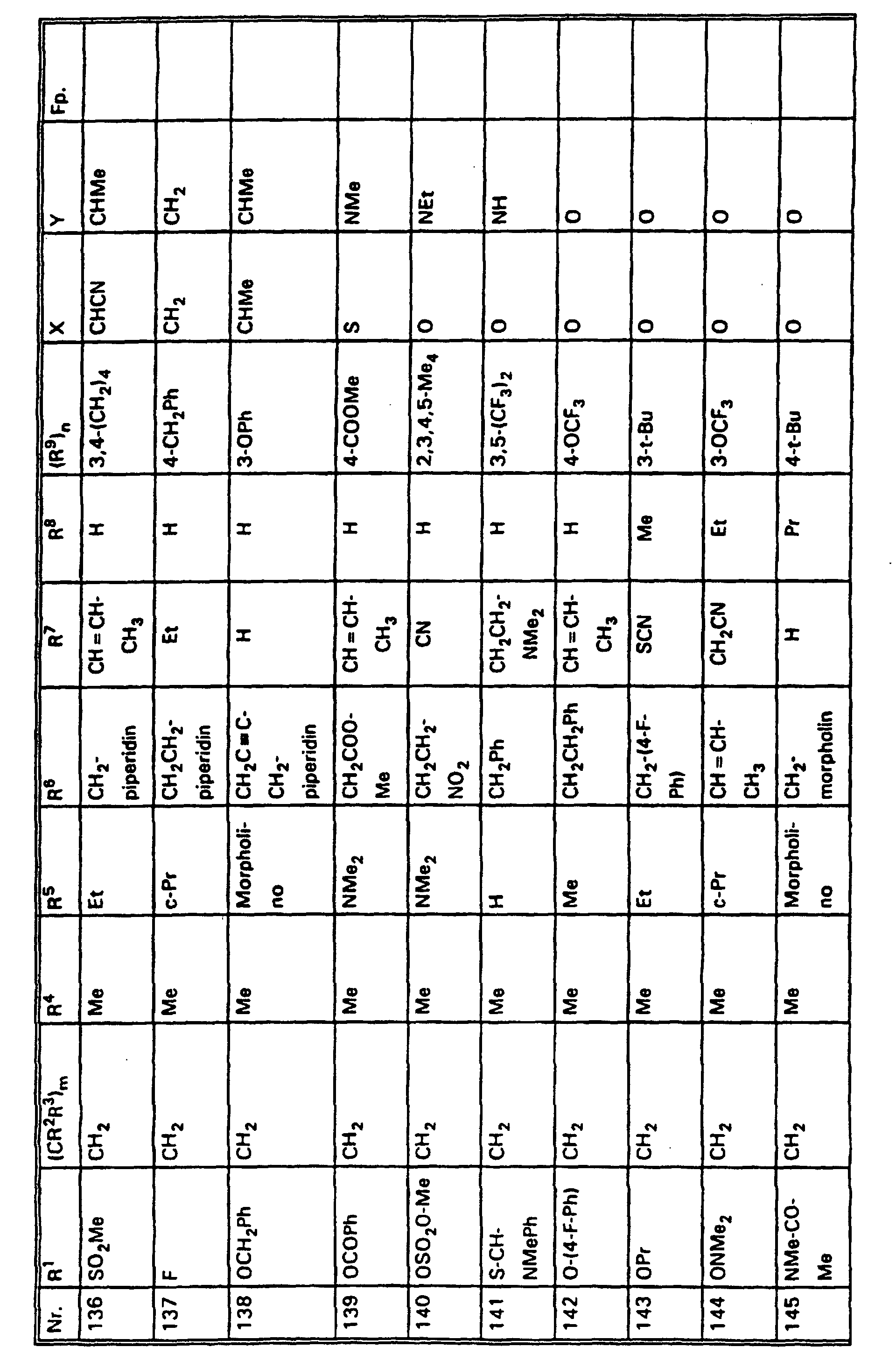

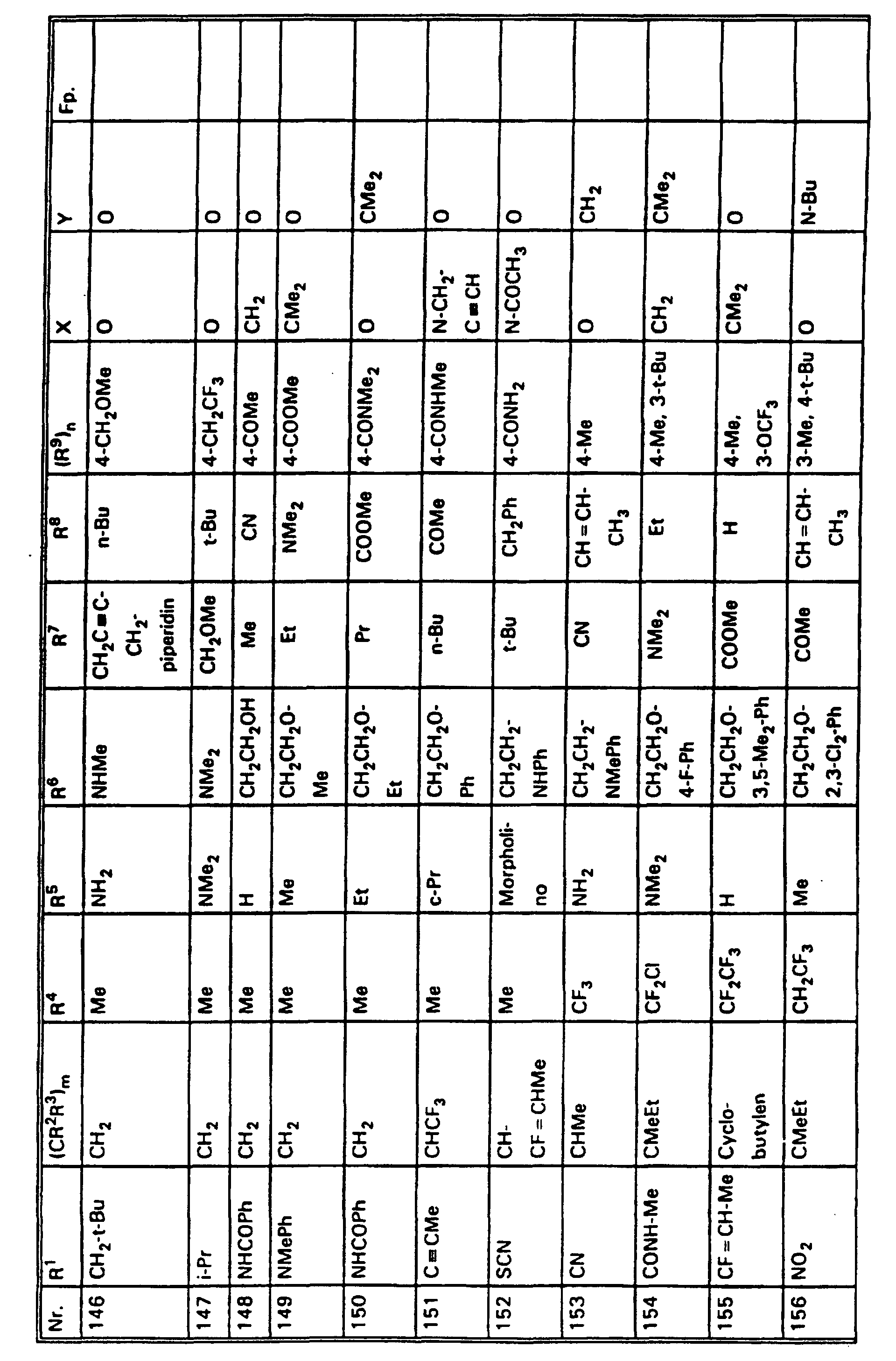

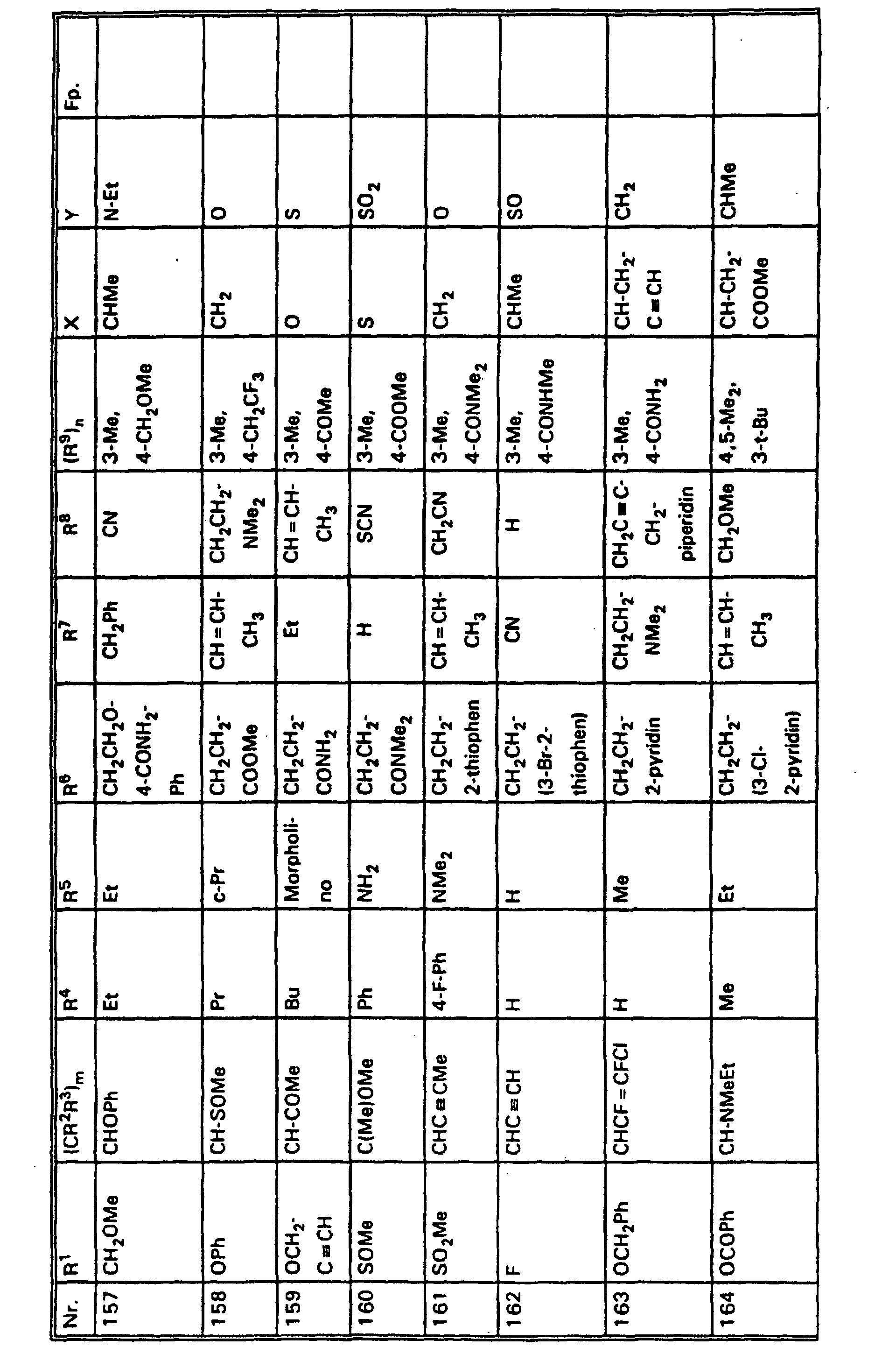

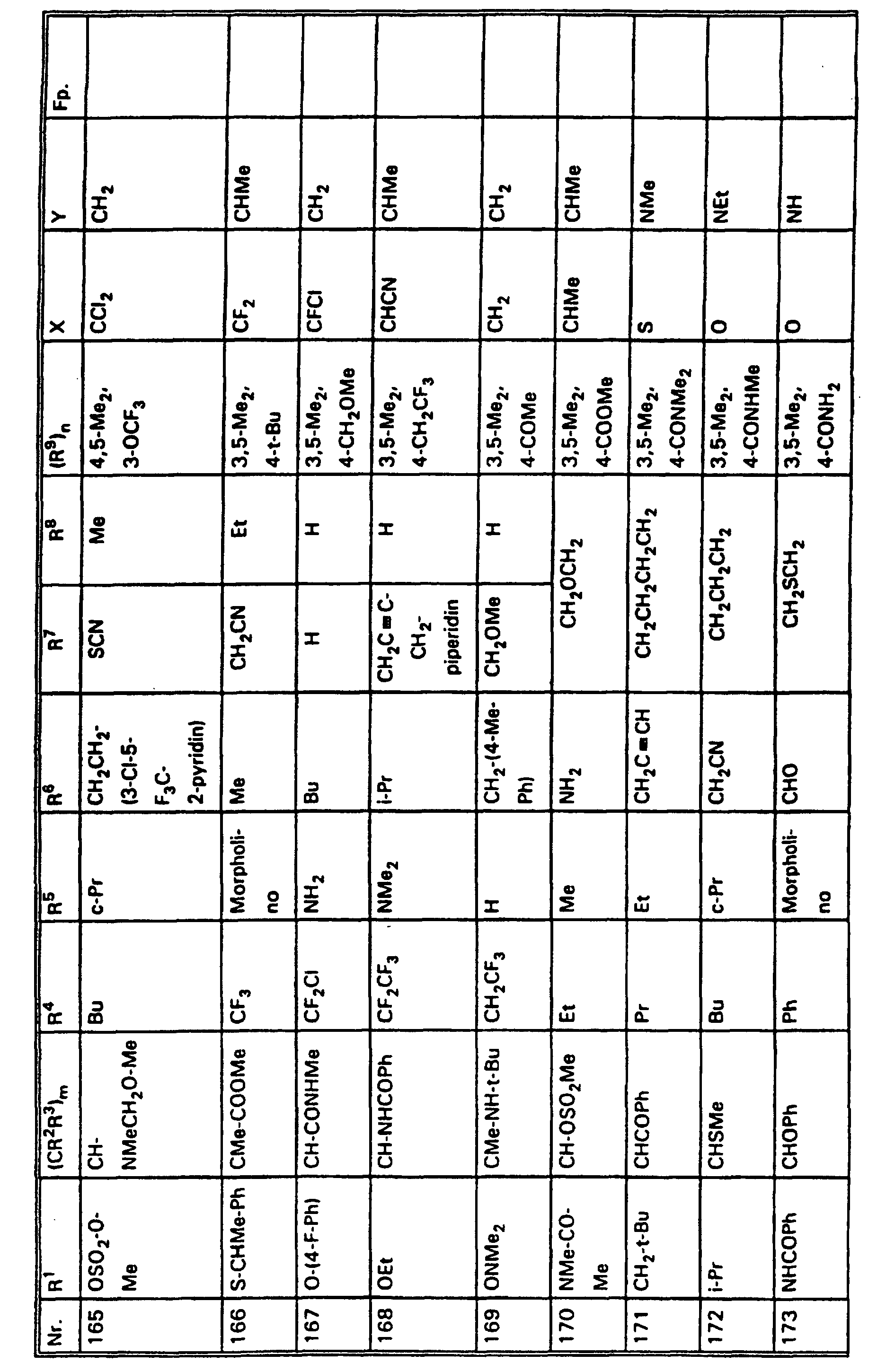

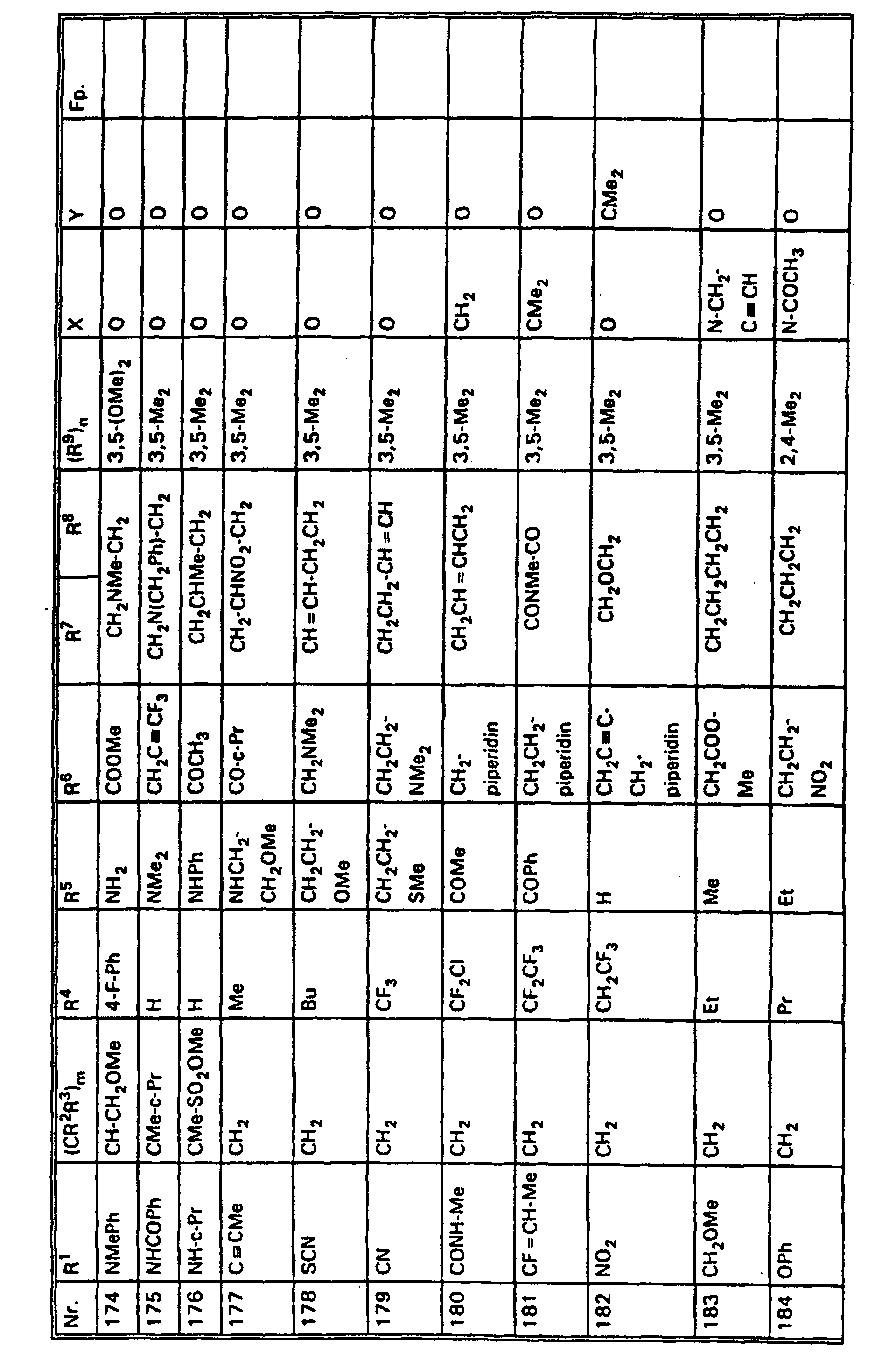

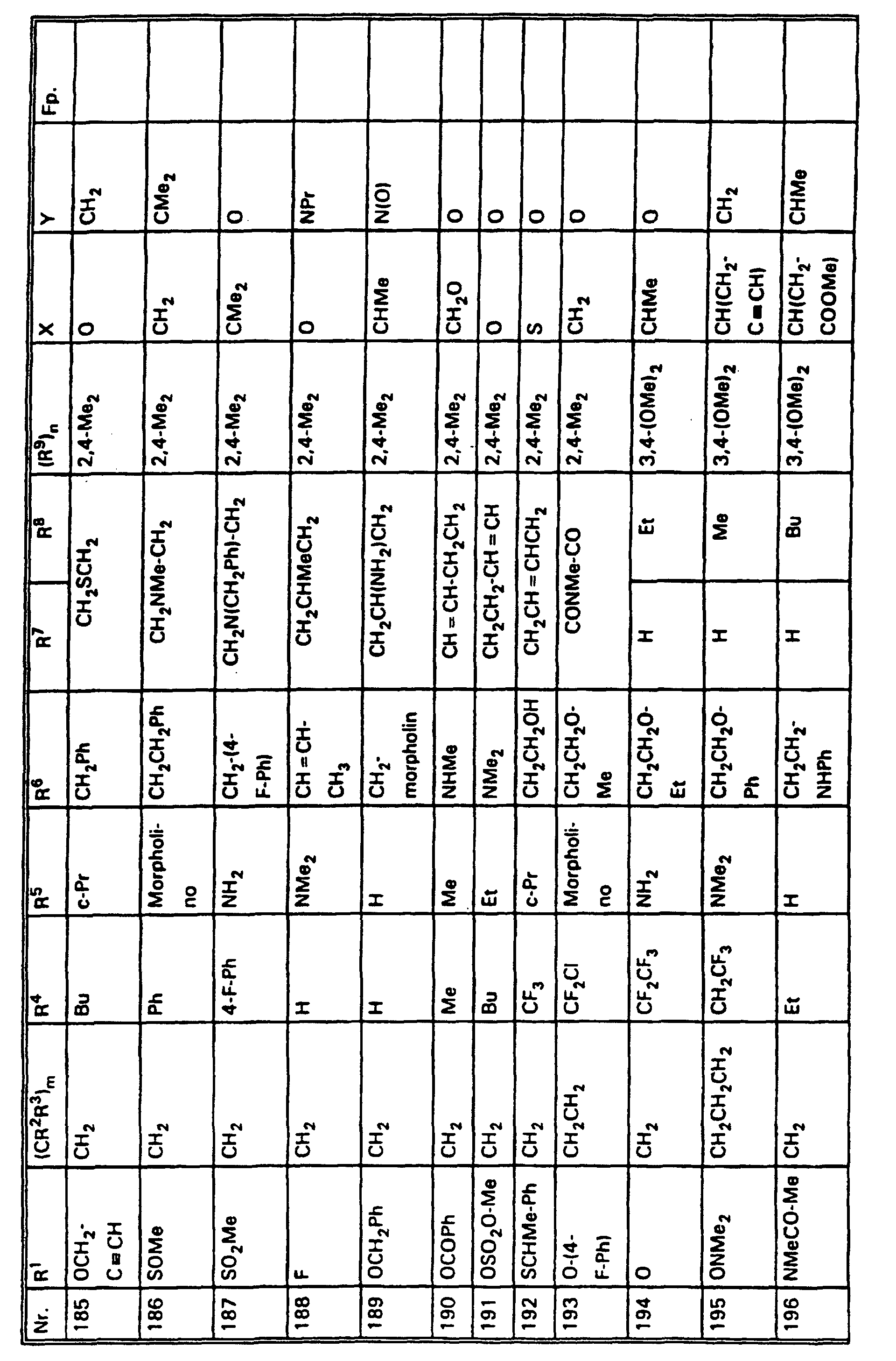

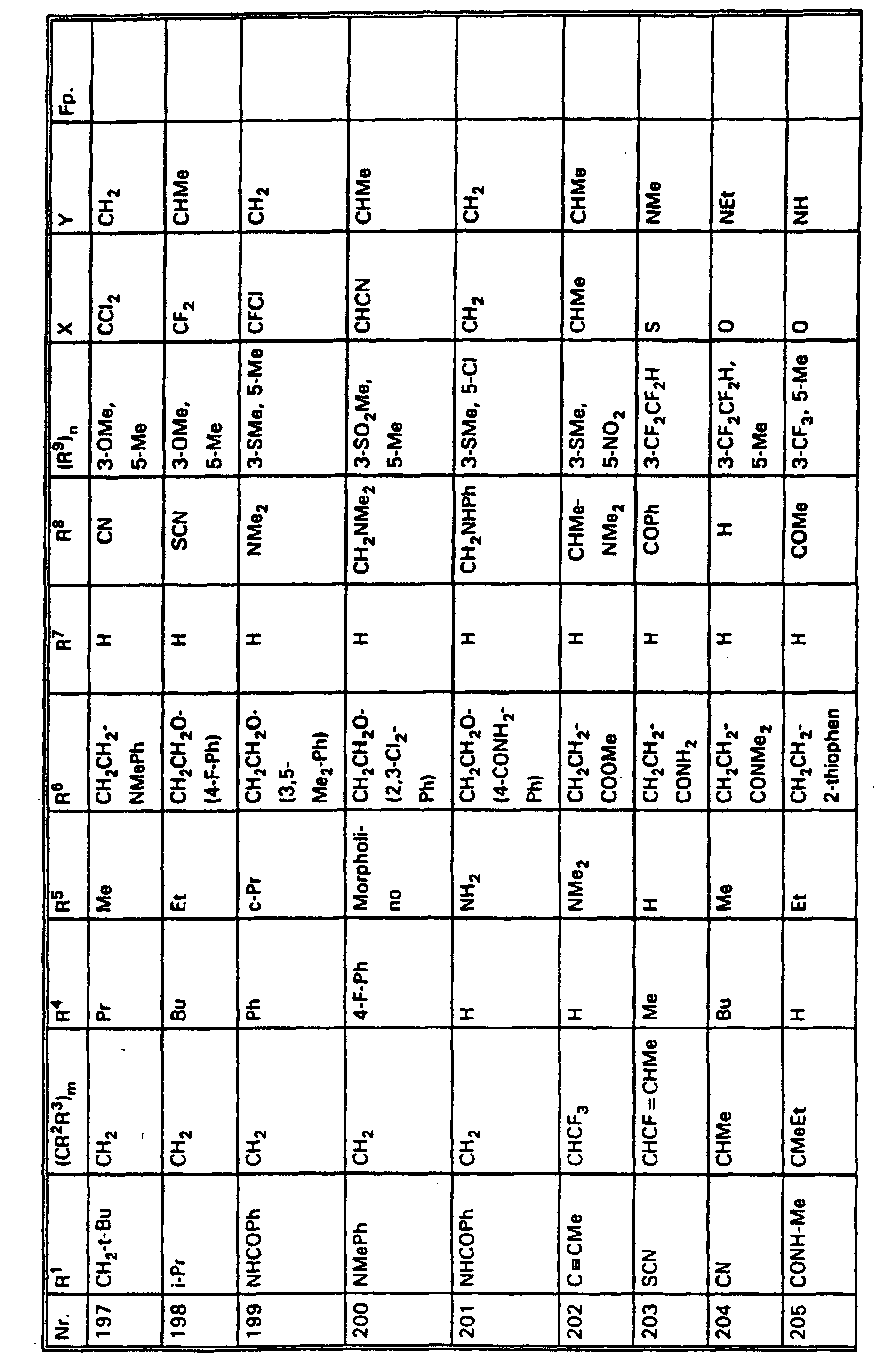

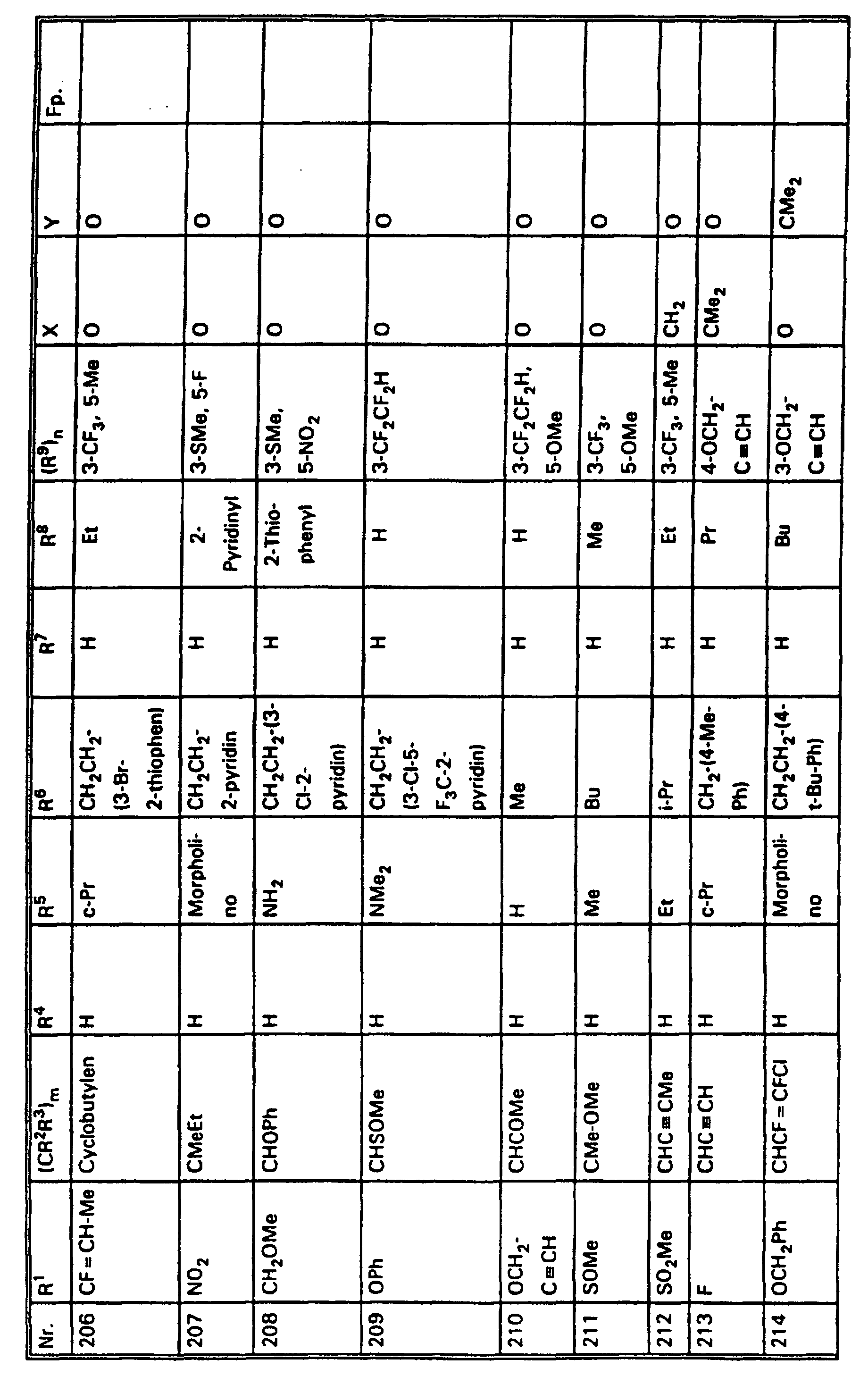

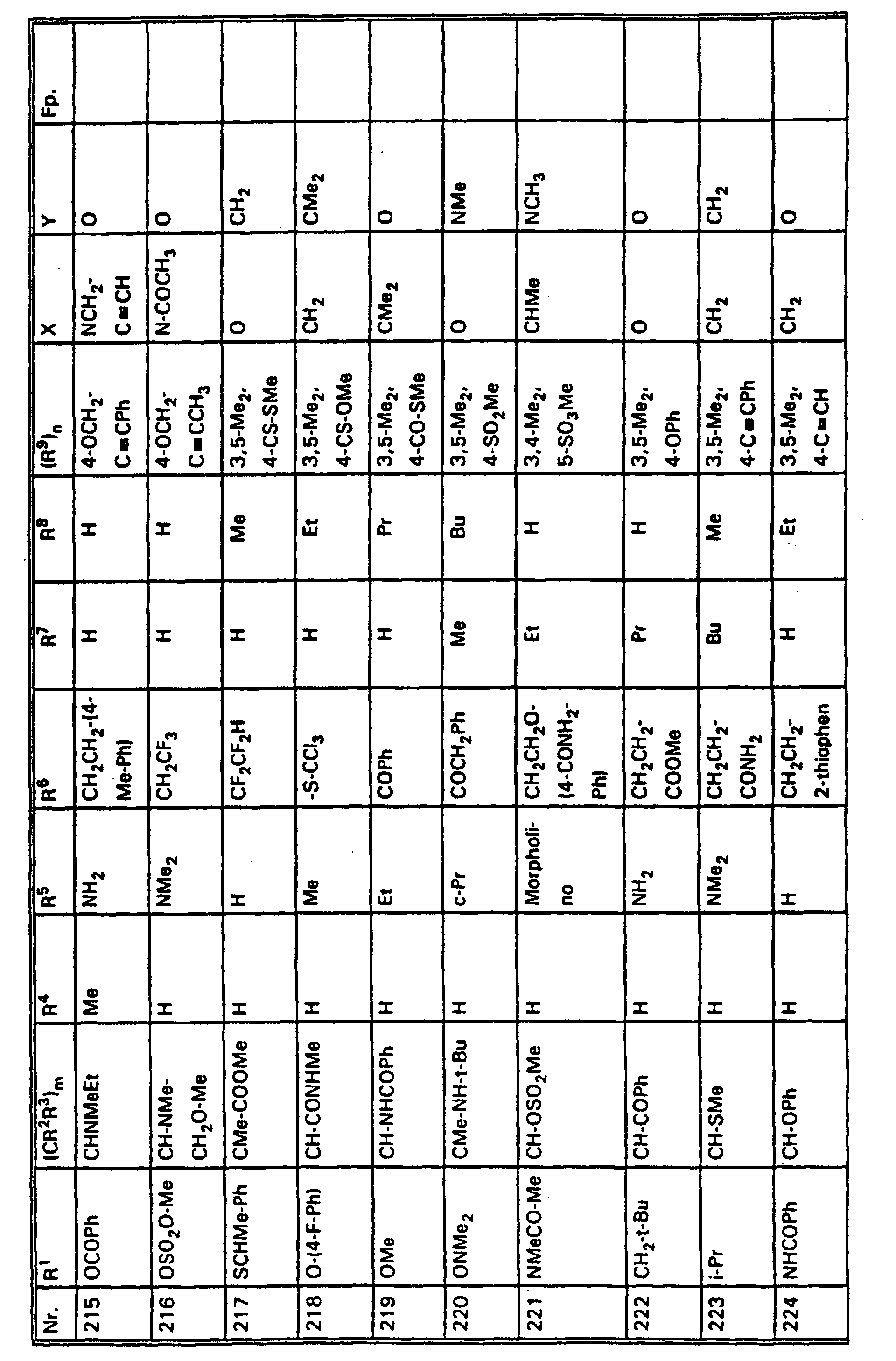

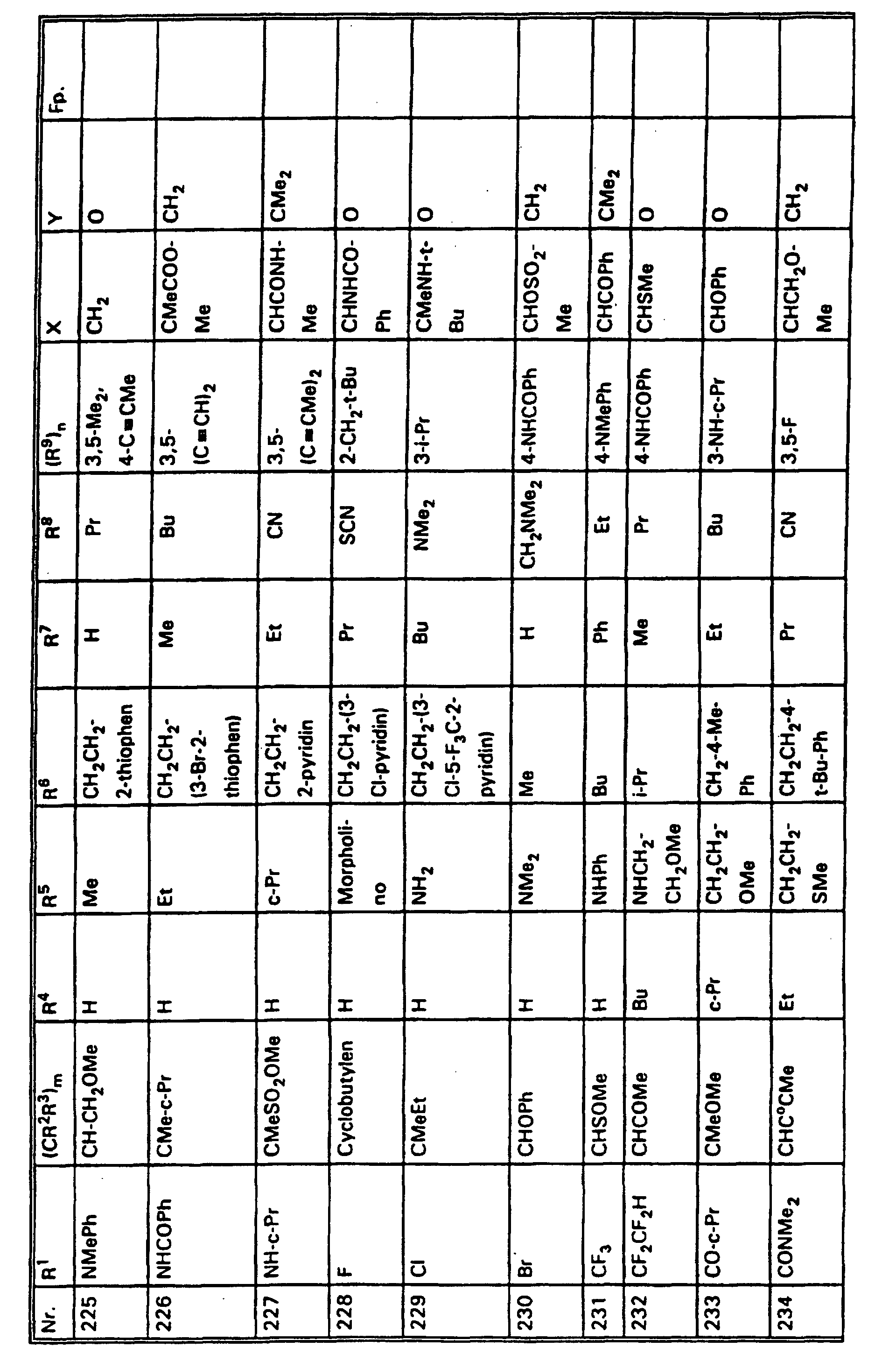

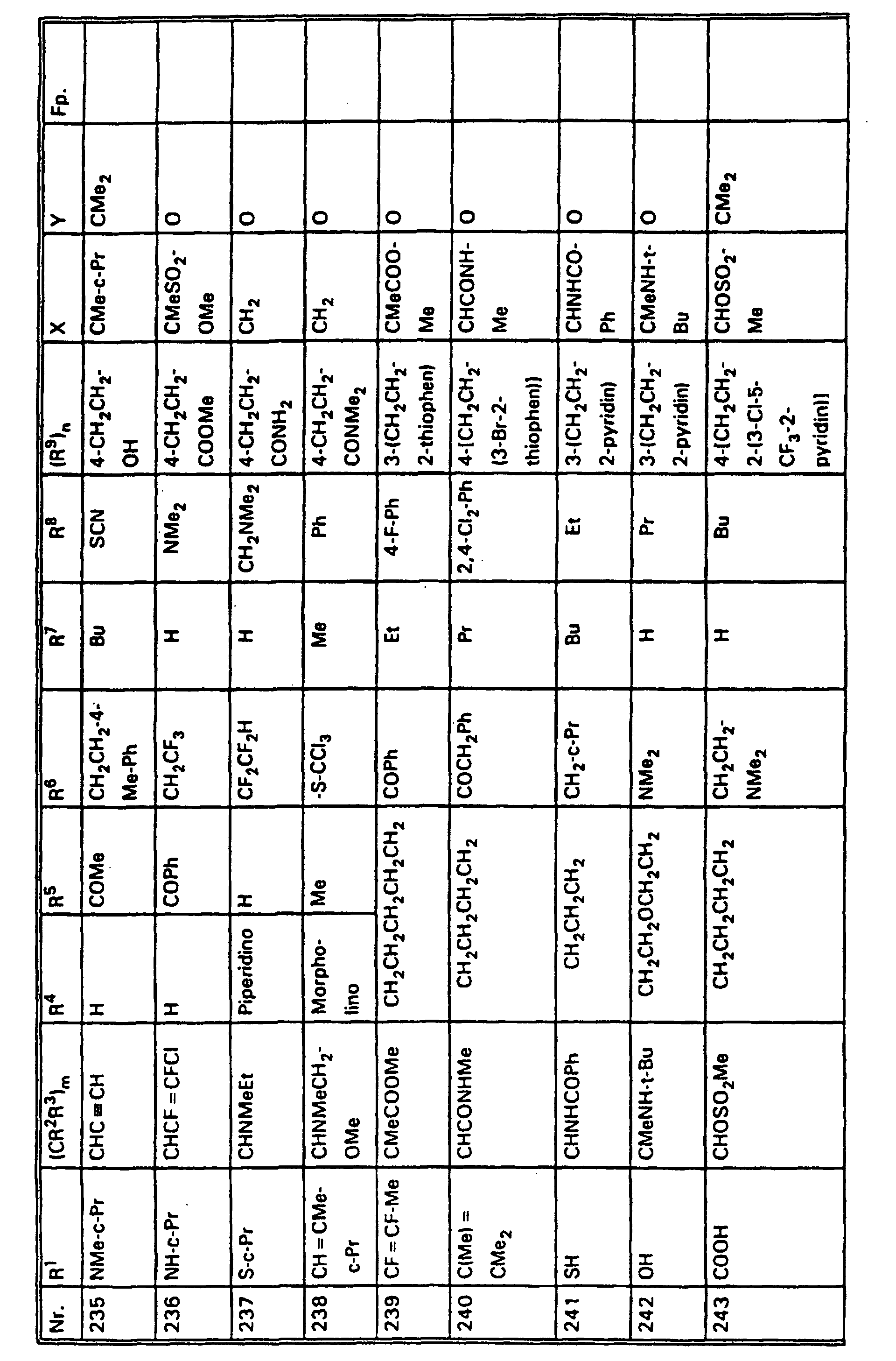

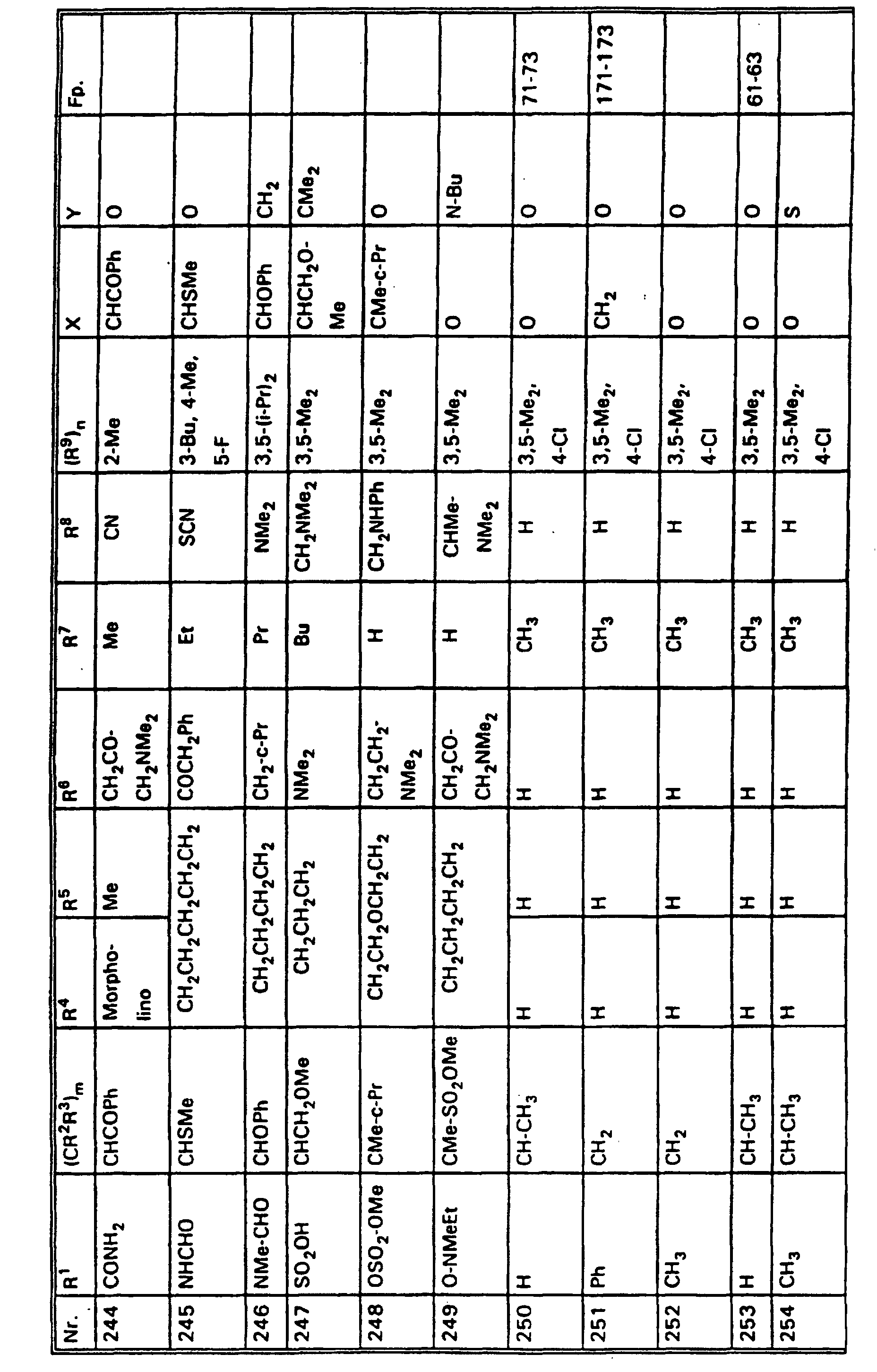

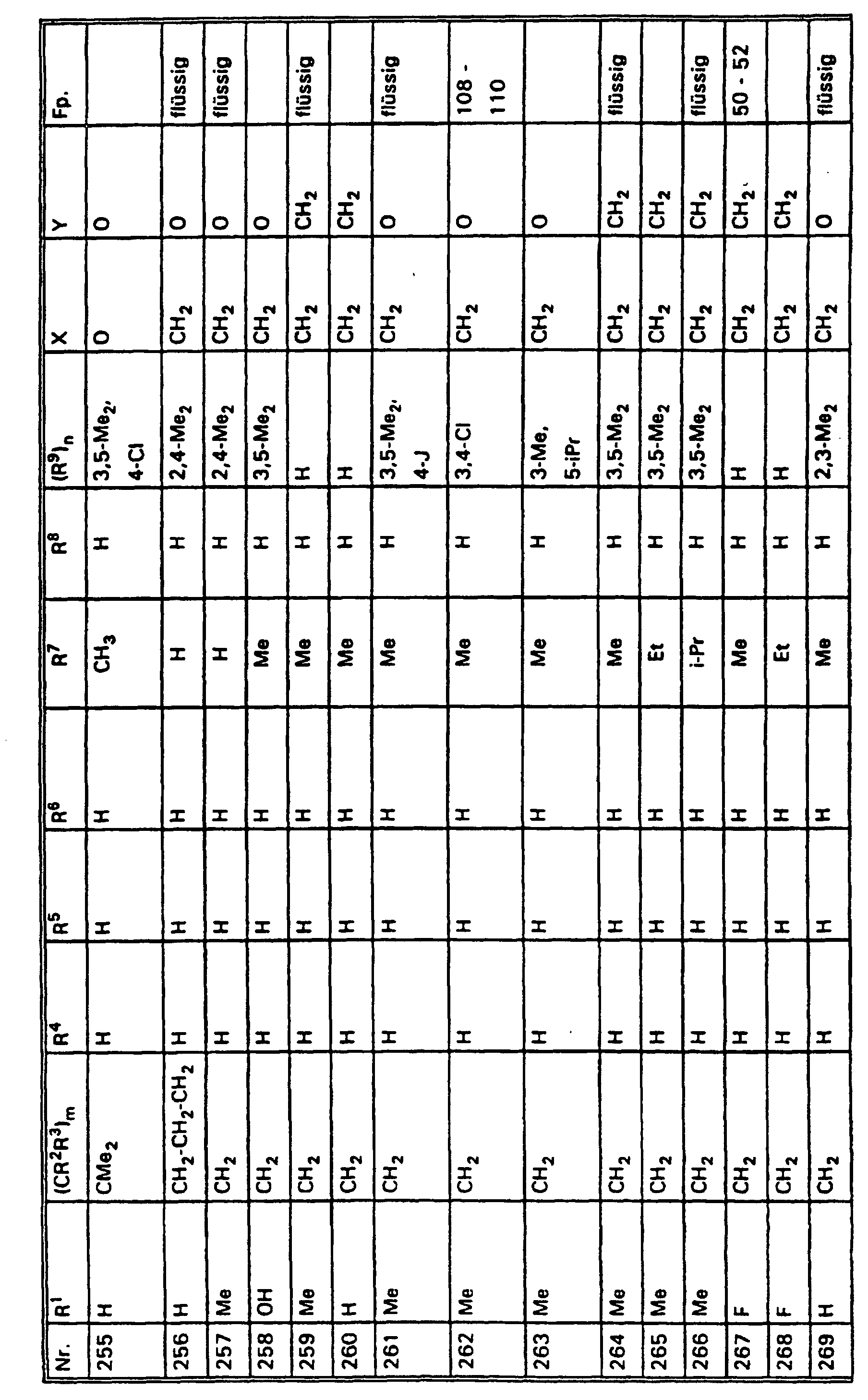

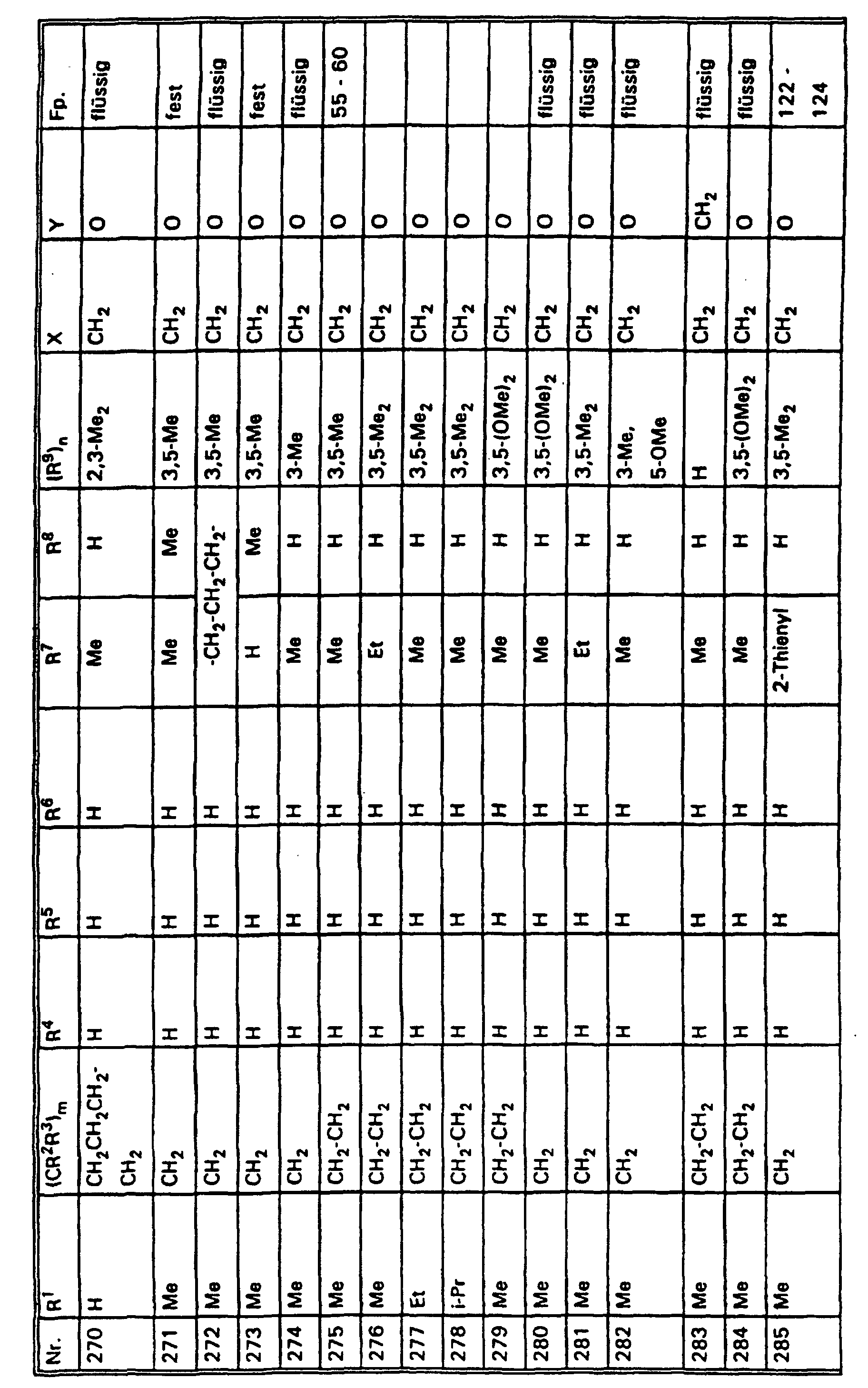

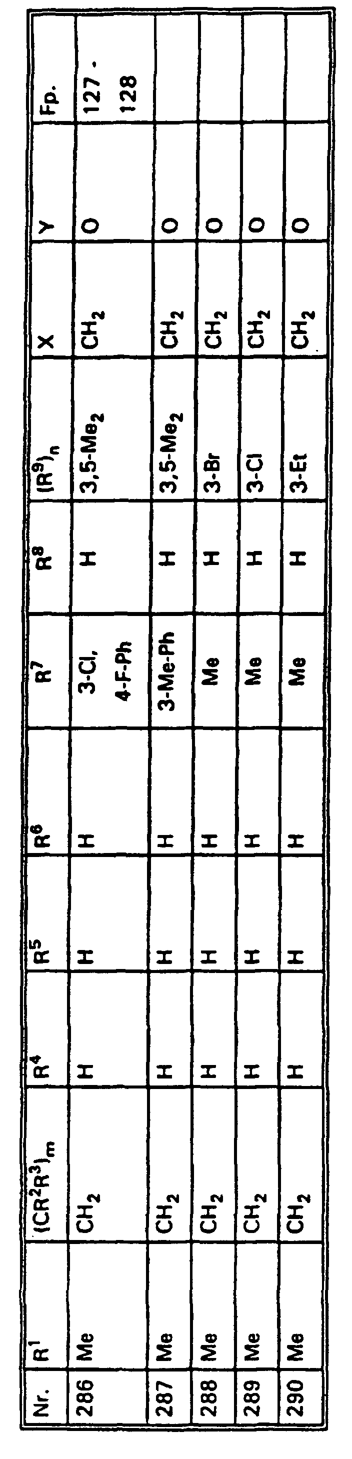

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | |
|---|---|
| 25 Gewichtsteile | einer Verbindung der Formel (I), |
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise zeigen die Beispiele Nr. 1, 3, 4, 5, 6, 7, 12, 13, 19, 25, 27, 29, 32, 37, 39, 40, 49, 50, 64, 68, 73, 74, 82, 84, 85, 88, 92, 96, 101, 102, 103, 104, 106, 107, 108, 109, 110, 250, 251, 253, 256, 257, 259, 261, 262, 264, 266, 267, 269, 270, 271, 272, 273, 274, 275, 280, 281, 282, 283, 284, 285 und 286 (s. Tabelle 1) im Test sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retrofluxus und Panicum miliaceum im Vorauflaufverfahren bei einer Aufwandmenge von 0,5 kg und weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen die Beispiele Nr. 1, 3, 4, 5, 6, 7, 12, 13, 19, 25, 27, 29, 32, 37, 39, 40, 49, 50, 64, 68, 73, 74, 82, 84, 85, 88, 92, 96, 101, 102, 103, 104, 106, 107, 108, 109, 110, 250, 251, 253, 256, 257, 259, 261, 264, 266, 267, 269, 270, 271, 272, 273, 274, 275, 280, 281, 282, 283, 284, 285 und 286 (s. Tabelle 1) im Test sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus, Panicum miliaceum und Avena sativa im Nachauflaufverfahren bei einer Aufwandmenge von 0,5 kg und weniger Aktivsubstanz pro Hektar.

### 3. Wirkung auf Schadpflanzen in Reis

Verpflanzter und gesäter Reis sowie typische Reisunkräuter werden im Gewächshaus bis zum Dreiblattstadium (Echinochloa 1,5-Blatt) unter Paddyreis-Bedingungen (Anstauhöhe des Wassers: 2 - 3 cm) in geschlossenen Plastiktöpfen angezogen. Danach erfolgt die Behandlung mit den erfindungsgemäßen Verbindungen. Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht. Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen, wobei beispielsweise die Verbindungen Nr. 1, 3, 4, 5, 6, 7, 12, 13, 19, 25, 27, 29, 32, 37, 39, 40, 49, 50, 64, 68, 73, 74, 82, 84, 85, 88, 92, 96, 101, 102, 103, 104, 106, 107, 108, 109, 110, 250, 251, 253, 256, 257, 259, 261, 262, 264, 266, 267, 269, 270, 271, 272, 273, 274, 275, 280, 281, 282, 283, 284, 285 und 286 (s. Tab. 1) sehr gute herbizide Wirkung gegen Schadpflanzen zeigen, die typisch für Reiskulturen sind, wie z.B. Cyperus monti, Echinochloa crus-galli, Eleocharis acicularis und Sagittaria pygmaea.

### 4. Kulturpflanzenverträglichkeit

In weiteren Versuchen im Gewächshaus werden Samen einer größeren Anzahl *von* Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum-Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 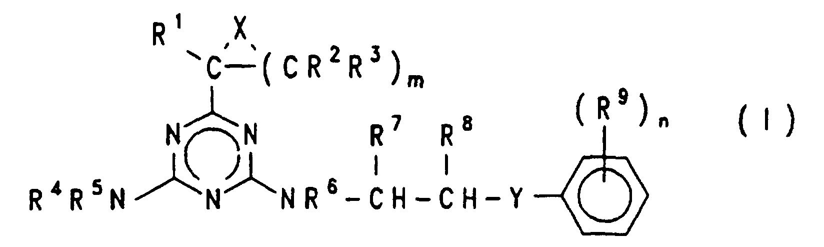 worin
R¹ Wasserstoff, Halogen, Nitro, Cyano, Thiocyanato oder einen Rest der Formel -Z¹-R¹⁰,
R² und R³ jeweils unabhängig voneinander Wasserstoff, Halogen, Nitro, Cyano, Thiocyanato oder einen Rest der Formel -Z²-R¹¹ oder
R² und R³ gemeinsam mit dem C-Atom der Gruppe CR²R³ einen carbocyclischen Rest mit 4 bis 10 Ringgliedern oder einen heterocyclischen Rest mit 4 bis 10 Ringgliedern und mit Heteroringatomen aus der Gruppe N, O und S, wobei jeder der letztgenannten zwei Reste unsubstituiert oder durch einen oder mehrere Reste aus der Gruppe Halogen, (C₁-C₄)Alkyl und Oxo substituiert ist,
R⁴ und R⁵ jeweils unabhängig voneinander Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest oder
R⁴ und R⁵ gemeinsam mit dem Stickstoffatom der Gruppe NR⁴R⁵ einen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder substituiert ist,
R⁶ Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest,
R⁷ und R⁸ jeweils unabhängig voneinander Wasserstoff, Halogen, Nitro, Cyano, Thiocyanato oder einen Rest der Formel -Z³-R¹² oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
R⁹ jeweils unabhängig voneinander Halogen, Nitro, Formyl, Carboxy, Cyano, Thiocyanato, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, (C₁-C₄)Alkylcarbonyl, (C₁-C₄)Alkyloxycarbonyl, (C₁-C₄)Alkoxy-thiocarbonyl, (C₁-C₄)Alkylthiocarbonyl oder (C₁-C₄)Alkylthio-thiocarbonyl, wobei die Alkylreste in den letztgenannten 11 Resten unsubstituiert oder substituiert sind, oder (C₁-C₄)Alkylsulfonyl, (C₁-C₄)Haloalkylsulfonyl, (C₁-C₄)Alkoxysulfonyl, Amino, Mono- oder Di-[(C₁-C₆)alkyl]amino, Aminocarbonyl, Mono- oder Di-[(C₁-C₆)alkyl]aminocarbonyl, (C₁-C₆)Alkanoylamino, N-(C₁-C₆)Alkanoyl-N-(C₁-C₄)alkyl-amino, oder einen Rest der Formel Z⁴-R^{o}, wobei Z⁴ wie unten definiert und R° einen aromatischen, gesättigten oder teilgesättigten carbocyclischen oder heterocyclischen Rest, wobei der cyclische Rest substituiert oder unsubstituiert ist, oder zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
R¹⁰, R¹¹, R¹² jeweils unabhängig voneinander H oder einen acyclischen Kohlenwasserstoffrest oder cyclischen Kohlenwasserstoffrest oder einen heterocyclischen Rest, wobei jeder der drei letztgenannten Reste unsubstituiert oder substituiert ist,
X eine Gruppe der Formel -O-, -S(O)_{q}-, -NR*- oder -N(O)-, wobei q = 0, 1 oder 2 ist und R* Wasserstoff oder Alkyl mit 1 bis 4 C-Atomen ist, oder eine Gruppe der Formel CR¹³R¹⁴, wobei die Definitionen für R¹³ und R¹⁴ aus den für R² und R³ möglichen Resten ausgewählt sind,
Y eine direkte Bindung oder eine Gruppe der Formel -O-, -S(O)ᵣ-, -NR**- oder -N(O)-, wobei r = 0, 1 oder 2 ist und R** Wasserstoff oder Alkyl mit 1 bis 4 C-Atomen ist, oder eine Gruppe der Formel -CH₂-, -C(CH₃)H- oder -C(CH₃)₂-,
Z¹, Z², Z³, Z⁴ jeweils unabhängig voneinander eine direkte Bindung oder eine divalente Gruppe der Formel
-O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'- bedeutet und dabei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
m 1, 2, 3 oder 4 und
n 0, 1, 2, 3, 4 oder 5,
bedeuten.

2. Verbindungen der Formel (I) und deren Salze nach Anspruch 1, **dadurch gekennzeichnet, daß**
R¹ H, Halogen, NO₂, CN, SCN oder -Z¹-R¹⁰,
R² und R³ jeweils unabhängig voneinander H, Halogen, NO₂, CN, SCN oder einen Rest der Formel -Z²-R¹¹ oder
R² und R³ gemeinsam mit dem C-Atom der Gruppe CR²R³ (C₃-C₈)Cycloalkyl, (C₃-C₈)Cycloalkenyl oder einen heterocyclischen Rest mit 3 bis 6 Ringgliedern und mit Heteroringatomen aus der Gruppe N, O und S, wobei jeder der letztgenannten 3 Reste unsubstituiert oder durch einen oder mehrere Reste aus der Gruppe Halogen, (C₁-C₄)Alkyl und Oxo substituiert ist,
R⁴ und R⁵ jeweils unabhängig voneinander Wasserstoff, Amino, Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
R⁶ Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest,
R⁷ und R⁸ jeweils unabhängig voneinander H, Halogen, NO₂, CN, SCN oder einen Rest der Formel -Z³-R¹² oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
R⁹ jeweils unabhängig voneinander Halogen, Nitro, Formyl, Carboxy, Cyano, Thiocyanato, (C₁-C₆)Alkyl, (C₁-C₆)Alkoxy, (C₁-C₄)Alkylcarbonyl, (C₁-C₄)Alkyloxycarbonyl oder (C₁-C₄)Alkylthiocarbonyl, wobei die letztgenannten fünf Reste unsubstituiert oder durch Halogen oder (C₁-C₄)Alkoxy substituiert sind, oder (C₁-C₄)Alkylsulfonyl, (C₁-C₄)Haloalkylsulfonyl, Mono- oder Di-[(C₁-C₄)alkyl]amino, Aminocarbonyl, Mono- oder Di-[(C₁-C₄)alkyl]aminocarbonyl, (C₁-C₄)Alkanoylamino oder einen Rest der Formel Z⁴-R^{o}, worin Z⁴ wie unten definiert ist und R^{o} (C₃-C₆)Cycloalkyl, (C₃-C₆)Cycloalkenyl oder Phenyl bedeutet, wobei jeder der letztgenannten drei Reste unsubstituiert oder durch Reste aus der Gruppe Halogen, (C₁-C₄)Alkyl, (C₁-C₄)Haloalkyl und (C₁-C₄)Alkoxy substituiert ist, oder
zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
R¹⁰, R¹¹, R¹² jeweils unabhängig voneinander H oder einen acyclischen Kohlenwasserstoffrest mit 1 bis 6 C-Atomen, einem cyclischen Kohlenwasserstoffrest mit 3 bis 6 C-Atomen oder einen heterocyclischen Rest mit 3 bis 6 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
X eine Gruppe der Formel -O-, -S(O)_{q}-, -NR*- oder -N(O)-, wobei q = 0, 1 oder 2 ist und R* Wasserstoff oder Alkyl mit 1 bis 4 C-Atomen ist, oder eine Gruppe der Formel CR¹³R¹⁴, wobei die Definitionen für R¹³ und R¹⁴ aus den für R² und R³ möglichen Resten ausgewählt sind,
Y eine direkte Bindung oder eine Gruppe der Formel -O-, -S(O)ᵣ-, -NR**- oder -N(O)-, wobei r = 0, 1 oder 2 ist und R** Wasserstoff oder Alkyl mit 1 bis 4 C-Atomen ist, oder eine Gruppe der Formel -CH₂-, -C(CH₃)H- oder -C(CH₃)₂-,
Z¹, Z², Z³, Z⁴ jeweils unabhängig voneinander eine direkte Bindung oder eine divalente Gruppe der Formel
-O-, -S-, -CO-, -O-CO-, -CO-O-, -NR'-, -NR'-CO- oder -CO-NR'-, wobei R' H oder (C₁-C₄)Alkyl ist,
bedeuten.

3. Verbindungen der Formel (I) und deren Salze nach Anspruch 1 oder 2,
**dadurch gekennzeichnet, daß**
R¹, R², R³ unabhängig voneinander Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils mit 3 bis 9 Ringatome und 1 bis 3 Heteroringatome aus der Gruppe N, O, S enthält,
R² und R³ gemeinsam mit dem C-Atom der Gruppe CR²R³ einen gesättigten oder teilweise ungesättigten carbocyclischen Rest mit 3 bis 6 Ringgliedern oder Heterocyclyl mit 3 bis 6 Ringgliedern und 1 bis 3 Heteroringatomen aus der Gruppe O, N und S, wobei jeder der letztgenannten 2 cyclischen Reste unsubstituiert oder mit einem oder mehreren Resten aus der Gruppe (C₁-C₄)Alkyl, Halogen und Oxo substituiert ist,
R⁴ und R⁵ unabhängig voneinander Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocycl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
R⁶ Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Hetercycl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält,
R⁷ und R⁸ unabhängig voneinander Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 16 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
R⁹, wenn n = 1 ist, und die Reste R⁹, jeweils unabhängig voneinander, wenn n größer als 1 ist, Wasserstoff, Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere este aus der Gruppe (C₁-C₄)Alkyl, Halogen und Cyano substituiert sind, oder
Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 16 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome oder 1 bis 3 Heteroringatome aus der Gruppe N, O und S enthält, oder
zwei benachbarte Reste R⁹ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
x eine Gruppe der Formel -O-, -S- oder -NR*-, wobei R* Wasserstoff oder Alkyl mit 1 bis 4 C-Atomen ist, oder
eine Gruppe der Formel CR¹³R¹⁴, wobei die Definitionen für R¹³ und R¹⁴ aus den für R² und R³ möglichen Resten ausgewählt sind,
Y eine direkte Bindung oder eine Gruppe der Formel -O-, -S- oder -NR**-, wobei R** Wasserstoff oder Alkyl mit 1 bis 4 C-Atomen ist, oder eine Gruppe der Formel -CH₂-, -C(CH₃)H- oder -C(CH₃)₂-
bedeuten.

4. Verbindungen der Formel (I) und deren Salze nach einem der Ansprüche 1 bis 3, **dadurch gekennzeichnet, daß**
R¹ Wasserstoff, Halogen, Hydroxy, Amino, (C₁-C₄)Alkyl, (C₁-C₄)Alkoxy, (C₁-C₄)Alkylamino, Di-[(C₁-C₄)alkyl]-amino, Halo-(C₁-C₄)alkyl, (C₁-C₄)Alkoxy-(C₁-C₄)alkyl, (C₂-C₆)Alkenyl, (C₂-C₆)Alkinyl, Phenyl, Phenoxy oder einen Phenyl- oder Phenoxyrest, der im Phenylteil durch einen oder mehrere Reste aus der Gruppe Halogen, (C₁-C₄)Alkyl, (C₁-C₄)Alkoxy und (C₁-C₄)Alkoxy-carbonyl substituiert ist,
R² und R³ unabhängig voneinander Wasserstoff, Halogen, Hydroxy, Amino, (C₁-C₄)Alkyl, (C₁-C₄)Alkoxy, (C₁-C₄)Alkylamino, Di-[(C₁-C₄)alkyl]-amino, Halo-(C₁-C₄)alkyl, (C₁-C₄)Alkoxy-(C₁-C₄)alkyl, (C₂-C₆)Alkenyl, (C₂-C₆)Alkinyl, Phenyl oder Phenoxy oder einen Phenyl- oder Phenoxyrest, der im Phenylteil durch einen oder mehrere Reste aus der Gruppe Halogen, (C₁-C₄)Alkyl, (C₁-C₄)Alkoxy und (C₁-C₄)Alkoxy-carbonyl substituiert ist, oder
R² und R³ gemeinsam mit dem C-Atom der Gruppe CR²R³ einen gesättigten carbocyclischen Rest mit 3 bis 6 Ringgliedern, der unsubstituiert oder mit einem oder mehreren Resten aus der Gruppe (C₁-C₄)Alkyl, Halogen und Oxo substituiert ist,
R⁴ und R⁵ unabhängig voneinander Wasserstoff, Amino, Formyl, (C₁-C₄)Alkyl, (C₁-C₄)Alkoxy-(C₁-C₄)alkyl, (C₂-C₆)Alkenyl, (C₂-C₆)Alkinyl, (C₁-C₄)Alkylamino-(C₁-C₄)alkyl, Di-[(C₁-C₄)alkyl]-amino-(C₁-C₄)alkyl oder Phenyl, Phenyl-(C₁-C₄)alkyl oder Phenoxy-carbonyl oder einen der letztgenannten drei Reste, der im Phenylteil bis zu dreifach durch Reste aus der Gruppe Halogen, (C₁-C₄)Alkyl, (C₁-C₄)Alkoxy und (C₁-C₄)Alkoxy-carbonyl substituiert ist, oder
R⁴ und R⁵ gemeinsam mit dem Stickstoffatom der Gruppe NR⁴R⁵ einen heterocyclischen Rest mit 3 bis 6 Ringatomen und 1 bis 2 Heteroringatomen, wobei neben dem N-Atom das gegebenenfalls weitere Heteroringatom aus der Gruppe N und O ausgewählt ist und der Rest unsubstituiert oder durch einen oder mehrere Reste aus der Gruppe Halogen, (C₁-C₄)Alkyl und Oxo substituiert ist,
R⁶ Wasserstoff, Amino, Formyl, (C₁-C₄)Alkyl, Di-[(C₁-C₄)alkyl]-amino, (C₁-C₄)Alkoxy-(C₁-C₄)alkyl, (C₂-C₆)Alkenyl, (C₂-C₆)Alkinyl, (C₁-C₄)Dialkylamino-(C₁-C₄)alkyl, Phenyl, Phenoxy-(C₁-C₄)alkyl, Phenyl-(C₁-C₄)alkyl, Phenoxy-carbonyl, Phenylamino-carbonyl oder einen der letztgenannten fünf Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist,
R⁷ und R⁸ unabhängig voneinander Wasserstoff, (C₁-C₄)Alkyl, (C₁-C₄)Alkoxy-(C₁-C₄)alkyl, (C₂-C₆)Alkenyl, (C₂-C₆)Alkinyl, Di-[(C₁-C₄)alkyl]-amino-(C₁-C₄)alkyl, Phenyl, Phenoxy-(C₁-C₄)alkyl, Phenyl-(C₁-C₄)alkyl oder einen der letztgenannten drei Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist, oder
R⁷ und R⁸ gemeinsam eine Alkylenkette mit 2 bis 4 C-Atomen, die unsubstituiert oder durch einen oder mehrere Reste aus der Gruppe Halogen, (C₁-C₄)Alkyl und Oxo substituiert ist,
R⁹ unabhängig voneinander Wasserstoff, Halogen, Hydroxy, Amino, Nitro, Cyano, (C₁-C₄)Alkyl, (C₁-C₄)Alkoxy, (C₁-C₄)Alkylamino, Di-[(C₁-C₄)alkyl]-amino, Perhalo-(C₁-C₄)alkyl, Hydroxy-(C₁-C₄)alkyl, (C₁-C₄)Alkoxy-(C₁-C₄)alkyl, (C₁-C₄)Alkylthio, (C₂-C₆)Alkenyl, (C₂-C₆)Alkinyl, Di-[(C₁-C₄)alkyl]-amino-(C₁-C₄)alkyl, Phenyl, Phenoxy, Phenylcarbonyl, Phenylcarbonyl-(C₁-C₄)alkyl, (C₁-C₄)Alkoxycarbonyl-(C₁-C₄)alkyl, (C₁-C₄)Alkylcarbonyl, (C₁-C₄)Alkyloxycarbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 13 Reste, der im acyclischen Teil oder, vorzugsweise, im cyclischen Teil durch einen oder mehrere Reste aus der Gruppe Halogen, (C₁-C₄)Alkyl, (C₁-C₄)Alkoxy, (C₁-C₄)Alkylthio, (C₁-C₄)Haloalkyl, (C₁-C₄)Haloalkoxy und (C₁-C₄)Alkoxy-carbonyl substituiert ist, wobei Heterocyclyl in den Resten 3 bis 6 Ringatome und 1 bis 3 Heteroringatome aus der Gruppe N und O aufweist, oder
zwei benachbarte Reste R⁹ gemeinsam einen ankondensierten Cyclus mit 4 bis 6 Ringatomen, der carbocyclisch ist oder Heteroringatome aus der Gruppe O und N enthält und der unsubstituiert oder durch einen oder mehrere Reste (C₁-C₄)Alkyl substituiert ist,
X eine Gruppe der Formel -O- oder -NR*-, wobei R Wasserstoff oder Methyl ist, oder
eine Gruppe der Formel CR¹³R¹⁴, wobei die Definitionen für R¹³ und R¹⁴ aus den für R² und R³ möglichen Resten ausgewählt sind,
Y eine direkte Bindung oder eine Gruppe der Formel -O- oder -NR**-, wobei R** Wasserstoff oder Methyl ist,
bedeuten.

5. Verbindungen der Formel (I) und deren Salze nach einem der Ansprüche 1 bis 4, **dadurch gekennzeichnet, daß**
R¹ Wasserstoff, Halogen oder (C₁-C₄)Alkyl,
R² und R³ unabhängig voneinander Wasserstoff, Halogen, (C₁-C₄)Alkyl oder Phenyl oder
R² und R³ gemeinsam mit dem C-Atom der Gruppe CR²R³ gesättigtes (C₄-C₆)Cycloalkyl,
R⁴ und R⁵ unabhängig voneinander Wasserstoff, Amino, Formyl oder (C₁-C₄)Alkyl oder
R⁴ und R⁵ gemeinsam mit dem Stickstoffatom der Gruppe NR⁴R⁵ einen heterocyclischen Rest mit 4 bis 6 Ringatomen und 1 bis 2 Heteroringatomen, wobei neben dem N-Atom das gegebenenfalls weitere Heteroringatom aus der Gruppe N und O ausgewählt ist,
R⁶ Wasserstoff oder (C₁-C₄)Alkyl,
R⁷ und R⁸ unabhängig voneinander Wasserstoff, (C₁-C₄)Alkyl oder (C₁-C₄)Alkoxy-(C₁-C₄)alkyl oder
R⁷ und R⁸ gemeinsam eine Alkylenkette mit 2 bis 4 C-Atomen,
R⁹ unabhängig voneinander Wasserstoff, Halogen, Hydroxy, (C₁-C₄)Alkyl oder (C₁-C₄)Alkoxy,
m 1 oder 2,
n 2 oder 3,
X eine Gruppe der Formel -O- oder -NH- oder
eine Gruppe der Formel CR¹³R¹⁴, wobei die Definitionen für R¹³ und R¹⁴ aus den für R² und R³ möglichen Resten ausgewählt sind,
Y eine direkte Bindung oder eine Gruppe der Formel -O- oder -NH-
bedeuten.

6. Verfahren zur Herstellung von Verbindungen der Formel (I) oder deren Salzen nach Anspruch 1, **dadurch gekennzeichnet, daß** man
a) eine Verbindung der Formel (II), 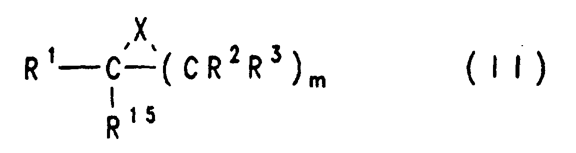 worin R¹⁵ eine funktionelle Gruppe aus der Gruppe Carbonsäureester, Carbonsäureorthoester, Carbonsäurechlorid, Carbonsäureamid, Carbonsäureanhydrid und Trichlormethyl bedeutet, mit einem Biguanidid der Formel (III) oder einem Säureadditionssalz hiervon 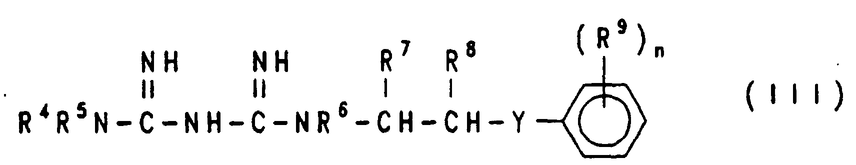 umsetzt oder
b) eine Verbindung der Formel (IV), 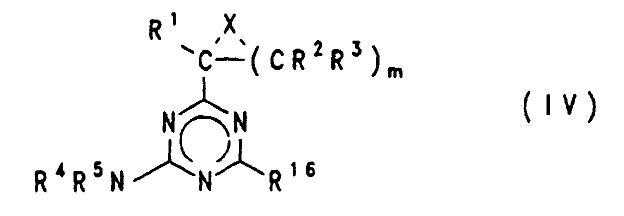 worin R¹⁶ einen austauschfähigen Rest oder eine Abgangsgruppe bedeutet, mit einem geeigneten Amin der Formel (V) oder einem Säureadditionssalz hiervon 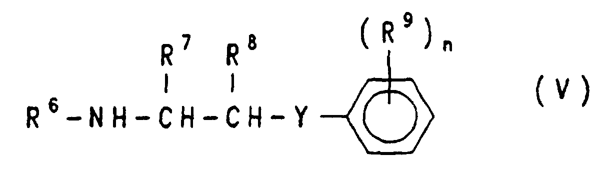 umsetzt oder
c) unter Reaktion einer austauschfähigen Gruppe am Triazin der Verbindungen der genannten Formel (IV) oder der Formel (X), 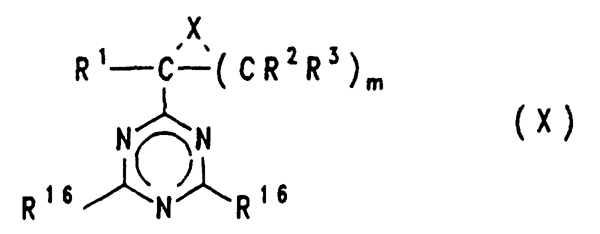 worin jeder der Reste R¹⁶ unabhängig voneinander einen austauschfähigen Rest oder eine Abgangsgruppe bedeutet, diese Verbindung mit einem geeigneten Aziridin der Formel (XI), 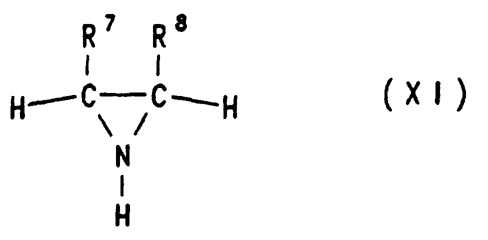 worin R⁷ und R⁸ wie in Formel (I) definiert sind, und nachfolgend mit einem geeigneten Nucleophil der Formel (XII) 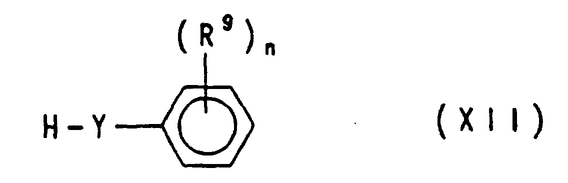 unter Ringöffnung des Aziridinrings und, falls ein Triazin der Formel (X). eingesetzt wurde, durch Reaktion der noch vorhandenen austauschfähigen Gruppe am Triazingrundgerüst mit einer Verbindung der Formel NHR⁴R⁵ umsetzt oder
d) eine Verbindung der Formel (XIII) 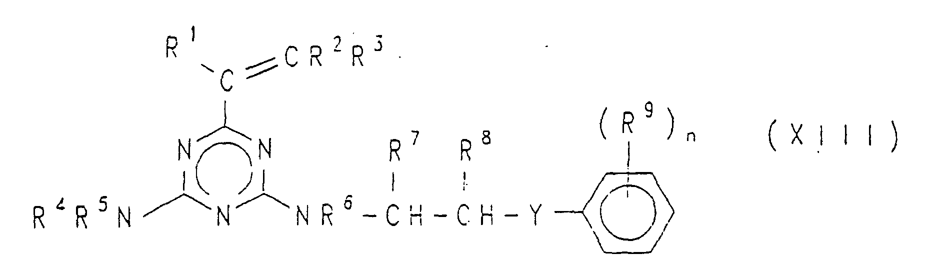 durch Epoxidierung oder Cyclopropanierung in entsprechende
Verbindungen der Formel (I) umsetzt,
wobei in den Formeln (II), (III), (IV), (V), (X), (XI), (XII) und (XIII) die Reste R¹, R², R³, R⁴, R⁵, R⁶, R⁷, R⁸, R⁹ sowie X, Y, m und n wie in Formel (I) definiert sind, mit der Maßgabe, daß in Variante d) für Formel (I) m = 1 ist.

7. Herbizides oder pflanzenwachstumsregulierende Mittel, **dadurch gekennzeichnet, daß** es mindestens eine Verbindung der Formel (I) oder deren Salz nach einem der Ansprüche 1 bis 5 und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 5 auf die Schadpflanzen bzw. Pflanzen, deren Pflanzensamen oder die Fläche, auf der sie wachsen, appliziert.

9. Verwendung der Verbindungen der Formel (I) oder deren Salze nach einem der Ansprüche 1 bis 5 als Herbizide oder Pflanzenwachstumsregulatoren.

10. Verbindungen der Formel (IV), 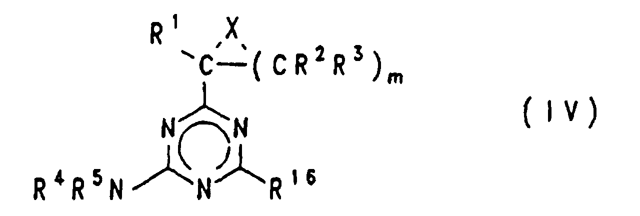 worin R¹ bis R⁵, X und m wie in Formel (I) nach einem der Ansprüche 1 bis 5 definiert sind und
R¹⁶ (C₁-C₄)Alkylthio, (C₁-C₄)Alkylsulfonyl, Phenyl-(C₁-C₄)alkylthio, Phenyl-(C₁-C₄)alkylsulfonyl, (C₁-C₄)Alkylphenylthio oder (C₁-C₄)Alkylphenylsulfonyl bedeutet.

## Claims

1. A compound of the formula (I) or salt thereof, 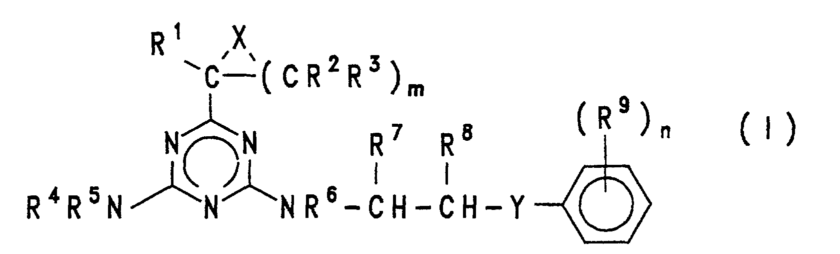 in which
R¹ is hydrogen, halogen, nitro, cyano, thiocyanato or a radical of the formula -Z¹-R¹⁰,
R² and R³ in each case independently of one another are hydrogen, halogen, nitro, cyano, thiocyanato or a radical of the formula -Z²-R¹¹,
or
R² and R³, together with the carbon atom of the group CR²R³, are a carbocyclic radical having 4 to 10 ring members or a heterocyclic radical having 4 to 10 ring members and hetero-ring atoms from the group consisting of N, O and S, each of the two latter radicals being unsubstituted or substituted by one or more radicals from the group consisting of halogen, (C₁-C₄)alkyl and oxo,
R⁴ and R⁵ in each case independently of one another are hydrogen, amino, alkylamino or dialkylamino having in each case 1 to 6 carbon atoms in the alkyl radical, an acyclic or cyclic hydrocarbon radical or hydrocarbonoxy radical having in each case 1 to 10 carbon atoms, or a heterocyclyl radical, heterocyclyloxy radical or heterocyclylamino radical having in each case 3 to 6 ring atoms and 1 to 3 hetero-ring atoms from the group consisting of N, O and S, each of the five latter radicals being unsubstituted or substituted, or an acyl radical, or
R⁴ and R⁵, together with the nitrogen atom of the group NR⁴R⁵, are a heterocyclic radical having 3 to 6 ring atoms and 1 to 4 hetero-ring atoms, any further hetero-ring atoms in addition to the N atom being selected from the group consisting of N, O and S, and the radical being unsubstituted or substituted,
R⁶ is hydrogen, amino, alkylamino or dialkylamino having in each case 1 to 6 carbon atoms in the alkyl radical, an acyclic or cyclic hydrocarbon radical or hydrocarbonoxy radical having in each case 1 to 10 carbon atoms, or a heterocyclyl radical, heterocyclyloxy radical or heterocyclylamino radical having in each case 3 to 6 ring atoms and 1 to 3 hetero-ring atoms from the group consisting of N, O and S, each of the five latter radicals being unsubstituted or substituted, or an acyl radical,
R⁷ and R⁸ in each case independently of one another are hydrogen, halogen, nitro, cyano, thiocyanato or a radical of the formula -Z³-R¹², or
R⁷ and R⁸ together are an alkylene chain having 2 to 4 carbon atoms which is unsubstituted or substituted by one or more radicals from the group consisting of halogen, (C₁-C₄)alkyl and oxo,
R⁹ independently at each occurrence is halogen, nitro, formyl, carboxyl, cyano, thiocyanato, (C₁-C₆)alkyl, (C₂-C₆)alkenyl, (C₂-C₆)alkynyl, (C₁-C₆)alkoxy, (C₂-C₆)alkenyloxy, (C₂-C₆)alkynyloxy, (C₁-C₄)alkylcarbonyl, (C₁-C₄)alkyloxycarbonyl, (C₁-C₄)alkoxy-thiocarbonyl, (C₁-C₄)alkylthio-carbonyl or (C₁-C₄)alkylthio-thiocarbonyl, the alkyl radicals in the 11 latter radicals being unsubstituted or substituted, or (C₁-C₄)alkylsulfonyl, (C₁-C₄)haloalkylsulfonyl, (C₁-C₄)alkoxysulfonyl, amino, mono- or di-[(C₁-C₆)alkyl]amino, aminocarbonyl, mono- or di-[(C₁-C₆)alkyl]aminocarbonyl, (C₁-C₆)alkanoylamino, N-(C₁-C₆)alkanoyl-N-(C₁-C₄)alkyl-amino or a radical of the formula Z⁴-R^{o}, in which Z⁴ is as defined below and R° is an aromatic, saturated or partially saturated carbocyclic or heterocyclic radical, the cyclic radical being substituted or unsubstituted, or two adjacent radicals R⁹ together are a fused-on ring having 4 to 6 ring atoms, which is carbocyclic or comprises hetero-ring atoms from the group consisting of O, S and N, and which is unsubstituted or substituted by one or more radicals from the group consisting of halogen, (C₁-C₄)alkyl and oxo,
R¹⁰, R¹¹, R¹² in each case independently of one another are H or an acyclic hydrocarbon radical or cyclic hydrocarbon radical or a heterocyclic radical, each of the three latter radicals being unsubstituted or substituted,
X is a group of the formula -O-, -S(O)_{q}-, -NR*- or -N(O)-, in which q is 0, 1 or 2 and R* is hydrogen or alkyl having 1 to 4 carbon atoms, or a group of the formula CR¹³R¹⁴ in which the definitions of R¹³ and R¹⁴ are selected from the radicals possible for R² and R³,
Y is a direct bond or a group of the formula -O-, -S(O)ᵣ-, -NR**- or -N(O)-, in which r is 0, 1 or 2 and R** is hydrogen or alkyl having 1 to 4 carbon atoms, or a group of the formula -CH₂-, -C(CH₃)H- or -C(CH₃)₂-,
Z¹, Z², Z³ and Z⁴ in each case independently of one another are a direct bond or a divalent group of the formula
-O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'- in which p is 0, 1 or 2 and R' is hydrogen, alkyl having 1 to 6 carbon atoms, phenyl, benzyl, cycloalkyl having 3 to 6 carbon atoms or alkanoyl having 1 to 6 carbon atoms,
m is 1, 2, 3 or 4, and
n is 0, 1, 2, 3, 4 or 5.

2. A compound of the formula (I) or salt thereof as claimed in claim 1, wherein
R¹ is H, halogen, NO₂, CN, SCN or -Z¹-R¹⁰,
R² and R³ in each case independently of one another are H, halogen, NO₂, CN, SCN or a radical of the formula -Z²-R¹¹ or
R² and R³, together with the carbon atom of the group CR²R³, are (C₃-C₈)cycloalkyl, (C₃-C₈)cycloalkenyl or a heterocyclic radical having 3 to 6 ring members and having hetero-ring atoms from the group consisting of N, O and S, each of the 3 latter radicals being unsubstituted or substituted by one or more radicals from the group consisting of halogen, (C₁-C₄)alkyl and oxo,
R⁴ and R⁵ in each case independently of one another are hydrogen, amino, alkylamino or dialkylamino having in each case 1 to 4 carbon atoms in the alkyl radical, an acyclic or cyclic hydrocarbon radical or hydrocarbonoxy radical having in each case 1 to 6 carbon atoms or a heterocyclyl radical, heterocyclyloxy radical or heterocyclylamino radical having in each case 3 to 6 ring atoms and 1 to 3 hetero-ring atoms from the group consisting of N, O and S, each of the five latter radicals being unsubstituted or substituted by one or more radicals from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]-carbonyl, [(C₁-C₄)alkyl]-carbonyl, formyl, carbamoyl, mono- and di-[(C₁-C₄)alkyl]-aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl, or an acyl radical, or
R⁴ and R⁵, together with the nitrogen atom of the group NR⁴R⁵, are a heterocyclic radical having 3 to 6 ring atoms and 1 to 2 hetero-ring atoms, any further hetero-ring atom in addition to the N atom being selected from the group consisting of N, O and S and the radical being unsubstituted or substituted by one or more radicals from the group consisting of halogen, (C₁-C₄)alkyl and oxo,
R⁶ is hydrogen, amino, alkylamino or dialkylamino having in each case 1 to 6 carbon atoms in the alkyl radical, an acyclic or cyclic hydrocarbon radical or hydrocarbonoxy radical having in each case 1 to 6 carbon atoms or a heterocyclyl radical, heterocyclyloxy radical or heterocyclylamino radical having in each case 3 to 6 ring atoms and 1 to 3 hetero-ring atoms from the group consisting of N, O and S, each of the five latter radicals being unsubstituted or substituted by one or more radicals from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]-carbonyl, [(C₁-C₄)alkyl]-carbonyl, formyl, carbamoyl, mono- and di-[(C₁-C₄)alkyl]-aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl,
or an acyl radical,
R⁷ and R⁸ in each case independently of one another are H, halogen, NO₂, CN, SCN or a radical of the formula - Z³-R¹² or
R⁷ and R⁸ together are an alkylene chain having 2 to 4 carbon atoms which is unsubstituted or substituted by one or more radicals from the group consisting of halogen, (C₁-C₄)alkyl and oxo,
R⁹ independently at each occurrence is halogen, nitro, formyl, carboxyl, cyano, thiocyanato, (C₁-C₆)alkyl, (C₁-C₆)alkoxy, (C₁-C₄)alkylcarbonyl, (C₁-C₄)alkyloxycarbonyl or (C₁-C₄)alkylthiocarbonyl, the five latter radicals being unsubstituted or substituted by halogen or (C₁-C₄)alkoxy, or (C₁-C₄)alkylsulfonyl, (C₁-C₄)haloalkylsulfonyl, mono- or di-[(C₁-C₄)alkyl]amino, aminocarbonyl, mono- or di-[(C₁-C₄)alkyl]aminocarbonyl, (C₁-C₄)alkanoylamino or a radical of the formula Z⁴-R^{o}, in which Z⁴ is as defined below and R° is (C₃-C₆)cycloalkyl, (C₃-C₆)cycloalkenyl or phenyl, each of the three latter radicals being unsubstituted or substituted by radicals from the group consisting of halogen, (C₁-C₄)alkyl, (C₁-C₄)haloalkyl and (C₁-C₄)alkoxy, or two adjacent radicals R⁹ together are a fused-on ring having 4 to 6 ring atoms, which is carbocyclic or comprises hetero-ring atoms from the group consisting of O, S and N, and which is unsubstituted or substituted by one or more radicals from the group consisting of halogen, (C₁-C₄)alkyl and oxo,
R¹⁰, R¹¹ and R¹² in each case independently of one another are H or an acyclic hydrocarbon radical having 1 to 6 carbon atoms, a cyclic hydrocarbon radical having 3 to 6 carbon atoms or a heterocyclic radical having 3 to 6 ring atoms and 1 to 3 hetero-ring atoms from the group consisting of N, O and S, each of the three latter radicals being unsubstituted or substituted by one or more radicals from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]-carbonyl, [(C₁-C₄)alkyl]-carbonyl, formyl, carbamoyl, mono- and di-[(C₁-C₄)alkyl]-aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl,
X is a group of the formula -O-, -S(O)_{q}-, -NR*- or -N(O)-, in which q is 0, 1 or 2 and R* is hydrogen or alkyl having 1 to 4 carbon atoms, or a group of the formula CR¹³R¹⁴ in which the definitions of R¹³ and R¹⁴ are selected from the radicals possible for R² and R³,
Y is a direct bond or a group of the formula -O-, -S(O)ᵣ-, -NR**- or -N(O)-, in which r is 0, 1 or 2 and R** is hydrogen or alkyl having 1 to 4 carbon atoms, or a group of the formula -CH₂-, -C(CH₃)H- or -C(CH₃)₂-,
Z¹, Z², Z³ and Z⁴ in each case independently of one another are a direct bond or a divalent group of the formula
-O-, -S-, -CO-, -O-CO-, -CO-O-, -NR'-, -NR'-CO- or
-CO-NR'-, in which R' is H or (C₁-C₄)alkyl.

3. A compound of the formula (I) or salt thereof as claimed in claim 1 or 2, wherein
R¹, R² and R³ independently of one another are hydrogen, halogen, hydroxyl, amino, nitro, formyl, carboxyl, cyano, thiocyanato, (C₁-C₄)alkyl, cyano-(C₁-C₄)-alkyl, (C₁-C₄)alkoxy, (C₁-C₄)alkylamino, di-[(C₁-C₄)alkyl]-amino, halo- (C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₁-C₄)alkylthio, halo-(C₁-C₄)alkylthio, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di-[(C₁-C₄)alkyl]-amino-(C₁-C₄)-alkyl, (C₃-C₉)cycloalkylamino-(C₁-C₄)alkyl, (C₃-C₉)-cycloalkyl, heterocyclyl-(C₁-C₄)alkyl having 3 to 9 ring members, the cyclic groups in the 3 latter radicals being unsubstituted or substituted by one or more radicals from the group consisting of (C₁-C₄)alkyl, halogen and cyano, or phenyl, phenoxy, phenylcarbonyl, phenylcarbonyl-(C₁-C₄)-alkyl, (C₁-C₄)alkoxy-carbonyl-(C₁-C₄)alkyl, (C₁-C₄)-alkylamino-carbonyl-(C₁-C₄)alkyl, (C₁-C₄)alkylcarbonyl, (C₁-C₄)alkoxy-carbonyl, aminocarbonyl, (C₁-C₄)alkylamino-carbonyl, phenoxy-(C₁-C₄)alkyl, phenyl-(C₁-C₄)alkyl, heterocyclyl, heterocyclylamino, heterocyclyloxy, heterocyclylthio, or one of the 16 latter radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄) haloalkyl, (C₁-C₄)haloalkoxy, formyl, (C₁-C₄)alkyl-carbonyl, (C₁-C₄)alkoxy-carbonyl and (C₁-C₄)alkoxy, heterocyclyl in the radicals containing in each case 3 to 9 ring atoms and 1 to 3 hetero-ring atoms from the group consisting of N, O and S , or
R² and R³ together with the carbon atom of the group CR²R³, are a saturated or partially unsaturated carbocyclic radical having 3 to 6 ring members or heterocyclyl having 3 to 6 ring members and 1 to 3 hetero-ring atoms from the group consisting of O, N and S, each of the 2 latter cyclic radicals being unsubstituted or substituted by one or more radicals from the group consisting of (C₁-C₄)alkyl, halogen and oxo,
R⁴ and R⁵ independently of one another are hydrogen, amino, formyl, (C₁-C₄)alkyl, cyano-(C₁-C₄)alkyl, (C₁-C₄)alkylamino, di-[(C₁-C₄)alkyl]-amino, halo-(C₁-C₄)alkyl, hydroxy-(C₁-C₄)alkyl, (C₁-C₄)alkoxy-(C₁-C₄)alkyl, halo(C₁-C₄)alkoxy-(C₁-C₄)alkyl, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di-[(C₁-C₄)alkyl]-amino-(C₁-C₄)alkyl, (C₃-C₉)cycloalkylamino-(C₁-C₄)alkyl, (C₃-C₉)cycloalkyl, (C₃-C₉)heterocyclyl-(C₁-C₄)alkyl having 3 to 9 ring members, the cyclic groups in the 3 latter radicals being unsubstituted or substituted by one or more radicals from the group consisting of (C₁-C₄)alkyl, halogen and cyano, or
phenyl, phenoxy, phenylcarbonyl, phenylcarbonyl-(C₁-C₄)alkyl, (C₁-C₄)alkoxy-carbonyl-(C₁-C₄)alkyl, (C₁-C₄)alkylamino-carbonyl-(C₁-C₄)alkyl, (C₁-C₄)-alkylcarbonyl, (C₁-C₄)alkoxy-carbonyl, aminocarbonyl, (C₁-C₄)alkylamino-carbonyl, phenoxy-(C₁-C₄)alkyl, phenyl-(C₁-C₄)alkyl, heterocyclyl, heterocyclylamino, heterocyclyloxy, heterocyclylthio, or one of the 16 latter radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkyl-carbonyl, (C₁-C₄)alkoxy-carbonyl and (C₁-C₄)alkoxy, heterocyclyl in the radicals containing in each case 3 to 9 ring atoms and 1 to 3 hetero-ring atoms from the group consisting of N, O and S, or
R⁴ and R⁵, together with the nitrogen atom of the group NR⁴R⁵, are a heterocyclic radical having 3 to 6 ring atoms and 1 to 2 hetero-ring atoms, any further hetero-ring atom in addition to the N atom being selected from the group consisting of N, O and S, and the radical being unsubstituted or substituted by one or more radicals from the group consisting of halogen, (C₁-C₄)alkyl and oxo,
R⁶ is hydrogen, amino, formyl, (C₁-C₄)alkyl, cyano-(C₁-C₄)alkyl, (C₁-C₄)alkylamino, di-[(C₁-C₄)-alkyl]-amino, halo-(C₁-C₄)alkyl, hydroxy-(C₁-C₄)-alkyl, (C₁-C₄)alkoxy-(C₁-C₄)alkyl, halo(C₁-C₄)-alkoxy-(C₁-C₄)alkyl, (C₂-C₆)alkenyl, halo-(C₂-C₆)-alkenyl, (C₂-C₆)alkynyl, halo-(C₂-C₆)alkynyl, (C₁-C₄)alkylamino-(C₁-C₄)alkyl, di-[(C₁-C₄)alkyl]-amino-(C₁-C₄)alkyl, (C₃-C₉)cycloalkylamino-(C₁-C₄)-alkyl, (C₃-C₉)cycloalkyl, (C₃-C₉)heterocyclyl-(C₁-C₄)alkyl having 3 to 9 ring members, the cyclic groups in the 3 latter radicals being unsubstituted or substituted by one or more radicals from the group consisting of (C₁-C₄)alkyl, halogen and cyano, or
phenyl, phenoxy, phenylcarbonyl, phenylcarbonyl-(C₁-C₄)alkyl, (C₁-C₄)alkoxy-carbonyl-(C₁-C₄)-alkyl, (C₁-C₄)alkylamino-carbonyl-(C₁-C₄)alkyl, (C₁-C₄)alkyl-carbonyl, (C₁-C₄)alkoxy-carbonyl, aminocarbonyl, (C₁-C₄)alkylamino-carbonyl, phenoxy-(C₁-C₄)alkyl, phenyl-(C₁-C₄)alkyl, heterocyclyl, heterocyclylamino, heterocyclyloxy, heterocyclylthio, or one of the 16 latter radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkyl-carbonyl, (C₁-C₄)alkoxy-carbonyl and (C₁-C₄)alkoxy, heterocyclyl in the radicals containing in each case 3 to 9 ring atoms and 1 to 3 hetero-ring atoms from the group consisting of N, O and S, or
R⁷ and R⁸ independently of one another are hydrogen, halogen, hydroxyl, amino, nitro, formyl, carboxyl, cyano, thiocyanato, (C₁-C₄)alkyl, cyano-(C₁-C₄)-alkyl, (C₁-C₄)alkoxy, (C₁-C₄)alkylamino, di-[(C₁-C₄)alkyl] -amino, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₁-C₄)alkylthio, halo-(C₁-C₄)alkylthio, (C₂-C₆)alkenyl, halo-(C₂-C₆)-alkenyl, (C₂-C₆)alkynyl, halo-(C₂-C₆)alkynyl, (C₁-C₄)alkylamino-(C₁-C₄)alkyl, di-[(C₁-C₄)alkyl]-amino-(C₁-C₄)alkyl, (C₃-C₉)cycloalkylamino-(C₁-C₄)-alkyl, (C₃-C₉)cycloalkyl, heterocyclyl-(C₁-C₄)alkyl having 3 to 9 ring members, the cyclic groups in the 3 latter radicals being unsubstituted or substituted by one or more radicals from the group consisting of (C₁-C₄)alkyl, halogen and cyano, or phenyl, phenoxy, phenylcarbonyl, phenylcarbonyl-(C₁-C₄)alkyl, (C₁-C₄)alkoxy-carbonyl-(C₁-C₄)-alkyl, (C₁-C₄)alkylamino-carbonyl-(C₁-C₄)alkyl, (C₁-C₄)alkyl-carbonyl, (C₁-C₄)alkoxy-carbonyl, aminocarbonyl, (C₁-C₄)alkylamino-carbonyl, phenoxy-(C₁-C₄)alkyl, phenyl-(C₁-C₄)alkyl, heterocyclyl, heterocyclylamino, heterocyclyloxy, heterocyclylthio, or one of the 16 latter radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkyl-carbonyl, (C₁-C₄)alkoxy-carbonyl and (C₁-C₄)alkoxy, heterocyclyl in the radicals containing in each case 3 to 9 ring atoms and 1 to 3 hetero-ring atoms from the group consisting of N, O and S, or
R⁷ and R⁸ together are an alkylene chain having 2 to 4 carbon atoms, which is unsubstituted or substituted by one or more radicals from the group consisting of halogen, (C₁-C₄)alkyl and oxo,
R⁹, if n is 1, and the radicals R⁹, independently at each occurrence, if n is greater than 1, is or are hydrogen, halogen, hydroxyl, amino, nitro, formyl, carboxyl, cyano, thiocyanato, (C₁-C₄)alkyl, cyano-(C₁-C₄)alkyl, (C₁-C₄)alkoxy, (C₁-C₄)alkyl-amino, di-[(C₁-C₄)alkyl]-amino, halo-(C₁-C₄)alkyl, hydroxy-(C₁-C₄)alkyl, (C₁-C₄)alkoxy-(C₁-C₄)alkyl, halo(C₁-C₄)alkoxy-(C₁-C₄)alkyl, (C₁-C₄)alkylthio, halo-(C₁-C₄)alkylthio, (C₂-C₆)alkenyl, halo-(C₂-C₆)-alkenyl, (C₂-C₆)alkynyl, halo-(C₂-C₆)alkinyl, (C₁-C₄)alkylamino-(C₁-C₄)alkyl, di-[(C₁-C₄)alkyl]-amino-(C₁-C₄)alkyl, (C₃-C₉)cycloalkylamino-(C₁-C₄)-alkyl, (C₃-C₉)cycloalkyl, heterocyclyl-(C₁-C₄)alkyl having 3 to 9 ring members, the cyclic groups in the 3 latter radicals being unsubstituted or substituted by one or more radicals from the group consisting of (C₁-C₄)alkyl, halogen and cyano, or phenyl, phenoxy, phenylcarbonyl, phenylcarbonyl-(C₁-C₄)alkyl, (C₁-C₄)alkoxy-carbonyl-(C₁-C₄)alkyl, (C₁-C₄)alkylamino-carbonyl-(C₁-C₄)alkyl, (C₁-C₄)-alkyl-carbonyl, (C₁-C₄)alkoxy-carbonyl, aminocarbonyl, (C₁-C₄)alkylamino-carbonyl, phenoxy--(C₁-C₄)alkyl, phenyl-(C₁-C₄)alkyl, heterocyclyl, heterocyclylamino, heterocyclyloxy, heterocyclylthio or one of the 16 latter radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkyl-carbonyl, (C₁-C₄)alkoxy-carbonyl and (C₁-C₄)alkoxy, heterocyclyl in the radicals containing in each case 3 to 9 ring atoms and 1 to 3 hetero-ring atoms from the group consisting of N, O and S, or
two adjacent radicals R⁹ together are a fused-on ring having 4 to 6 ring atoms which is carbocyclic or comprises hetero-ring atoms from the group consisting of O, S and N, and which is unsubstituted or substituted by one or more radicals from the group consisting of halogen, (C₁-C₄)alkyl and oxo,
X is a group of the formula -O-, -S- or -NR*-, in which R* is hydrogen or alkyl having 1 to 4 carbon atoms, or
a group of the formula CR¹³R¹⁴, in which the definitions of R¹³ and R¹⁴ are selected from the radicals possible for R² and R³,
Y is a direct bond or a group of the formula -O-, -S- or -NR**-, in which R** is hydrogen or alkyl having 1 to 4 carbon atoms, or a group of the formula -CH₂-, -C(CH₃)H- or -C(CH₃)₂-.

4. A compound of the formula (I) or salt thereof as claimed in any of claims 1 to 3, wherein
R¹ is hydrogen, halogen, hydroxyl, amino, (C₁-C₄)-alkyl, (C₁-C₄)alkoxy, (C₁-C₄)alkylamino, di-[(C₁-C₄)-alkyl]-amino, halo-(C₁-C₄)alkyl, (C₁-C₄)alkoxy-(C₁-C₄)alkyl, (C₂-C₆)alkenyl, (C₂-C₆)alkynyl, phenyl, phenoxy or a phenyl or phenoxy radical which is substituted in the phenyl moiety by one or more radicals from the group consisting of halogen, (C₁-C₄)alkyl, (C₁-C₄)alkoxy and (C₁-C₄)alkoxy-carbonyl,
R² and R³ independently of one another are hydrogen, halogen, hydroxyl, amino, (C₁-C₄)alkyl, (C₁-C₄)alkoxy, (C₁-C₄)alkylamino, di-[(C₁-C₄)alkyl]-amino, halo-(C₁-C₄)alkyl, (C₁-C₄)alkoxy-(C₁-C₄)alkyl, (C₂-C₆)alkenyl, (C₂-C₆)alkynyl, phenyl or phenoxy or a phenyl or phenoxy radical which is substituted in the phenyl moiety by one or more radicals from the group consisting of halogen, (C₁-C₄)alkyl, (C₁-C₄)alkoxy and (C₁-C₄)alkoxy-carbonyl, or
R² and R³, together with the carbon atom of the group CR²R³, are a saturated carbocyclic radical having 3 to 6 ring members which is unsubstituted or substituted by one or more radicals from the group consisting of (C₁-C₄)alkyl, halogen and oxo,
R⁴ and R⁵ independently of one another are hydrogen, amino, formyl, (C₁-C₄)alkyl, (C₁-C₄)alkoxy(C₁-C₄)alkyl, (C₂-C₆)alkenyl, (C₂-C₆)alkynyl, (C₁-C₄)alkylamino-(C₁-C₄)alkyl, di-[(C₁-C₄)alkyl]-amino-(C₁-C₄)alkyl or
phenyl, phenyl-(C₁-C₄)alkyl or phenoxy-carbonyl or one of the three latter radicals which is substituted in the phenyl moiety up to three times by radicals from the group consisting of halogen, (C₁-C₄)alkyl, (C₁-C₄)alkoxy and (C₁-C₄)alkoxy-carbonyl, or
R⁴ and R⁵, together with the nitrogen atom of the group NR⁴R⁵, are a heterocyclic radical having 3 to 6 ring atoms and 1 to 2 hetero-ring atoms, any further hetero-ring atom in addition to the N atom being selected from the group consisting of N and O, and the radical being unsubstituted or substituted by one or more radicals from the group consisting of halogen, (C₁-C₄)alkyl and oxo,
R⁶ is hydrogen, amino, formyl, (C₁-C₄)alkyl, di-[(C₁-C₄)alkyl]-amino, (C₁-C₄)alkoxy-(C₁-C₄)alkyl, (C₂-C₆)alkenyl, (C₂-C₆)alkynyl, (C₁-C₄)dialkylamino-(C₁-C₄)alkyl, phenyl, phenoxy-(C₁-C₄)alkyl, phenyl-(C₁-C₄)alkyl, phenoxy-carbonyl, phenyl-amino-carbonyl or one of the five latter radicals which is substituted in the phenyl moiety from one to three times by radicals from the group consisting of halogen, (C₁-C₄)alkyl, (C₁-C₄)alkoxy and (C₁-C₄)alkoxy-carbonyl,
R⁷ and R⁸ independently of one another are hydrogen, (C₁-C₄)alkyl, (C₁-C₄)alkoxy-(C₁-C₄)alkyl, (C₂-C₆)alkenyl, (C₂-C₆)alkynyl, di-[(C₁-C₄)alkyl]amino-(C₁-C₄)alkyl, phenyl, phenoxy-(C₁-C₄)alkyl, phenyl-(C₁-C₄)alkyl or one of the three latter radicals, which is substituted in the phenyl moiety from one to three times by radicals from the group consisting of halogen, (C₁-C₄)alkyl, (C₁-C₄)alkoxy and (C₁-C₄)alkoxy-carbonyl, or
R⁷ and R⁸ together are an alkylene chain having 2 to 4 carbon atoms, which is unsubstituted or substituted by one or more radicals from the group consisting of halogen, (C₁-C₄)alkyl and oxo,
R⁹ independently at each occurrence is hydrogen, halogen, hydroxyl, amino, nitro, cyano, (C₁-C₄)alkyl, (C₁-C₄)alkoxy, (C₁-C₄)alkylamino, di-[(C₁-C₄)alkyl]-amino, perhalo-(C₁-C₄)alkyl, hydroxy-(C₁-C₄)alkyl, (C₁-C₄)alkoxy-(C₁-C₄)alkyl, (C₁-C₄)alkylthio, (C₂-C₆)alkenyl, (C₂-C₆)alkynyl, di-[(C₁-C₄)alkyl]-amino-(C₁-C₄)alkyl, phenyl, phenoxy, phenylcarbonyl, phenylcarbonyl-(C₁-C₄)alkyl, (C₁-C₄)alkoxycarbonyl-(C₁-C₄)alkyl, (C₁-C₄)alkylcarbonyl, (C₁-C₄)alkyloxycarbonyl, phenoxy-(C₁-C₄)alkyl, phenyl-(C₁-C₄)alkyl, heterocyclyl, heterocyclylamino, heterocyclyloxy, heterocyclylthio or one of the 13 latter radicals which is substituted in the acyclic moiety or, preferably, in the cyclic moiety by one or more radicals from the group consisting of halogen, (C₁-C₄)alkyl, (C₁-C₄)alkoxy, (C₁-C₄)alkylthio, (C₁-C₄)haloalkyl, (C₁-C₄)haloalkoxy and (C₁-C₄)alkoxy-carbonyl, heterocyclyl in the radicals having 3 to 6 ring atoms and 1 to 3 hetero-ring atoms from the group consisting of N and O, or
two adjacent radicals R⁹ together are a fused-on ring having 4 to 6 ring atoms which is carbocyclic or comprises hetero-ring atoms from the group consisting of O and N, and which is unsubstituted or substituted by one or more (C₁-C₄)alkyl radicals,
X is a group of the formula -O- or -NR*-, in which R* is hydrogen or methyl, or
a group of the formula CR¹³R¹⁴, in which the definitions of R¹³ and R¹⁴ are selected from the radicals possible for R² and R³,
Y is a direct bond or a group of the formula -O- or -NR**-, in which R** is hydrogen or methyl.

5. A compound of the formula (I) or salt thereof as claimed in any of claims 1 to 4, wherein
R¹ is hydrogen, halogen or (C₁-C₄)alkyl,
R² and R³ independently of one another are hydrogen, halogen, (C₁-C₄)alkyl or phenyl or
R² and R³ together with the carbon atom of the group CR²R³, are saturated (C₄-C₆)cycloalkyl,
R⁴ and R⁵ independently of one another are hydrogen, amino, formyl or (C₁-C₄)alkyl, or
R⁴ and R⁵ together with the nitrogen atom of the group NR⁴R⁵, are a heterocyclic radical having 4 to 6 ring atoms and 1 to 2 hetero-ring atoms, any further hetero-ring atom in addition to the N atom being selected from the group consisting of N and O,
R⁶ is hydrogen or (C₁-C₄)alkyl,
R⁷ and R⁸ independently of one another are hydrogen, (C₁-C₄)alkyl or (C₁-C₄)alkoxy-(C₁-C₄)alkyl or
R⁷ and R⁸ together are an alkylene chain having 2 to 4 carbon atoms,
R⁹ independently at each occurrence is hydrogen, halogen, hydroxyl, (C₁-C₄)alkyl or (C₁-C₄)alkoxy,
m is 1 or 2,
n is 2 or 3,
X is a group of the formula -O- or -NH-, or
a group of the formula CR¹³R¹⁴, in which the definitions of R¹³ and R¹⁴ are selected from the radicals possible for R² and R³,
Y is a direct bond or a group of the formula -O- or -NH-.

6. A process for the preparation of a compound of the formula (I) or salt thereof as claimed in claim 1, which comprises
a) reacting a compound of the formula (II), 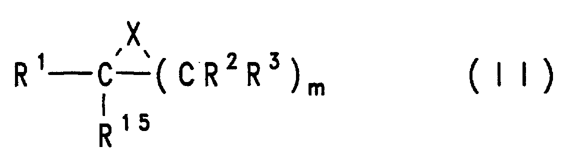 in which R¹⁵ is a functional group from the group consisting of carboxylate, carboxylic acid ortho ester, carbonyl chloride, carboxamide, carboxylic anhydride and trichloromethyl with a biguanidide of the formula (III) or an acid addition salt thereof 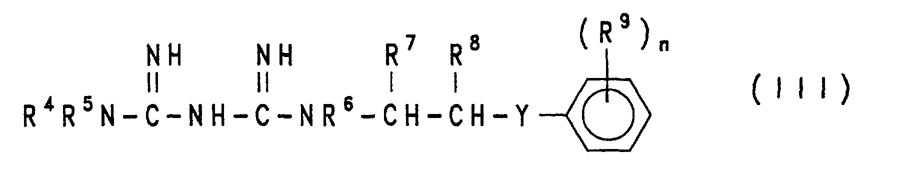 or
a) reacting a compound of the formula (IV), 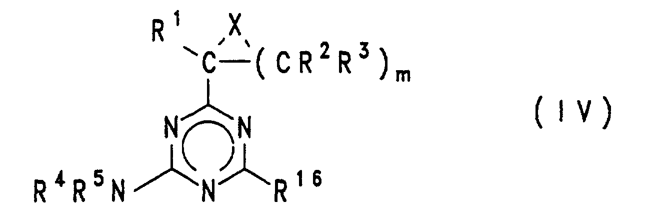 in which R¹⁶ is an exchangeable radical or a leaving group with a suitable amine of the formula (V) or an acid addition salt thereof 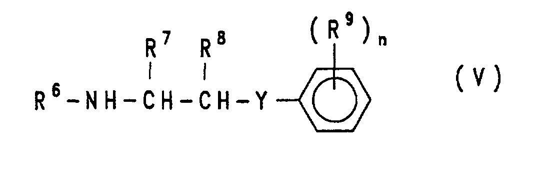 or
b) with reaction of an exchangeable group on the triazine of the compounds of the abovementioned formula (IV) or of the formula (X) 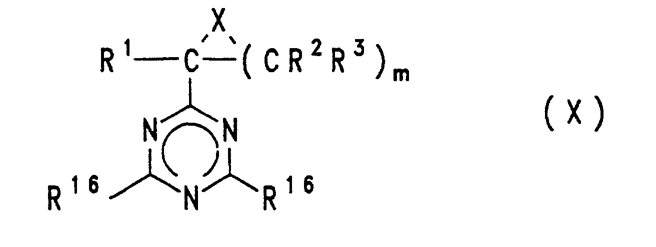 in which each of the radicals R¹⁶ independently of one another is an exchangeable radical or a leaving group, reacting this compound with a suitable aziridine of the formula (XI) 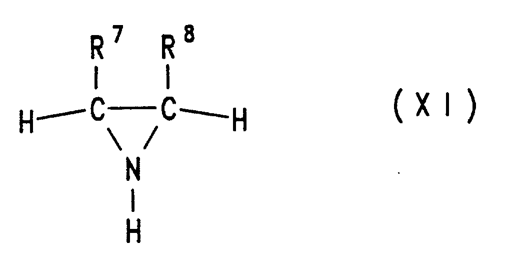 in which R⁷ and R⁸ are as defined for formula (I) and subsequently with a suitable nucleophile of the formula (XII) 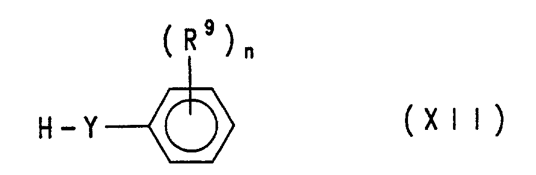 with ring opening of the aziridine ring, and, if a triazine of the formula (X) has been used, by reaction of the exchangeable group which is still present on the triazine framework with a compound of the formula NHR⁴R⁵, or
d) reacting a compound of the formula (XIII) 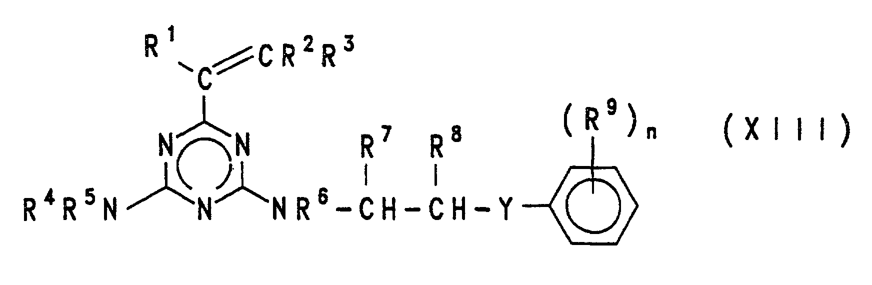 by epoxidization or cyclopropanization to give corresponding compounds of the formula (I),
where, in the formulae (II), (III), (IV), (V), (X), (XI), (XII) and (XIII), the radicals R¹, R², R³, R⁴, R⁵, R⁶, R⁷, R⁸ and R⁹ and also X, Y, m and n are as defined for formula (I), with the proviso that in variant d) m is 1 in formula (I).

7. A herbicidal or plant growth-regulating composition, which comprises at least one compound of the formula (I) or salt thereof as claimed in any one of claims 1 to 5 and formulation auxiliaries which are customary in plant protection.

8. A method of combating weeds or of regulating the growth of plants, which comprises applying an effective quantity of at least one compound of the formula (I) or a salt thereof as claimed in any one of claims 1 to 5 to the weeds or plants, their seeds or the area on which they grow.

9. The use of a compound of the formula (I) or salt thereof as claimed in any one of claims 1 to 5 as a herbicide or plant growth regulator.

10. A compound of the formula (IV), 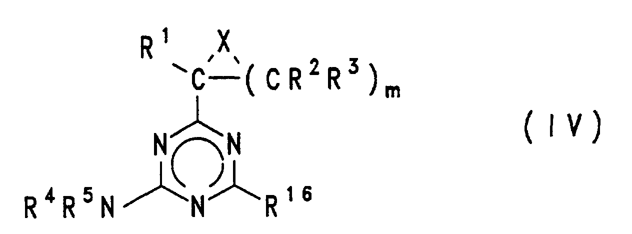 in which R¹ to R⁵, X and m are as defined for formula (I) as set forth in any one of claims 1 to 5 and
R¹⁶ is (C₁-C₄)alkylthio, (C₁-C₄)alkylsulfonyl, phenyl-(C₁-C₄)alkylthio, phenyl-(C₁-C₄)alkylsulfonyl, (C₁-C₄)alkylphenylthio or (C₁-C₄)alkylphenylsulfonyl.

## Revendications

1. Composés de formule (I) ou leurs sels, 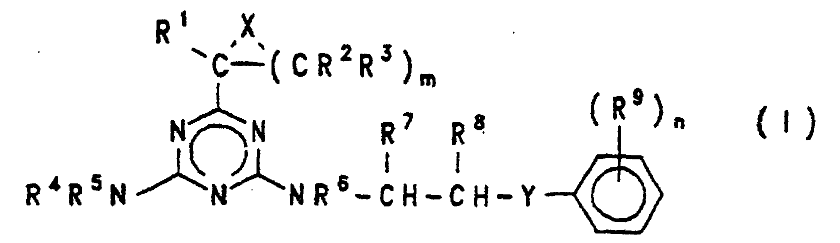 dans laquelle
R¹ représente un atome d'hydrogène, d'halogène, un groupe nitro, cyano, thiocyanato ou un groupe de formule -Z¹-R¹⁰,
R² et R³ représentent respectivement indépendamment l'un de l'autre un atome d'hydrogène, d'halogène, un groupe nitro, cyano, thiocyanato ou un groupe de formule -Z²-R¹¹,
ou R² et R³ représentent ensemble avec l'atome de carbone du groupe CR²R³ un groupe carbocyclique ayant de 4 à 10 chaînons dans le cycle ou un groupe hétérocyclique ayant de 4 à 10 chaînons dans le cycle et avec des atomes d'hétérocycle choisis parmi N, O et S, dans lequel chacun des deux derniers groupes cités est non substitué ou substitué par un ou plusieurs groupes choisis parmi un atome d'halogène, un groupe alkyle en C₁ à C₄ et oxo,
R⁴ et R⁵ représentent indépendamment respectivement un atome d'hydrogène, un groupe amino, alkylamino ou dialkylamino ayant respectivement de 1 à 6 atomes de carbone dans le radical alkyle, un groupe hydrocarboné acyclique ou cyclique ou un groupe oxyde d'hydrocarbure ayant respectivement de 1 à 10 atomes de carbone ou un groupe hétérocyclyle, un groupe hétérocyclyloxy ou hétérocyclylamino ayant respectivement de 3 à 6 atomes de cycle et de 1 à 3 atomes d'hétérocycle choisis parmi N, O ou S, dans lequel chacun des cinq derniers groupes mentionnés est non substitué ou substitué, ou un groupe acyle ou
ou R⁴ et R⁵ représentent ensemble avec l'atome d'azote du groupe NR⁴R⁵ un groupe hétérocyclique ayant de 3 à 6 atomes de cycle et de 1 à 4 atomes d'hétérocycle, dans lequel en plus de l'atome d'azote les éventuels autres atomes d'hétérocycle sont choisis parmi N, O et S et le groupe est non substitué ou substitué,
R⁶ représente un atome d'hydrogène, un groupe amino, alkylamino ou dialkylamino ayant respectivement de 1 à 6 atomes de carbone dans le radical alkyle, un groupe hydrocarboné acyclique ou cyclique ou un groupe d'oxyde d'hydrocarbure ayant respectivement de 1 à 10 atomes de carbone ou un groupe hétérocyclyle, hétérocyclyloxy ou hétérocyclylamino ayant respectivement de 3 à 6 atomes de cycle et de 1 à 3 atomes d'hétérocycle choisis parmi N, O et S, dans lequel chacun des cinq derniers groupes mentionnés est non substitué ou substitué, ou un groupe acyle,
R⁷ et R⁸ représentent indépendamment l'un de l'autre un atome d'hydrogène, d'halogène, un groupe nitro, cyano, thiocyanato ou un groupe de formule -Z³-R¹² ou
R⁷ et R⁸ forment ensemble une chaîne alkylène ayant de 2 à 4 atomes de carbone, qui est non substituée ou substituée par un ou plusieurs groupes choisis parmi un atome d'halogène, un groupe alkyle en C₁ à C₄ et oxo,
les R⁹ représentent indépendamment les uns des autres un atome d'halogène, un groupe nitro, formyle, carboxy, cyano, thiocyanato, alkyle en C₁ à C₆, alcényle en C₂ à C₆, alcynyle en C₂ à C₆, alcoxy en C₂ à C₆, alcényloxy en C₂ à C₆, alcynyloxy en C₂ à C₆, alkyle en C₁ à C₄-carbonyle, alkyloxy en C₁ à C₄-carbonyle, alcoxy en C₁ à C₄-thiocarbonyle, alkyle en C₁ à C₄-thiocarbonyle ou alkylthio en C₁ à C₄-thiocarbonyle, dans lesquels les groupes alkyle dans les 11 derniers groupes mentionnés sont non substitués ou substitués, ou alkylsulfonyle en C₁ à C₄, halogénoalkylsulfonyle en C₁ à C₄, alcoxysulfonyle en C₁ à C₄, amino, mono- ou di(alkyle en Ci à C₆)amino, aminocarbonyle, mono- ou di-(alkyle en C₁ à C₆)aminocarbonyle, alcanoylamino en C₁ à C₆, N-(alcanoyle en C₁ à C₆)-N-(alkyle en C₁ à C₄)-amino, ou un groupe de formule Z⁴-R⁰, dans laquelle Z⁴ est défini comme ci-dessous et R° représente un groupe aromatique, carbocyclique ou hétérocyclique, saturé ou partiellement saturé, dans lequel le groupe cyclique est substitué ou non substitué, ou deux groupes R⁹ voisins forment ensemble un cycle condensé ayant de 4 à 6 atomes de cycle, qui est carbocyclique ou contient des atomes d'hétérocycle choisis parmi O, S et N et qui est non substitué ou substitué par un ou plusieurs groupes choisis parmi un atome d'halogène, un groupe alkyle en C₁ à C₄ et oxo,
R¹⁰, R¹¹, R¹² représentent respectivement indépendamment les uns des autres H ou un groupe hydrocarboné acyclique ou un groupe hydrocarboné cyclique ou un groupe hétérocyclique, chacun des trois derniers groupes cités étant non substitué ou substitué,
X représente un groupe de formule -O-, -S(O)_{q}-, -NR*- ou -N(O)-, dans lesquelles q = 0, 1 ou 2 et R* est un atome d'hydrogène ou un groupe alkyle ayant de 1 à 4 atomes de carbone, ou un groupe de formule CR¹³R¹⁴, dans lequel les définitions de R¹³ et R¹⁴ sont choisies parmi les groupes possibles pour R² et R³,
Y représente une liaison directe ou un groupe de formule -O-, -S(O)ᵣ-, -NR**- ou -N(O)-, dans laquelle r = 0, 1 ou 2 et R** représente un atome d'hydrogène ou un groupe alkyle ayant de 1 à 4 atomes de carbone, ou un groupe de formule -CH₂-, -C(CH₃)H- ou -C(CH₃)₂-,
Z¹, Z², Z³, Z⁴ représentent respectivement indépendamment les uns des autres une liaison directe ou un groupe divalent de formule
-O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'-
et p est alors = 0, 1 ou 2 et R' est un atome d'hydrogène, un groupe alkyle ayant de 1 à 6 atomes de carbone, phényle, benzyle, cycloalkyle ayant de 3 à 6 atomes de carbone ou alcanoyle ayant de 1 à 6 atomes de carbone,
m représente 1, 2, 3 ou 4 et
n représente 0, 1, 2, 3, 4 ou 5.

2. Composés de formule (I) et leurs sels selon la revendication 1, **caractérisés en ce que**
R¹ représente H, un atome d'halogène, NO₂, CN, SCN ou -Z¹-R¹⁰,
R² et R³ représentent respectivement H, un atome d'halogène, NO₂, CN, SCN ou un groupe de formule - Z²-R¹¹ ou
R² et R³ représentent ensemble avec l'atome de carbone du groupe CR²R³ un groupe cycloalkyle en C₃ à C₈, cycloalcényle en C₃ à C₈ ou un groupe hétérocyclique ayant de 3 à 6 chaînons dans le cycle et avec des atomes d'hétérocycle choisis parmi N, O et S, dans lequel chacun des trois derniers groupes cités est non substitué ou substitué par un ou plusieurs groupes choisis parmi un atome d'halogène, un groupe alkyle en C₁ à C₄ et oxo,
R⁴ et R⁵ représentent indépendamment respectivement un atome d'hydrogène, un groupe amino, alkylamino ou dialkylamino ayant respectivement de 1 à 4 atomes de carbone dans le radical alkyle, un groupe hydrocarboné acyclique ou cyclique ou un groupe oxyde d'hydrocarbure ayant respectivement de 1 à 6 atomes de carbone ou un groupe hétérocyclyle, un groupe hétérocyclyloxy ou un groupe hétérocyclylamino ayant respectivement de 3 à 6 atomes de cycle et de 1 à 3 atomes d'hétérocycle choisis parmi N, O ou S, dans lequel chacun des cinq derniers groupes mentionnés est non substitué ou substitué par un ou plusieurs groupes choisis parmi un atome d'halogène, un groupe alcoxy en C₁ à C₄, halogénoalcoxy en C₁ à C₄, alkylthio en C₁ à C₄, alcényle en C₂ à C₄, alcynyle en C₂ à C₄, alcényloxy en C₂ à C₄, alcynyloxy en C₂ à C₄, hydroxy, amino, acylamino, mono- et dialkyl-amino, nitro, carboxy, cyano, azido, (alcoxy en C₁ à C₄)-carbonyle, (alkyle en C₁ à C₄)-carbonyle, formyle, carbamoyle, mono- et di-(alkyle en C₁ à C₄)-aminocarbonyle, alkylsulfinyle en C₁ à C₄, halogénoalkylsulfinyle en C₁ à C₄, alkylsulfonyle en C₁ à C₄, halogénoalkylsulfonyle en C₁ à C₄ et, dans le cas de groupes cycliques, aussi un groupe alkyle en C₁ à C₄ et halogénoalkyle en C₁ à C₄, ou un groupe acyle ou
R⁴ et R⁵ représentent ensemble avec l'atome d'azote du groupe NR⁴R⁵ un groupe hétérocyclique ayant de 3 à 6 atomes de cycle et de 1 à 2 atomes d'hétérocycle, dans lequel en plus de l'atome d'azote les éventuels autres atomes d'hétérocycle sont choisis parmi N, O et S et le groupe est non substitué ou substitué par un ou plusieurs groupes choisis parmi un atome d'halogène, un groupe alkyle en C₁ à C₄ et oxo,
R⁶ représente un atome d'hydrogène, un groupe amino, alkylamino ou dialkylamino ayant respectivement de 1 à 6 atomes de carbone dans le radical alkyle, un groupe hydrocarboné acyclique ou cyclique ou un groupe d'oxyde d'hydrocarbure ayant respectivement de 1 à 6 atomes de carbone ou un groupe hétérocyclyle, un groupe hétérocyclyloxy ou un groupe hétérocyclylamino ayant respectivement de 3 à 6 atomes de cycle et de 1 à 3 atomes d'hétérocycle choisis parmi N, O et S, dans lequel chacun des cinq derniers groupes mentionnés est non substitué ou substitué par un ou plusieurs groupes choisis parmi un atome d'halogène, un groupe alcoxy en C₁ à C₄, halogénoalcoxy en C₁ à C₄, alkylthio en C₁ à C₄, alcényle en C₂ à C₄, alcynyle en C₂ à C₄, alcényloxy en C₂ à C₄, alcynyloxy en C₂ à C₄, hydroxy, amino, acylamino, mono- et dialkylamino, nitro, carboxy, cyano, azido, (alcoxy en C₁ à C₄)-carbonyle, (alkyle en C₁ à C₄)-carbonyle, formyle, carbamoyle, mono- et di-(alkyle en C₁ à C₄)-aminocarbonyle, alkylsulfinyle en C₁ à C₄, halogénoalkylsulfinyle en C₁ à C₄, alkylsulfonyle en C₁ à C₄, halogéno-alkylsulfonyle en C₁ à C₄ et, dans le cas de groupes cycliques, aussi un groupe alkyle en C₁ à C₄ et halogénoalkyle en C₁ à C₄,
ou un groupe acyle,
R⁷ et R ⁸ représentent indépendamment l'un de l'autre H, un atome d'halogène, NO₂, CN, SCN ou un groupe de formule -Z³-R¹² ou
R⁷ et R⁸ représentent ensemble une chaîne alkylène ayant de 2 à 4 atomes de carbone, qui est non substituée ou substituée par un ou plusieurs groupes choisis parmi un atome d'halogène, un groupe alkyle en C₁ à C₄ et oxo,
les R⁹ représentent indépendamment les uns des autres respectivement un atome d'halogène, un groupe nitro, formyle, carboxy, cyano, thiocyanato, alkyle en C₁ à C₆, alcoxy en C₁ à C₆, alkylcarbonyle en C₁ à C₄, alkyloxycarbonyle en C₁ à C₄ ou alkyle en C₁ à C₄-thiocarbonyle dans lesquels les cinq derniers groupes alkyle mentionnés sont non substitués ou substitués par un atome d'halogène ou un groupe alcoxy en C₁ à C₄, ou alkylsulfonyle en C₁ à C₄, halogénoalkylsulfonyle en C₁ à C₄, mono- ou di-(alkyle en C₁ à C₄)amino, aminocarbonyle, mono- ou di-(alkyle en C₁ à C₄)aminocarbonyle, alcanoylamino en C₁ à C₄ ou un groupe de formule Z⁴-R⁰, dans laquelle Z⁴ est défini comme ci-dessous et R° représente un groupe cycloalkyle en C₃ à C₆, cycloalcényle en C₃ à C₆ ou phényle, dans lequel chacun des trois derniers groupes cités est non substitué ou substitué par des groupes choisis parmi un atome d'halogène, un groupe alkyle en C₁ à C₄, halogénoalkyle en C₁ à C₄ et alcoxy en C₁ à C₄, ou
deux groupes R⁹ voisins forment ensemble un cycle condensé ayant de 4 à 6 atomes de cycle, qui est carbocyclique ou contient des atomes d'hétérocycle choisis parmi 0, S et N et qui est non substitué ou substitué par un ou plusieurs groupes choisis parmi un atome d'halogène, un groupe alkyle en C₁ à C₄ et oxo,
R¹⁰, R¹¹, R¹² représentent respectivement indépendamment les uns des autres H ou un groupe hydrocarboné acyclique ayant de 1 à 6 atomes de carbone ou un groupe hydrocarboné cyclique, un groupe hydrocarboné cyclique ayant de 3 à 6 atomes de carbone ou un groupe hétérocyclique ayant de 3 à 6 atomes de cycle et de 1 à 3 atomes d'hétérocycle choisis parmi N, O et S, chacun des trois derniers groupes cités étant non substitué ou substitué par un ou plusieurs groupes choisis parmi un atome d'halogène, un groupe alcoxy en C₁ à C₄, halogénoalcoxy en C₁ à C₄, alkylthio en C₁ à C₄, alcényle en C₂ à C₄, alcynyle en C₂ à C₄, alcényloxy en C₂ à C₄, alcynyloxy en C₂ à C₄, hydroxy, amino, acylamino, mono- et dialkylamino, nitro, carboxy, cyano, azido, (alcoxy en C₁ à C₄)-carbonyle, (alkyle en C₁ à C₄)-carbonyle, formyle, carbamoyle, mono- et di-(alkyle en C₁ à C₄)-aminocarbonyle, alkylsulfinyle en C₁ à C₄, halogénoalkylsulfinyle en C₁ à C₄, alkylsulfonyle en C₁ à C₄, halogénoalkylsulfonyle en C₁ à C₄ et, dans le cas de groupes cycliques, aussi un groupe alkyle en C₁ à C₄ et halogénoalkyle en C₁ à C₄,
X représente un groupe de formule -O-, -S(O)_{q}-, -NR*-ou -N(O)-, dans lequel q = 0, 1 ou 2 et R* est un atome d'hydrogène ou un groupe alkyle ayant de 1 à 4 atomes de carbone, ou un groupe de formule CR¹³R¹⁴, dans lequel les définitions de R¹³ et R¹⁴ sont choisies parmi les groupes possibles pour R² et R³,
Y représente une liaison directe ou un groupe de formule -O-, -S(O)ᵣ-, -NR**- ou -N(O)-, dans lesquelles r = 0, 1 ou 2 et R** représente un atome d'hydrogène ou un groupe alkyle ayant de 1 à 4 atomes de carbone, ou un groupe de formule -CH₂-, -C(CH₃)H- ou -C(CH₃)₂-,
Z¹, Z², Z³, Z⁴ représentent respectivement indépendamment les uns des autres une liaison directe ou un groupe divalent de formule
-O-, -S-, -CO-, -O-CO-, -CO-O, -NR'-, -NR'-CO-, ou
-CO-NR'-, dans laquelle R' est H ou un groupe alkyle en C₁ à C₄.

3. Composés de formule (I) et leurs sels selon la revendication 1 ou 2, **caractérisés en ce que**
R¹, R² et R³ représentent indépendamment les uns des autres un atome d'hydrogène, d'halogène, un groupe hydroxy, amino, nitro, formyle, carboxy, cyano, thiocyanato, alkyle en C₁ à C₄, cyano-alkyle en C₁ à C₄, alcoxy en C₁ à C₄, alkylamino en C₁ à C₄, di-(alkyle en C₁ à C₄)-amino, halogéno-alkyle en C₁ à C₄, hydroxy-alkyle en C₁ à C₄, alcoxy en C₁ à C₄-alkyle en C₁ à C₄, halogénoalcoxy en C₁ à C₄-alkyle en C₁ à C₄, alkylthio en C₁ à C₄, halogénoalkylthio en C₁ à C₄, alcényle en C₂ à C₆, halogénoalcényle en C₂ à C₆, alcynyle en C₂ à C₆, halogénoalcynyle en C₂ à C₆, alkylamino en C₁ à C₄-alkyle en C₁ à C₄, di-(alkyle en C₁ à C₄)-amino-alkyle en C₁ à C₄, cycloalkylamino en C₃ à C₉-alkyle en C₁ à C₄, cycloalkyle en C₃ à C₉, hétérocyclyl-alkyle en C₁ à C₄ avec de 3 à 9 chaînons de cycle, dans lesquelles les groupes cycliques dans les trois derniers groupes cités sont non substitués ou substitués par un ou plusieurs groupes choisis parmi un groupe alkyle en C₁ à C₄, un atome d'halogène et un groupe cyano, ou phényle, phénoxy, phénylcarbonyle, phénylcarbonyl-alkyle en C₁ à C₄, alcoxy en C₁ à C₄-carbonyl-alkyle en C₁ à C₄, alkylamino en C₁ à C₄-carbonyl-alkyle en C₁ à C₄, alkyle en C₁ à C₄-carbonyle, alcoxy en C₁ à C₄-carbonyle, aminocarbonyle, alkylamino en C₁ à C₄-carbonyle, phénoxy-alkyle en C₁ à C₄, phényl-alkyle en C₁ à C₄, hétérocyclyle, hétérocyclylamino, hétérocyclyloxy, hétérocyclylthio, ou un des 16 derniers groupes cités, qui est substitué dans la partie acyclique ou dans la partie cyclique par un ou plusieurs groupes choisis parmi un atome d'halogène, un groupe nitro, cyano, alkyle en C₁ à C₄, alcoxy en C₁ à C₄, alkylthio en C₁ à C₄, halogénoalkyle en C₁ à C₄, halogénoalcoxy en C₁ à C₄, formyle, alkyle en C₁ à C₄-carbonyle, alcoxy en C₁ à C₄-carbonyle, alcoxy en C₁ à C₄, dans lesquelles le noyau hétérocyclyle contient dans les groupes respectivement 3 à 9 atomes de cycle et de 1 à 3 atomes d'hétérocycle choisis parmi N, O, S,
R² et R³ représentent ensemble avec l'atome de carbone du groupe CR²R³ un groupe carbocyclique saturé ou partiellement insaturé ayant de 3 à 6 chaînons dans le cycle ou hétérocyclyle ayant de 3 à 6 chaînons dans le cycle et de 1 à 3 atomes d'hétérocycle choisis parmi N, O et S, dans lequel chacun des deux derniers groupes cités est non substitué ou substitué par un ou plusieurs groupes choisis parmi un groupe alkyle en C₁ à C₄, un atome d'halogène, et un groupe oxo,
R⁴ et R⁵ représentent indépendamment l'un de l'autre un atome d'hydrogène, un groupe amino, formyle, alkyle en C₁ à C₄, cyano-alkyle en C₁ à C₄, alkylamino en C₁ à C₄, di-(alkyle en C₁ à C₄)-amino, halogéno-alkyle en C₁ à C₄, hydroxy-alkyle en C₁ à C₄, alcoxy en C₁ à C₄-alkyle en C₁ à C₄, halogénoalcoxy en C₁ à C₄-alkyle en C₁ à C₄, alcényle en C₂ à C₆, halogénoalcényle en C₂ à C₆, alcynyle en C₂ à C₆, halogénoalcynyle en C₂ à C₆, alkylamino en C₁ à C₄-alkyle en C₁ à C₄, di-(alkyle en C₁ à C₄)-amino-alkyle en C₁ à C₄, cycloalkylamino en C₃ à C₉-alkyle en C₁ à C₄, cycloalkyle en C₃ à C₉, hétérocyclyle en C₃ à C₉-alkyle en C₁ à C₄ avec de 3 à 9 chaînons de cycle, dans lesquelles les groupes cycliques dans les trois derniers groupes cités sont non substitués ou substitués par un ou plusieurs groupes choisis parmi un groupe alkyle en C₁ à C₄, un atome d'halogène et un groupe cyano, ou phényle, phénoxy, phénylcarbonyle, phénylcarbonyl-alkyle en C₁ à C₄, alcoxy en C₁ à C₄-carbonyl-alkyle en C₁ à C₄, alkylamino en C₁ à C₄-carbonyl-alkyle en C₁ à C₄, alkyle en C₁ à C₄-carbonyle, alcoxy en C₁ à C₄-carbonyle, aminocarbonyle, alkylamino en C₁ à C₄-carbonyle, phénoxy-alkyle en C₁ à C₄, phényl-alkyle en C₁ à C₄, hétérocyclyle, hétérocyclylamino, hétérocyclyloxy, hétérocyclylthio, ou un des 16 derniers groupes cités, qui est substitué dans la partie acyclique ou dans la partie cyclique par un ou plusieurs groupes choisis parmi un atome d'halogène, un groupe nitro, cyano, alkyle en C₁ à C₄, alcoxy en C₁ à C₄, alkylthio en C₁ à C₄, halogénoalkyle en C₁ à C₄, halogénoalcoxy en C₁ à C₄, formyle, alkyle en C₁ à C₄-carbonyle, alcoxy en C₁ à C₄-carbonyle, alcoxy en C₁ à C₄, dans lesquelles le noyau hétérocyclyle contient dans les groupes respectivement 3 à 9 atomes de cycle et de 1 à 3 atomes d'hétérocycle choisis parmi N, O et S, ou
R⁴ et R⁵ représentent ensemble avec l'atome d'azote du groupe NR⁴R⁵ un groupe hétérocyclique ayant de 3 à 6 atomes de cycle et de 1 à 2 atomes d'hétérocycle, dans lequel en plus de l'atome d'azote les éventuels autres atomes d'hétérocycle sont choisis parmi N, O et S et le groupe est non substitué ou substitué par un ou plusieurs groupes choisis parmi un atome d'halogène, un groupe alkyle en C₁ à C₄ et oxo,
R⁶ représente un atome d'hydrogène, un groupe amino, formyle, alkyle en C₁ à C₄, cyano-alkyle en C₁ à C₄, alkylamino en C₁ à C₄, di-(alkyle en C₁ à C₄)-amino, halogéno-alkyle en C₁ à C₄, hydroxy-alkyle en C₁ à C₄, alcoxy en C₁ à C₄-alkyle en C₁ à C₄, halogéno-alcoxy en C₁ à C₄-alkyle en C₁ à C₄, alcényle en C₂ à C₆, halogénoalcényle en C₂ à C₆, alcynyle en C₂ à C₆, halogénoalcynyle en C₂ à C₆, alkylamino en C₁ à C₄-alkyle en C₁ à C₄, di-(alkyle en C₁ à C₄)-amino-alkyle en C₁ à C₄, cycloalkylamino en C₃ à C₉-alkyle en C₁ à C₄, cycloalkyle en C₃ à C₉, hétérocyclyle en C₃ à C₉-alkyle en C₁ à C₄ avec de 3 à 9 chaînons de cycle, dans lesquelles les groupes cycliques dans les trois derniers groupes cités sont non substitués ou substitués par un ou plusieurs groupes choisis parmi un groupe alkyle en C₁ à C₄, un atome d'halogène et un groupe cyano, ou phényle, phénoxy, phénylcarbonyle, phénylcarbonyl-alkyle en C₁ à C₄, alcoxy en C₁ à C₄-carbonyl-alkyle en C₁ à C₄, alkylamino en C₁ à C₄-carbonyl-alkyle en C₁ à C₄, alkyle en C₁ à C₄-carbonyle, alcoxy en C₁ à C₄-carbonyle, aminocarbonyle, alkylamino en C₁ à C₄-carbonyle, phénoxy-alkyle en C₁ à C₄, phényl-alkyle en C₁ à C₄, hétérocyclyle, hétérocyclylamino, hétérocyclyloxy, hétérocyclylthio, ou un des 16 derniers groupes cités, qui est substitué dans la partie acyclique ou dans la partie cyclique par un ou plusieurs groupes choisis parmi un atome d'halogène, un groupe nitro, cyano, alkyle en C₁ à C₄, alcoxy en C₁ à C₄, alkylthio en C₁ à C₄, halogénoalkyle en C₁ à C₄, halogénoalcoxy en C₁ à C₄, formyle, alkyle en C₁ à C₄-carbonyle, alcoxy en C₁ à C₄-carbonyle, alcoxy en C₁ à C₄, dans lesquelles le noyau hétérocyclyle contient dans les groupes respectivement 3 à 9 atomes de cycle et de 1 à 3 atomes d'hétérocycle choisis parmi N, O et S,
R⁷ et R⁸ représentent indépendamment l'un de l'autre un atome d'hydrogène, d'halogène, un groupe hydroxy, amino, nitro, formyle, carboxy, cyano, thiocyanato, alkyle en C₁ à C₄, cyano-alkyle en C₁ à C₄, alcoxy en C₁ à C₄, alkylamino en C₁ à C₄, di-(alkyle en C₁ à C₄)-amino, halogénoalkyle en C₁ à C₄, hydroxy-alkyle en C₁ à C₄, alcoxy en C₁ à C₄-alkyle en C₁ à C₄, halogénoalcoxy en C₁ à C₄-alkyle en C₁ à C₄, alkylthio en C₁ à C₄, halogénoalkylthio en C₁ à C₄, alcényle en C₂ à C₆, halogénoalcényle en C₂ à C₆, alcynyle en C₂ à C₆, halogénoalcynyle en C₂ à C₆, alkylamino en C₁ à C₄-alkyle en C₁ à C₄, di-(alkyle en C₁ à C₄)-amino-alkyle en C₁ à C₄, cycloalkylamino en C₃ à C₉-alkyle en C₁ à C₄, cycloalkyle en C₃ à C₉, hétérocyclyl-alkyle en C₁ à C₄ ayant de 3 à 9 chaînons dans le cycle, dans lequel les groupes cycliques dans les trois derniers groupes cités sont non substitués ou substitués par un ou plusieurs groupes choisis parmi un groupe alkyle en C₁ à C₄, un atome d'halogène et un groupe cyano, ou phényle, phénoxy, phénylcarbonyle, phénylcarbonyl-alkyle en C₁ à C₄, alcoxy en C₁ à C₄-carbonyl-alkyle en C₁ à C₄, alkylamino en C₁ à C₄-carbonyl-alkyle en C₁ à C₄, alkyle en C₁ à C₄-carbonyle, alcoxy en C₁ à C₄-carbonyle, aminocarbonyle, alkylamino en C₁ à C₄-carbonyle, phénoxy-alkyle en C₁ à C₄, phényl-alkyle en C₁ à C₄, hétérocyclyle, hétérocyclylamino, hétérocyclyoxy, hétérocyclylthio, ou un des 16 derniers groupes cités, qui est substitué dans la partie acyclique ou dans la partie cyclique par un ou plusieurs groupes choisis parmi un atome d'halogène, un groupe nitro, cyano, alkyle en C₁ à C₄, alcoxy en C₁ à C₄, alkylthio en C₁ à C₄, halogénoalkyle en C₁ à C₄, halogénoalcoxy en C₁ à C₄, formyle, alkyle en C₁ à C₄-carbonyle, alcoxy en C₁ à C₄-carbonyle, alcoxy en C₁ à C₄, dans lesquelles le noyau hétérocyclyle contient dans les groupes respectivement 3 à 9 atomes de cycle et de 1 à 3 atomes d'hétérocycle choisis parmi N, O et S, ou
R⁷ et R⁸ forment ensemble une chaîne alkylène ayant de 2 à 4 atomes de carbone, qui est non substituée ou substituée par un ou plusieurs groupes choisis parmi un atome d'halogène, un groupe alkyle en C₁ à C₄ et oxo,
les R⁹, lorsque n = 1, et le groupes R⁹, respectivement indépendant les uns des autres, lorsque n est supérieur à 1, représentent un atome d'hydrogène, d'halogène, un groupe hydroxy, amino, nitro, formyle, carboxy, cyano, thiocyanato, alkyle en C₁ à C₄, cyano-alkyle en C₁ à C₄, alcoxy en C₁ à C₄, alkylamino en C₁ à C₄, di(alkyle en C₁ à C₄)-amino, halogéno-alkyle en C₁ à C₄, hydroxy-alkyle en C₁ à C₄, alcoxy en C₁ à C₄-alkyle en C₁ à C₄, halogénoalcoxy en C₁ à C₄-alkyle en C₁ à C₄, alkylthio en C₁ à C₄, halogéno-alkylthio en C₁ à C₄, alcényle en C₂ à C₆, halogénoalcényle en C₂ à C₆, alcynyle en C₂ à C₆, halogénoalcynyle en C₂ à C₆, alkylamino en C₁ à C₄-alkyle en C₁ à C₄, di(alkyle en C₁ à C₄)-amino-alkyle en C₁ à C₄, cycloalkylamino en C₃ à C₉-alkyle en C₁ à C₄, cycloalkyle en C₃ à C₉, hétérocyclyl-alkyle en C₁ à C₄ avec de 3 à 9 chaînons de cycle, dans lesquelles les groupes cycliques dans les trois derniers groupes cités sont non substitués ou substitués par un ou plusieurs groupes choisis parmi un groupe alkyle en C₁ à C₄, un atome d'halogène et un groupe cyano, ou phényle, phénoxy, phénylcarbonyle, phénylcarbonyl-alkyle en C₁ à C₄, alcoxy en C₁ à C₄-carbonyl-alkyle en C₁ à C₄, alkylamino en C₁ à C₄-carbonyl-alkyle en C₁ à C₄, alkyle en C₁ à C₄-carbonyle, alcoxy en C₁ à C₄-carbonyle, aminocarbonyle, alkylamino en C₁ à C₄-carbonyle, phénoxy-alkyle en C₁ à C₄, phényl-alkyle en C₁ à C₄, hétérocyclyle, hétérocyclylamino, hétérocyclyloxy, hétérocyclylthio, ou un des 16 derniers groupes cités, qui est substitué dans la partie acyclique ou dans la partie cyclique par un ou plusieurs groupes choisis parmi un atome d'halogène, un groupe nitro, cyano, alkyle en C₁ à C₄, alcoxy en C₁ à C₄, alkylthio en C₁ à C₄, halogénoalkyle en C₁ à C₄, halogénoalcoxy en C₁ à C₄, formyle, alkyle en C₁ à C₄-carbonyle, alcoxy en C₁ à C₄-carbonyle, alcoxy en C₁ à C₄, dans lesquelles le noyau hétérocyclyle contient dans les groupes respectivement 3 à 9 atomes de cycle et de 1 à 3 atomes d'hétérocycle choisis parmi N, O et S, ou
deux groupes R⁹ voisins forment ensemble un cycle condensé ayant de 4 à 6 atomes de cycle, qui est carbocyclique ou contient des atomes d'hétérocycle choisis parmi O, S et N et qui est non substitué ou substitué par un ou plusieurs groupes choisis parmi un atome d'halogène, un groupe alkyle en C₁ à C₄ et oxo,
X représente un groupe de formule -O-, -S-, -NR*-,
dans lequel R* est un atome d'hydrogène ou
un groupe de formule CR¹³R¹⁴, dans lequel les définitions de R¹³ et R¹⁴ sont choisies parmi les groupes possibles pour R² et R³,
Y représente une liaison directe ou un groupe de formule -O-, -S- ou -NR**-, dans lequel R** représente un atome d'hydrogène ou un groupe alkyle ayant de 1 à 4 atomes de carbone, ou un groupe de formule -CH₂-, -C(CH₃)H- ou -C(CH₃)₂-.

4. Composés de formule (I) et leurs sels selon l'une des revendications 1 à 3, **caractérisés en ce que**
R¹ représente un atome d'hydrogène, d'halogène, un groupe hydroxy, amino, alkyle en C₁ à C₄, alcoxy en C₁ à C₄, alkylamino en C₁ à C₄, di-(alkyle en C₁ à C₄)-amino, halogéno-alkyle en C₁ à C₄, alcoxy en C₁ à C₄-alkyle en C₁ à C₄, alcényle en C₂ à C₆, àlcynyle en C₂ à C₆, phényle, phénoxy ou un groupe phényle ou phénoxy qui est substitué dans la partie phényle par un ou plusieurs groupes choisis parmi un atome d'halogène, un groupe alkyle en C₁ à C₄, alcoxy en C₁ à C₄ et alcoxy en C₁ à C₄-carbonyle,
R² et R³ représentent indépendamment l'un de l'autre un atome d'hydrogène, d'halogène, un groupe hydroxy, amino, alkyle en C₁ à C₄, alcoxy en C₁ à C₄, alkylamino en C₁ à C₄, di-(alkyle en C₁ à C₄)-amino, halogéno-alkyle en C₁ à C₄, alcoxy en C₁ à C₄-alkyle en C₁ à C₄, alcényle en C₂ à C₆, alcynyle en C₂ à C₆, phényle ou phénoxy ou un groupe phényle ou phénoxy qui est substitué dans la partie phényle par un ou plusieurs groupes choisis parmi un atome d'halogène, un groupe alkyle en C₁ à C₄, alcoxy en C₁ à C₄ et alcoxy en C₁ à C₄-carbonyle, ou
R² et R³ forment ensemble avec l'atome de carbone du groupe CR²R³ un groupe carbocyclique saturé ayant de 3 à 6 chaînons dans le cycle, qui est non substitué ou substitué par un ou plusieurs groupes choisis parmi un atome d'halogène, un groupe alkyle en C₁ à C₄ et oxo,
R⁴ et R⁵ représentent indépendamment l'un de l'autre un atome d'hydrogène, un groupe amino, formyle, alkyle en C₁ à C₄, alcoxy en C₁ à C₄-alkyle en C₁ à C₄, alcényle en C₂ à C₆, alcynyle en C₂ à C₆, alkylamino en C₁ à C₄-alkyle en C₁ à C₄, di(alkyle en C₁ à C₄)-amino-alkyle en C₁ à C₄ ou phényle, phényl-alkyle en C₁ à C₄ ou phénoxy-carbonyle ou un des trois derniers groupes cités, qui est substitué jusqu'à trois fois dans la partie phényle par des groupes choisis parmi un atome d'halogène, un groupe alkyle en C₁ à C₄, alcoxy en C₁ à C₄ et alcoxy en C₁ à C₄-carbonyle, ou
R⁴ et R⁵ forment ensemble avec l'atome d'azote du groupe NR⁴R⁵ un groupe hétérocyclique ayant de 3 à 6 atomes de cycle et de 1 à 2 atomes d'hétérocycle, dans lequel en plus de l'atome d'azote les éventuels autres atomes d'hétérocycle sont choisis parmi N et O et le groupe est non substitué ou substitué par un ou plusieurs groupes choisis parmi un atome d'halogène, un groupe alkyle en C₁ à C₄ et oxo,
R⁶ représente un atome d'hydrogène, un groupe amino, formyle, alkyle en C₁ à C₄, di-(alkyle en C₁ à C₄)-amino, alcoxy en C₁ à C₄-alkyle en C₁ à C₄, alcényle en C₂ à C₆, alcynyle en C₂ à C₆, dialkylamino en C₁ à C₄-alkyle en C₁ à C₄, phényle, phénoxy-alkyle en C₁ à C₄, phényle-alkyle en C₁ à C₄, phénoxy-carbonyle, phénylamino-carbonyle ou un des cinq derniers groupes cités, qui est substitué une fois jusqu'à trois fois par des groupes choisis parmi un atome d'halogène, un groupe alkyle en C₁ à C₄, alcoxy en C₁ à C₄ et alcoxy en C₁ à C₄-carbonyle.
R⁷ et R⁸ représentent indépendamment l'un de l'autre un atome d'hydrogène, un groupe alkyle en C₁ à C₄, alcoxy en C₁ à C₄-alkyle en C₁ à C₄, alcényle en C₂ à C₆, alcynyle en C₂ à C₆, dialkylamino en C₁ à C₄-alkyle en C₁ à C₄, phényle, phénoxy-alkyle en C₁ à C₄, phényl-alkyle en C₁ à C₄ ou un des trois - derniers groupes cités, qui est substitué une fois jusqu'à trois fois par des groupes choisis parmi un atome d'halogène, un groupe alkyle en C₁ à C₄, alcoxy en C₁ à C₄ et alcoxy en C₁ à C₄-carbonyle,
ou
R⁷ et R⁸ forment ensemble une chaîne alkylène ayant de 2 à 4 atomes de carbone, qui est non substituée ou substituée par un ou plusieurs groupes choisis parmi un atome d'halogène, un groupe alkyle en C₁ à C₄ et oxo,
les R⁹ représentent indépendamment les uns des autres un atome d'hydrogène, d'halogène, un groupe hydroxy, amino, nitro, cyano, alkyle en C₁ à C₄, alcoxy en C₁ à C₄, alkylamino en C₁ à C₄, di(alkyle en C₁ à C₄)-amino, perhalogénoalkyle en C₁ à C₄, hydroxy-alkyle en C₁ à C₄, alcoxy en C₁ à C₄-alkyle en C₁ à C₄, alkylthio en C₁ à C₄, alcényle en C₂ à C₆, alcynyle en C₂ à C₆, di(alkyle en C₁ à C₄)-amino-alkyle en C₁ à C₄, phényle, phénoxy, phénylcarbonyle, phénylcarbonyl-alkyle en C₁ à C₄, alcoxy en C₁ à C₄-carbonyl-alkyle en C₁ à C₄, alkyle en C₁ à C₄-carbonyle, alkyloxy en C₁ à C₄-carbonyle, phénoxy-alkyle en C₁ à C₄, phényl-alkyle en C₁ à C₄, hétérocyclyle, hétérocyclylamino, hétérocyclyoxy, hétérocyclythio ou un des 13 derniers groupes cités, qui est substitué dans la partie acyclique ou, de préférence, dans la partie cyclique par un ou plusieurs groupes choisis parmi un atome d'halogène, un groupe alkyle en C₁ à C₄, alcoxy en C₁ à C₄, alkylthio en C₁ à C₄, halogénoalkyle en C₁ à C₄, halogénoalcoxy en C₁ à C₄ et alcoxy en C₁ à C₄-carbonyle, dans lequel hétérocyclyle présente dans les groupes de 3 à 6 atomes de cycle et de 1 à 3 atomes d'hétérocycle choisis parmi N et O, ou deux groupes R⁹ voisins forment ensemble un cycle condensé ayant de 4 à 6 atomes de cycle, qui est carbocyclique ou contient des hétéroatomes choisis parmi O et N et qui est non substitué ou substitué par un ou plusieurs groupes alkyle en C₁ à C₄,
X représente un groupe de formule -O- ou -NR*-, dans lequel R* est un atome d'hydrogène ou un groupe méthyle, ou
un groupe de formule CR¹³R¹⁴, dans lequel les définitions pour R¹³ et R¹⁴ sont choisies parmi les groupes possibles pour R² et R³,
Y représente une liaison directe ou un groupe de formule -O- ou -NR**-, dans laquelle R** est un atome d'hydrogène ou un groupe méthyle.

5. Composés de formule (I) et leurs sels selon l'une des revendications 1 à 4, **caractérisés en ce que**
R¹ représente un atome d'hydrogène, d'halogène ou un groupe alkyle en C₁ à C₄,
R² et R³ représentent indépendamment l'un de l'autre un atome d'hydrogène, d'halogène, un groupe alkyle en C₁ à C₄ ou phényle ou
R² et R³ forment ensemble avec l'atome de carbone du groupe CR²R³ un groupe cycloalkyle saturé en C₄ à C₆,
R⁴ et R⁵ représentent indépendamment l'un de l'autre un atome d'hydrogène, un groupe amino, formyle ou alkyle en C₁ à C₄ ou
R⁴ et R⁵ forment ensemble avec l'atome d'azote du groupe NR⁴R⁵ un groupe hétérocyclique ayant de 4 à 6 atomes de cycle et de 1 à 2 atomes d'hétérocycle, dans lequel en plus de l'atome d'azote l'autre atome d'hétérocycle éventuel est choisi parmi N et O,
R⁶ représente un atome d'hydrogène ou un groupe alkyle en C₁ à C₄,
R⁷ et R⁸ représentent indépendamment l'un de l'autre un atome d'hydrogène, un groupe alkyle en C₁ à C₄ ou alcoxy en C₁ à C₄-alkyle en C₁ à C₄ ou
R⁷ et R⁸ forment ensemble une chaîne alkylène ayant de 2 à 4 atomes de carbone,
les R⁹ représentent indépendamment les uns des autres un atome d'hydrogène, d'halogène, un groupe hydroxy, alkyle en C₁ à C₄, ou alcoxy en C₁ à C₄,
m est 1 ou 2,
n est 2 ou 3,
X représente un groupe de formule -O- ou -NH- ou
un groupe de formule CR¹³R¹⁴, dans lequel les définitions pour R¹³ et R¹⁴ sont choisies parmi les groupes possibles pour R² et R³,
Y représente une liaison directe ou un groupe de formule -O- ou -NH-.

6. Procédé pour la préparation de composés de formule (I) ou de leurs sels selon la revendication 1, **caractérisé en ce qu'**on met à réagir
a) un composé de formule (II), 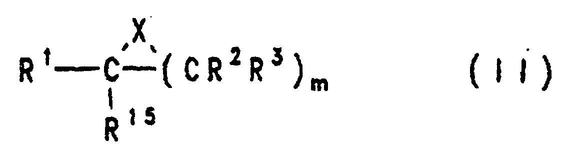 dans laquelle R¹⁵ représente un groupe fonctionnel choisi parmi un ester d'acide carboxylique, un orthoester d'acide carboxylique, un chlorure d'acide carboxylique, un carboxamide, un anhydride d'acide carboxylique et le trichlorométhyle, avec un biguanidide de formule (III) ou un sel d'addition de celui-ci avec un acide 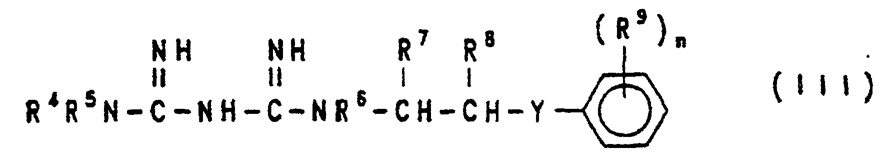 ou on met à réagir
b) un composé de formule (IV), 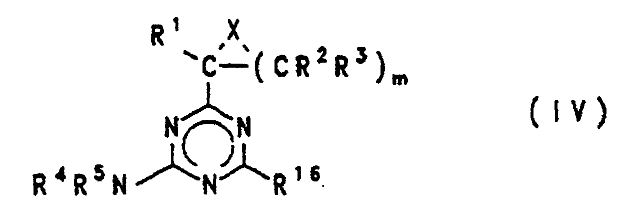 dans laquelle R¹⁶ représente un groupe capable de s'échanger ou un groupe partant, avec une amine appropriée de formule (V) ou un sel d'addition de celle-ci avec un acide 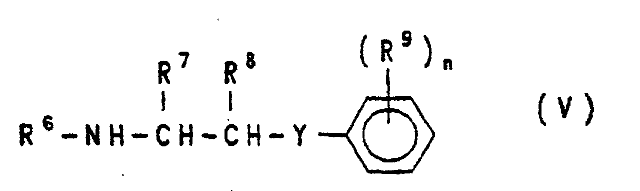 ou
c) par réaction d'un groupe capable de s'échanger sur la triazine des composés de la formule mentionnée (IV) ou de formule (X), 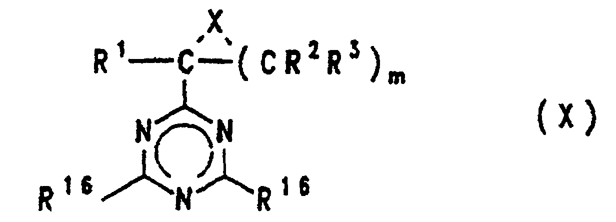 dans laquelle chacun des groupes R¹⁶ représente indépendamment l'un de l'autre un groupe capable de s'échanger ou un groupe partant, on met à réagir ce composé avec une aziridine appropriée de formule (XI), 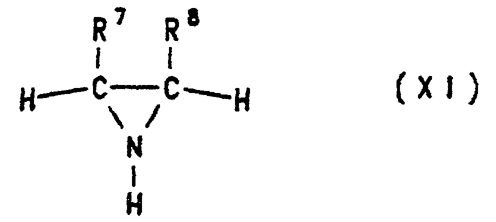 dans laquelle R⁷ et R⁸ sont définis comme à la formule (I), et ensuite avec un nucléophile approprié de formule (XII) 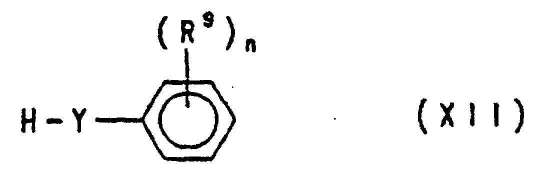 par ouverture de cycle du noyau aziridine et, au cas où on a utilisé une triazine de formule (X), par réaction du groupe capable de s'échanger encore présent sur le squelette de base triazine avec un composé de formule NHR⁴R⁵ ou
d) on met à réagir un composé de formule (XIII) 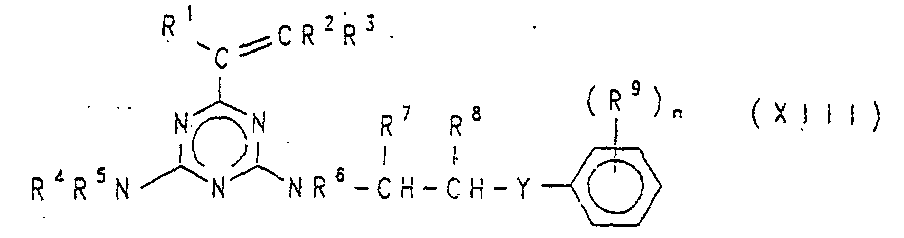 par époxylation ou cyclopropanation pour obtenir les composés correspondants de formule (I),
dans lesquels dans les formules (II), (III), (IV), (V), (X), (XI), (XII) et (XIII) les groupes R¹, R², R³, R⁴, R⁵, R⁶, R⁷, R⁸, R⁹ ainsi que X, Y, m et n sont définis comme à la formule (I), avec la condition que dans la variante d) m soit égal à 1 pour la formule (I).

7. Agent herbicide ou régulateur de croissance des plantes, **caractérisé en ce qu'**il contient au moins un composé de formule (I) ou un sel de celui-ci selon l'une des revendications 1 à 5 et des adjuvants de formulation usuels dans la protection des plantes.

8. Procédé de lutte contre les plantes nuisibles ou de régulation de croissance de plantes, **caractérisé en ce qu'**on applique une quantité efficace d'au moins un composé de formule (I) ou d'un sel de celui-ci selon l'une des revendications 1 à 5 sur les plantes nuisibles ou les plantes, leurs semences ou la surface, sur laquelle elles poussent.

9. Utilisation des composés de formule (I) ou de leurs sels selon l'une des revendications 1 à 5 comme herbicides ou régulateurs de croissance des plantes.

10. Composés de formule (IV), 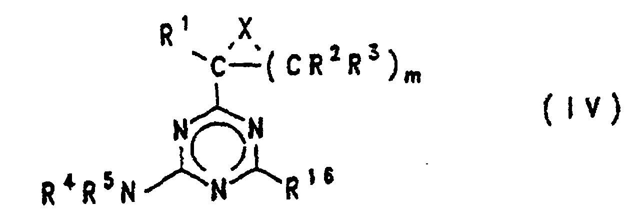 dans laquelle R¹ à R⁵, X et m sont définis comme à la formule (I) selon l'une des revendications 1 à 5 et
R¹⁶ représente un groupe alkylthio en C₁ à C₄, alkylsulfonyle en C₁ à C₄, phényl-alkylthio en C₁ à C₄, phényl-alkylsulfonyle en C₁ à C₄, alkyle en C₁ à C₄-phénylthio ou alkyle en C₁ à C₄-phénylsulfonyle.
